# EUROPEAN PATENT SPECIFICATION

(11) **EP 3 701 043 B1**
(45) Date of publication and mention of the grant of the patent: **22.11.2023**
(21) Application number: 18871519.7
(22) Date of filing: 23.10.2018
(51) Int. Cl.: C12Q 1/68, G01N 33/50, C12Q 1/6883

(54) **A NONINVASIVE MOLECULAR CLOCK FOR FETAL DEVELOPMENT PREDICTS GESTATIONAL AGE AND PRETERM DELIVERY**
NICHTINVASIVER MOLEKULARER TAKT FÜR DIE FÖTALE ENTWICKLUNG ZUR VORHERSAGE DES GESTATIONSALTERS UND EINER FRÜHGEBURT
HORLOGE MOLÉCULAIRE NON INVASIVE RELATIVE AU DÉVELOPPEMENT FOETAL ET PRÉDISANT L'ÂGE GESTATIONNEL ET L'ACCOUCHEMENT PRÉMATURÉ

(30) Priority: 23.10.2017 US 201762576033 P; 27.10.2017 US 201762578360 P
(43) Date of publication of application: 02.09.2020
(73) Proprietor: CZ Biohub SF, LLC, San Francisco, CA 94158 (US); The Board of Trustees of the Leland Stanford Junior University, Stanford, CA 94305-2038 (US); Statens Serum Institut, 2300 Copenhagen (DK)
(72) Inventor: MOUFARREJ, Mira N., Stanford California 94305 (US); NGO, Thuy T. M., Stanford California 94305 (US); CAMUNAS-SOLER, Joan, Stanford California 94305 (US); MELBYE, Mads, Stanford California 94305 (US); QUAKE, Stephen R., San Francisco California 94158 (US)
(74) Representative: Patent Boutique LLP
(86) International application number: PCT/US2018/057142
(87) International publication number: WO 2019/084033

(56) References cited:
- WO-A1-2014/105985
- WO-A1-2014/105985
- WO-A1-2015/010442
- WO-A1-2017/096405
- WO-A1-2017/096405
- WO-A2-2009/134452
- YUJING JAN HENG ET AL: "Whole Blood Gene Expression Profile Associated with Spontaneous Preterm Birth in Women with Threatened Preterm Labor", PLOS ONE, vol. 9, no. 5, 14 May 2014 (2014-05-14), pages 1-13, XP055734200, ISSN: 1932-6203, DOI: 10.1371/journal.pone.0096901
- HENG YUJING J. ET AL: "Maternal Whole Blood Gene Expression at 18 and 28 Weeks of Gestation Associated with Spontaneous Preterm Birth in Asymptomatic Women", PLOS ONE, vol. 11, no. 6, 22 June 2016 (2016-06-22), page e0155191, XP055820316, DOI: 10.1371/journal.pone.0155191
- SILER-KHODR, T. M. et al.: "Gonadotropin-releasing Hormone Effects on Placental Hormones during Gestation: I. Alpha-Human Chorionic Gonadotropin, Human Chorionic Gonadotropin and Human Chorionic Somatomammotropin", Biology of Reproduction, vol. 34, 1986, pages 245-254, XP055596325,
- NGO, T. T. M. et al.: "A Noninvasive Molecular Clock for Fetal Development Predicts Gestational Age and Preterm Delivery", bioRxiv, Bioengineering, XP055596331, Retrieved from the Internet: URL:https://www.biorxiv.org/content/biorxi v/early/2017/11/01/212910.full.pdf [retrieved on 2017-11-01]
- WESTERGAARD, J. G. et al.: "Single Measurements of Chorionic Gonadotropin and Schwangerschafts Protein for Assessing Gestational Age and Predicting the Day of Delivery", The Journal of Reproductive Medicine, vol. 30, no. 1, 1985, pages 57-60, XP008055921,
- CONDE-AGUDELO, A. et al.: "Novel biomarkers for the prediction of the spontaneous preterm birth phenotype: a systematic review and meta-analysis", BJOG: An International Journal of Obstetrics & Gynaecology, vol. 118, 2011, pages 1042-1054, XP055596334,

## Description

### FIELD OF THE INVENTION

The invention is in the field of medicine.

### CROSS REFERENCE TO RELATED APPLICATIONS

This application claims benefit of U.S. Provisional Application No. 62/576,033 (filed October 23, 2017) and No. 62/578,360 (filed October 27, 2017).

### SEQUENCE LISTING

The instant application contains a Sequence Listing

### BACKGROUND

Understanding the timing and program of human development has been a topic of interest for thousands of years. In antiquity, the ancient Greeks had surprisingly detailed knowledge of various details of stages of fetal development, and they developed mathematical theories to try to account for the timing of important landmarks during development including delivery of the baby (Hanson 1995; Hanson 1987; Parker 1999). In the modern era, biologists have put together a detailed cellular and molecular portrait of both fetal and placental development. However, these results relate to pregnancy in general and have not led to molecular tests, which might enable monitoring of development and prediction of delivery for a given set of parents. The most widely used molecular metrics of development are determining the levels of human chorionic gonadotropin (HCG) and alpha-fetoprotein (AFP), which can be used to detect conception and fetal complications, respectively; however, neither molecule either individually or in conjunction has been found to precisely establish gestational age (Dugoff et al. 2005; Yefet et al. 2017).

Due to the lack of a useful molecular test, most clinicians use either ultrasound imaging or the patient's estimate of last menstruation period (LMP) in order to establish gestational age and a rough estimate for delivery date. However, these methods are neither particularly precise nor useful for predicting preterm delivery, which is a substantial source of mortality and cost in prenatal healthcare. Moreover, inaccurate dating can misguide the assessment of fetal development even for normal term pregnancies, which has been shown to ultimately lead to unnecessary induction of labor and cesarean sections, extended post-natal care, and increased expendable medical expenses (Bennett et al. 2004; Whitworth et al. 2015).

It would be useful both to develop a more precise approach to measure the gestational age of the fetus at various points in pregnancy, and more generally to monitor fetal and placental development for signs of abnormality or preterm delivery. Approximately 15 million neonates are born preterm every year worldwide (Blencowe et al. 2013). As the leading cause of neonatal death and the second cause of childhood death under the age of 5 years (Liu et al. 2012), premature delivery is estimated to annually cost the United States upward of $26.2 billion (Institute of Medicine (US) Committee on Understanding Premature Birth and Assuring Healthy Outcomes 2007). The complications continue later into life as preterm birth is a leading cause of life years lost to ill health, disability, or early death (Murray et al. 2012). Two-thirds of preterm delivery occur spontaneously, and the only predictors are a history of preterm birth, multiple gestations, and vaginal bleeding (Institute of Medicine (US) Committee on Understanding Premature Birth and Assuring Healthy Outcomes 2007). Efforts to find a genetic cause have had only limited success (Ward et al. 2005; York et al. 2009) and therefore most effort is focused on phenotypic and environmental causes (Muglia and Katz 2010). WO 2015/010442 A1 describes a method for determining increased risk of premature birth in a pregnant woman by detecting altered expression levels of one or more marker genes in the woman's blood. Heng et al, PLOS ONE, 9, 5, 14 May 2014, pages 1-13 describes the study of differential whole blood gene expression associated with spontaneous preterm birth. WO 2009/134452 A2 describes the identification and use of a plurality of biomarkers to provide a risk assessment of a woman for preterm delivery, and products and processes related thereto. Heng et al, PLOS ONE, 11, 6, 22 June 2016, page e0155191, describes the investigation of maternal whole blood gene expression profiles associated with spontaneous preterm birth. WO 2014/105985 A1 describes biomarkers of preterm birth, biomarkers of term birth, and methods of use thereof. WO 2017/096405 A1 describes proteomic biomarkers of spontaneous preterm birth, proteomic biomarkers of term birth, and methods of use thereof.

### BRIEF SUMMARY

The invention provides a method for assessing risk of preterm delivery by a pregnant woman as defined by claim 1. The invention also provides a method performed using a computer for assessing risk of preterm delivery by a pregnant woman as defined by claim 14. The invention also provides a computer system as defined by claim 15.

Risk of preterm delivery may be determined by (a) generating an expression profile using cfRNA (or protein) from a maternal sample, and (b) determining whether the expression profile is or is not characteristic of a population with a history of preterm delivery and/or whether the expression profile is or is not characteristic of a population with a history of full-term delivery.

In some embodiments, the method includes determining the expression profile by measuring cell-free RNAs (cfRNAs) in the maternal sample. In some embodiments, the method includes determining the expression profile by measuring placental proteins in the maternal sample.

In some embodiments, the method includes a maternal sample from blood, blood plasma, blood serum, or urine. In some embodiments, the method includes a maternal sample obtained from the mother during the third trimester of pregnancy. In some embodiments, the method includes a maternal sample obtained from the mother during the second trimester of pregnancy.

In some embodiments, the method includes one or more reference expression levels for the full-term population are established using a machine learning technique. In some versions, the method further includes obtaining a plurality of training samples, each labeled as preterm or full-term, obtaining one or more measured expression levels for the panel of genes for each of the plurality of training samples, and iteratively adjusting the one or more reference expression levels using the machine learning technique to increase a number of the training samples that are classified correctly as a result of comparing the one or more measured expression levels to the one or more reference expression levels.

In some embodiments, the method further includes the steps: comparing the expression levels to other reference expression levels for the panel of genes, wherein the other reference expression levels are obtained from a preterm delivery population, to determine whether the maternal expression profile is similar to, or is different from, the other reference expression levels within a threshold.

In a first aspect, the invention provides a method for assessing risk of preterm delivery by a pregnant woman comprising, analyzing a maternal sample to determine an expression profile from a panel comprising RAB27B and two or more genes selected from PPBP, DAPP1, RGS18, CLCN3, MAP3K7CL, MOB1B, POLE2, LYPLAL1, and TBC1D15.

In some embodiments, the method includes genes having higher expression levels in a preterm population than in a term population. The method includes genes selected from: RAB27B and two or more genes selected from CLCN3, DAPP1, POLE2, PPBP, LYPLAL1, MAP3K7CL, MOB1B, RGS18, and TBC1D15, or from: CLCN3, DAPP1, PPBP, MAP3K7CL, MOB1B, and RGS18. In some embodiments, the method includes a panel comprising three genes selected from: RAB27B and two genes selected from CLCN3, DAPP1, POLE2, PPBP, LYPLAL1, MAP3K7CL, MOB1B, RGS18, and TBC1D15 (ten transcript panel), or from: CLCN3, DAPP1, PPBP, MAP3K7CL, MOB1B, and RGS18 (seven transcript panel).

In some embodiments, the method includes the expression profiles in which a panel of three to ten genes are determined. In some embodiments, the method includes the expression profile in which a panel comprising exactly three genes are determined.

In some versions the method includes, determining the expression profile by measuring cell-free RNAs (cfRNAs) in the maternal sample. In some embodiments, the method includes determining the expression profile by measuring proteins in the maternal sample.

In some embodiments, the method includes a maternal sample from blood, blood plasma, blood serum, or urine. In some embodiments, the method includes a maternal sample obtained more than 28 days prior to preterm delivery. In some embodiments, the method includes a maternal sample obtained more than 45 days prior to preterm delivery. In some embodiments, the method includes a maternal sample obtained after the second month and prior to the eighth month of pregnancy. In some embodiments, the method includes a maternal sample obtained during the second trimester of pregnancy.

In some versions, a maternal sample is obtained during the third trimester of pregnancy.

In some embodiments, the method of the first aspect includes, a maternal sample obtained at a specified week of pregnancy, comprising the steps: comparing the expression profile to a time matched reference profile, wherein the time matched reference profile is characteristic of a normal term pregnancy at the specified week of pregnancy, and identifying the pregnant woman as an elevated risk for preterm delivery if the expression profile differs significantly from the time matched reference profile within a threshold.

In some embodiments, the method of the first aspect includes a maternal sample obtained at a specified week of pregnancy, comprising the steps: comparing the expression profile to a time matched reference profile, wherein the time matched reference profile is characteristic of a preterm pregnancy, and identifying the pregnant woman as an elevated risk for preterm delivery if the expression profile is significantly similar to the time matched reference profile within a threshold.

In an second aspect, the disclosure provides a method for assessing risk of preterm delivery of a pregnant woman comprising the steps: (a) obtaining a maternal expression profile for a sample, comprising expression levels for a panel of genes according to the first aspect of the disclosure, and (b) comparing the expression levels to reference expression levels for the panel of genes, wherein the reference expression levels are obtained from a preterm delivery population, a full-term delivery population, or both populations, to determine whether the maternal expression profile is similar to, or is different from, the reference expression levels within a threshold.

In some embodiments, the method one or more reference levels are established using a machine learning technique.

In some embodiments, the methods of the first or second aspect are carried out by a computer.

In some embodiments, the method includes carrying out the steps provided in the first or second aspect with two or more maternal samples obtained at different times during the course of a pregnancy.

The method of the first aspect, wherein the expression levels of individual genes are determined by qPCR or massively parallel sequencing.

The method of the first aspect, wherein the expression levels of individual genes are determined by mass spectrometry or an antibody array.

In some embodiments, the method includes a maternal sample from blood, blood plasma, blood serum, or urine.

In some embodiments, the method includes a maternal sample obtained from the mother during the third trimester of pregnancy.

In some embodiments, the method includes a maternal sample obtained from the mother during the second trimester of pregnancy.

### BRIEF DESCRIPTION OF THE DRAWINGS

FIGS. 1A-1B are temporal graphs showing collection timelines from pregnant women in three different cohorts: Denmark (FIG. 1A), Pennsylvania and Alabama (FIG. 1B). Squares, inverted triangles, and lines indicate sample collection, delivery date, and individual patients, respectively.
FIG. 2A shows data from representative gene expression arrays of placenta, immune or organ specific genes (last row). Gene-specific inter-patient monthly averages ± standard error of the mean (SEM) plotted over the course of gestation (shaded in gray). ^{†} represents genes for which data for only 21 patients was available.
FIG. 2B is a heatmap showing correlation between gene-specific estimated transcript counts. Genes are listed in the same order as FIG. 2A while omitting genes for which data was only available for 21 patients. Placental (rows/columns 1-20), immune (rows/columns 21-29) and organ specific genes (rows/columns 30-36) are shown.
FIGS. 2C-2D show solid lines and shading that indicate linear fit and 95% confidence intervals, respectively. FIG. 2C shows an exemplary random forest model prediction of time to delivery for training data (n=21, R=0.91, P < 2.2 × 10⁻¹⁶, cross-validation). FIG. 2D shows an exemplary random forest model prediction of time to delivery for validation data (n=10, R=0.89, P < 2.2 × 10⁻¹⁶).
FIG. 2E are graphs showing comparison of expected delivery date prediction during the second, third trimester, or both second and third trimesters, by ultrasound or cell-free RNA methods of the invention.
FIG. 3A shows a heat map for 40 differentially expressed genes (p<0.001) between preterm deliveries and normal deliveries. RNA-Seq was performed on samples from Pennsylvania.
FIG. 3B shows individual plots of 10 genes identified and validated in an independent cohort from Alabama, which accurately predicted preterm delivery using any unique combination of 3 genes from this set. All p-values reported are calculated using the Fisher exact test (FDR < 5%). *, **, and *** indicate significance levels below 0.05, 0.005, and 0.0005, respectively.
FIG. 3C is a graph showing predictive performance of the 10 validated preterm biomarkers in unique combinations of 3 genes from FIG. 3B. Area under the curve (AUC) values are highlighted both for the discovery (Pennsylvania and Denmark) and validation (Alabama) cohorts.
FIG. 4 shows data from representative gene expression arrays of placenta or immune genes. Gene-specific inter-patient monthly averages ± standard error of the mean (SEM) plotted over the course of gestation (shaded in gray). ^{†} represents genes for which data for only 21 patients was available.
FIG. 5 shows a random forest model built using 9 placental genes outperforming a random forest model built using 51 genes of placental, immune and tissue-specific organ origin to predict gestational age by root mean squared error (RMSE).
FIGS. 6A and 6B show solid lines and shading indicating a linear fit and 95% confidence intervals, respectively. FIG. 6A shows an exemplary random forest model prediction of gestational age for training data (n=21, R=0.91, P < 2.2 × 10⁻¹⁶, cross-validation) and FIG. 6B shows an exemplary random forest model prediction of gestational age for validation data (n=10, R=0.90, P < 2.2 × 10⁻¹⁶).

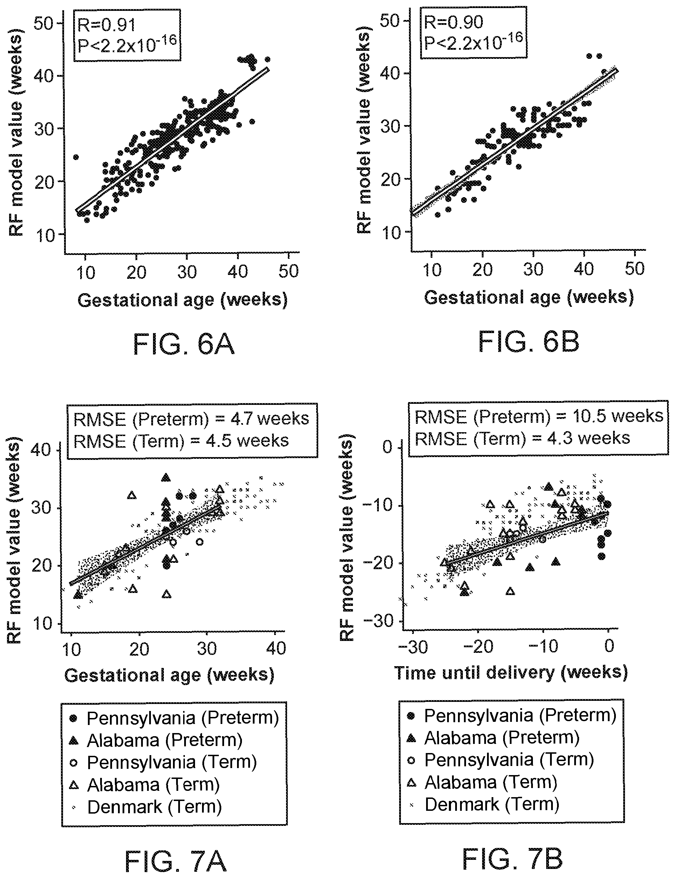

FIGS. 7A and 7B show solid lines and shading indicating a linear fit and 95% confidence intervals, respectively. Training and validation data are reported above each graph. Random forest model prediction of gestational age and time to delivery for normal and preterm samples reveals that although the model works well for prediction of gestational age for normal deliveries (RMSE = 4.5) and preterm deliveries (RMSE = 4.7) (FIG. 7A), it fails to accurately predict time to delivery in the preterm cases (RMSE = 10.5 weeks) (FIG. 7B); while accurately predicting time to delivery for normal deliveries (FIG. 7B).
FIG. 8 shows RT-qPCR measurements agree with previously determined RNA-Seq values.

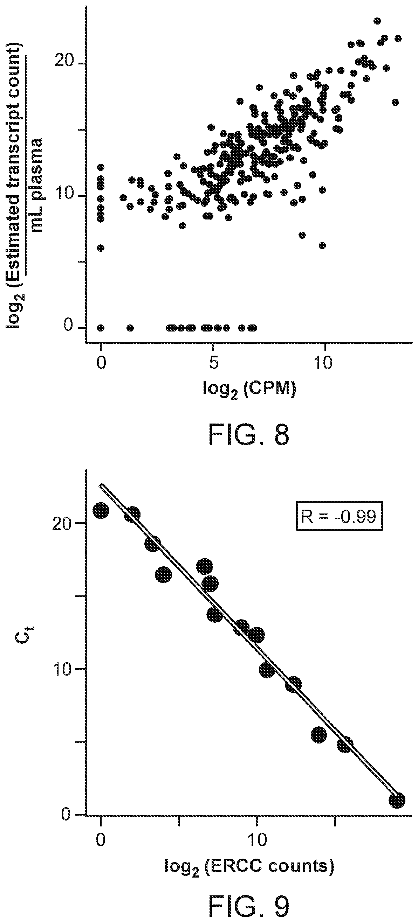

FIG. 9 shows Cₜ counts for each gene under evaluation are back-calculated from Cₜ values using a standard curve generated using a common set of external RNA controls developed by the External RNA Controls Consortium (ERCC). The control consists of a set of unlabeled, polyadenylated transcripts designed to be added to an RNA analysis experiment after sample isolation and prior to interrogation. ERCC Spike-In Control Mixes are commercially available, pre-formulated blends of 92 transcripts, designed to be 250 to 2,000 nucleotides in length, which mimic natural eukaryotic mRNAs (e.g., ERCC RNA Spike-In Mix, Invitrogen, CA, Catalog No. 4456740).
FIGS. 10A-10D provide an exemplary list of genes found to be significantly different between spontaneous preterm delivery and normal delivery samples using three statistical analyses.

### DETAILED DESCRIPTION OF THE INVENTION

### 1. INTRODUCTION

We have discovered a panel of genetic biomarkers for non-invasively predicting gestational age or time to delivery of a fetus in a pregnant woman. We have also discovered an orthogonal set of genetic biomarkers for non-invasively predicting whether a woman is at risk for preterm delivery of a fetus. The discovery that a set of genetic markers for predicting gestational age or time to delivery of a fetus is significant, in part, because of the potential advantages of replacing ultrasounds as the gold standard for predicting gestational age and thus avoiding substantial health care expenses associated with ultrasounds and sonographers. Additionally, the discovery that a set of genetic markers for predicting whether a woman is at risk for preterm delivery is also significant, in part, because of the potential advantages of prophylactically treating women at risk from preterm delivery and thus negating substantial health care expenses associated with neonatal intensive care units (NICU's).

We performed a high time-resolution study of normal human development by measuring cfRNA in blood from pregnant women longitudinally during each week of pregnancy. Analysis of tissue-specific transcripts in these samples enabled us to follow fetal and placental development with high resolution and sensitivity, and also to detect gene-specific response of the maternal immune system to pregnancy. The data from this study establish a "clock" for normal human development and enable a direct molecular approach to establish expected delivery date with comparable accuracy to ultrasound at a fraction of the cost. We also identified an orthogonal gene set that accurately discriminates women at risk of preterm delivery up to two months in advance of labor, forming the basis of a screening or diagnostic test for risk of prematurity.

### 2. DEFINITIONS

As used herein, the terms "cell free RNA" or "cfRNA" refer to RNA, especially mRNA, expressed by cells of the mother, fetus and/or placenta and recoverable from the non-cellular fraction of maternal blood, and includes fragments of full-length RNA transcripts. In some embodiments "cfRNA" does not include rRNA. In some embodiments "cfRNA" does not include miRNA. In some embodiments "cfRNA" refers to mRNA. Cf RNA can also be recovered from maternal urine.

As used herein, the terms "placental gene," "placental gene product," "placental cfRNA," or "placental protein" refer to a gene or corresponding gene product that is expressed in the placenta but not expressed (or expressed at significantly lower levels) by maternal or fetal tissues. Publicly available resources exist to identify placental genes including databases such as Tissue-Specific Gene Expression and Regulation (TiGER) which identifies 377 RefSeq (NCBI Reference Sequence Database) genes as being preferentially expressed in the placenta (http://bioinfo.wilmer.jhu.edu/tiger). Other databases such as Expression Atlas (https://www.ebi.ac.uk/gxa/home) can also be used to identify placental genes. Placental gene products include mRNA and protein.

As used herein, the term "expression profile," refers to the level of expression of one or a plurality of gene products obtained from a maternal sample. The gene products may be cfRNAs or proteins. For gene products recovered from maternal plasma, expression levels may be expressed as the number of transcripts of a specified RNA per mL maternal plasma, mass of a specified polypeptide per mL maternal plasma, transcript count calculated from RNA-Seq, or any other suitable units. Analogous units may be used for gene products obtained from other maternal samples, such as urine. Expression of gene products may be determined using any suitable method (*e.g*., as described below). Measured values are typically normalized to account for variations in the quantity and quality of the sample, reverse-transcription efficiency, and the like. When an expression profile reflects expression from multiple different gene products (*e.g*., different cfRNA transcripts) the gene products may be given different weights when generating or comparing expression profiles or reference profiles. For example, when comparing an expression profile comprising cfRNA 1 and cfRNA 2 in a sample from a pregnant woman with a reference profile (discussed below), a 2-fold difference in values for cfRNA 1 may be given more weight than a 2-fold difference in values for cfRNA 2 in determining a degree of similarity or difference between the expression profile and the reference profile. An expression profile from a maternal (*e.g*., patient) sample is sometimes referred to as a "maternal expression profile" and a maternal expression profile from a sample collected at a specified time may be referred to as a "[time] maternal expression profile," e.g., a "24 week maternal expression profile."

As used herein, a "reference profile" is an expression profile derived from a reference population. For illustration, examples of reference populations are pregnant women, pregnant women who delivered at term, or pregnant women who delivered prematurely. In some embodiments the reference population is a subpopulation of pregnant women characterized by maternal age (e.g., women 20-25 years old who delivered at term), race or ethnicity (e.g., African-American women who delivered at term), and the like. A reference profile is generated by combining expression profiles of a statistically significant number of women in the population and, for a specified gene product, may reflect the mean transcript level in the population, the median transcript level in the population, or may be determined using any of a number of methods known in the fields of epidemiology and medicine. A reference population will typically comprise at least 10 subjects (e.g., 10-200 subjects), sometimes 50 or more subjects, and sometimes 1000 or more subjects.

As used herein, the term "profile panel" refers to the set of gene products measured in a particular assay. For example, in an assay for six (6) different cfRNAs ("RNAs A-F"), those six cfRNAs would be the profile panel. Likewise, in an assay for six (6) different proteins from maternal plasma or urine, those six proteins would be the profile panel. As another illustration, in an assay in which expression data are collected for transcripts of a large number of genes (e.g., the entire transcriptome, or a large number of placental gene transcripts) the subset used for estimating gestational age or time to delivery, or assessing risk of preterm delivery may be referred to as the profile panel. It will be recognized that measurements of RNAs or proteins not included in the panel may be used as controls, to normalize measurements within or across samples, or for similar uses. In some embodiments a profile panel may include a set of gene products that includes both cfRNAs and proteins. A profile panel is sometimes referred to as a "panel."

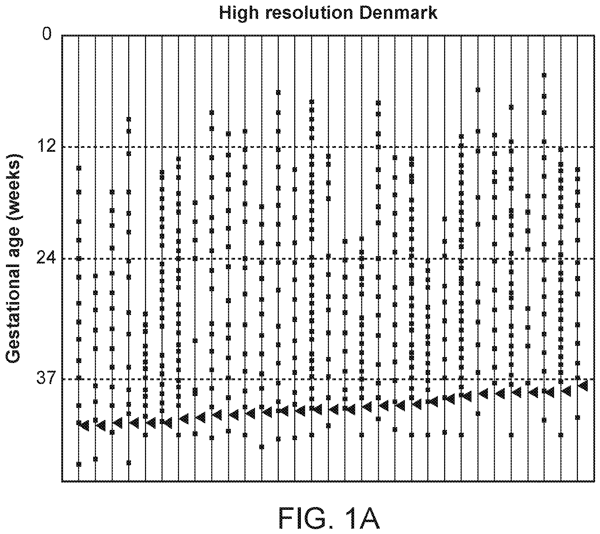

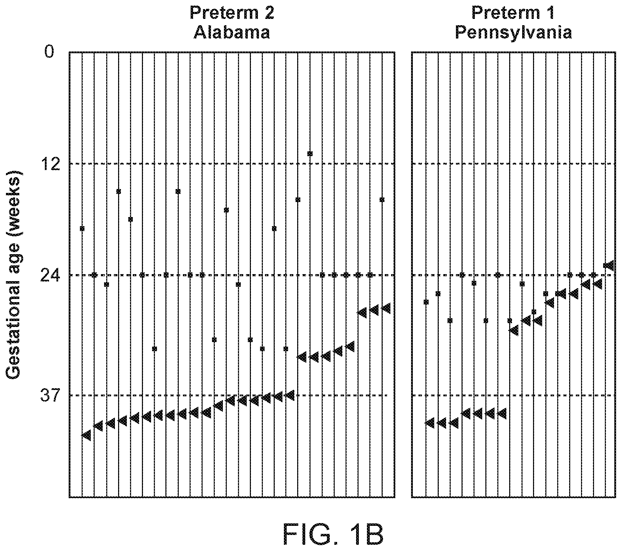

As used herein, the terms "preterm pregnancy," "preterm delivery," "full-term pregnancy," "full-term delivery," and "normal term pregnancy" have their normal meanings. Full-term refers to delivery after the fetus reached a gestational age of 37 weeks and preterm refers to delivery prior to the fetus reaching a gestational age of 37 weeks. In some contexts preterm refers to delivery in the period from 16 weeks to 35 weeks gestational age or 24 weeks to 30 weeks gestational age. Preterm populations used in the studies discussed below (see Examples) delivered a fetus prior to 29 weeks gestational age in one case (Pennsylvania cohort) and 33 weeks gestational age in another (Alabama cohort). See Fig. 1.

As used herein, "maternal sample" refers sample of a body fluid obtained from a pregnant woman. The body fluid is typically serum, plasma, or urine, and is usually serum. In some embodiments a sample of a different body fluid may be used, such as saliva, cerebrospinal fluid, pleural effusions, and the like. Maternal samples may be obtained at multiple different time points during pregnancy and stored (e.g., frozen) until assayed. It will be appreciated that the date of collection of a maternal sample is an integral property of the sample.

As used herein, "time to delivery" refers to the number of weeks from a specified time (present time, date of maternal sample collection) to the delivery date or predicted delivery date. Time to delivery is calculated as (gestational age at delivery) minus (gestational age at sample collection).

As used herein, the terms "protein" and "polypeptide" are used interchangeably. Reference to a protein obtained from a maternal sample does not necessarily imply that the protein is a full-length gene expression product. Portions, fragments, and cleavage products may be detected and identifed according to the invention.

### 3. ILLUSTRATIVE METHODS AND EMBODIMENTS USING CELL-FREE RNA EXPRESSION PROFILES

The invention relates to discovery of a high resolution molecular clock for fetal development and the invention of methods to establish time to delivery (not claimed), fetal gestational age (not claimed), and risk of preterm delivery. In one aspect, methods and materials for estimating gestational age or time to delivery of a fetus using expression profiles of placental gene(s) are described. In another aspect, methods and materials for assessing risk of preterm delivery are described.

For illustration and not limitation, gestational age or time to delivery may be determined by (a) generating an expression profile using cfRNA (or protein) from a maternal sample and (b) comparing the expression profile with one or more reference profiles that reflect an expression profile characteristic of a defined gestational age. For illustration, the maternal expression profile is compared to 37 reference profiles (characteristic of 1 through 37 weeks of gestational age) and gestational age or time to delivery is estimated based on the relatedness of the maternal expression profile to one of the 37 reference profiles. For illustration and not limitation, risk of preterm delivery may be determined by (a) generating an expression profile using cfRNA (or protein) from a maternal sample and (b) determining whether the expression profile is or is not characteristic of a population with a history of preterm delivery and/or whether the expression profile is or is not characteristic of a population with a history of full-term delivery. In another approach, machine learning (e.g,. random forest regression, support vector machines, elastic net, lasso) is used to predict gestational age, time to delivery, and risk of prematurity based on the maternal expression profile generated from a maternal sample.

### 3.1 Obtaining the Maternal Sample

A maternal sample (e.g., plasma or urine) may be collected and cfRNA may be isolated from the sample immediately or after storage. See Example 1 below. Art-known methods may be employed to guard the RNA fraction against degradation including, for example, use of special collection tubes (e.g. PAXgene RNA tubes from Preanalytix, Tempus Blood RNA tubes from Applied Biosystems) or additives (e.g. RNAlater from Ambion, RNAsin from Promega) that stabilize the RNA fraction.

Multiple maternal samples may be collected. For example, maternal samples can be collected each trimester, or monthly for a period during the course of pregnancy (e.g., months 3-8). When indicated, maternal samples may be collected more frequently. For example, gestational age or time to delivery may be monitored frequently (e.g., biweekly) as a method for monitoring fetal health.

As another example, a woman identified at 24 weeks as at risk of preterm delivery may elect biweekly assays to monitor risk. In cases in which intervention to avoid preterm delivery (e.g., progesterone supplementation) has been used, a maternal sample may be obtained after the initiation of the intervention to assess whether the intervention has changed the maternal expression profile. Remarkably, methods of the invention may be used to accurately discriminate women at risk of preterm delivery up to two months in advance of labor. See Example 6. In some embodiments of the invention a maternal sample is obtained more than 28 days prior to the preterm delivery. In some embodiments of the invention a maternal sample is obtained more than 45 days prior to the preterm delivery. In some embodiments a maternal sample is obtained after the second month and prior to the eighth month of pregnancy. In some embodiments a maternal sample is obtained during the second trimester of pregnancy In some embodiments a maternal sample is obtained during the third trimester of pregnancy. As discussed above, in many cases a maternal sample may be obtained and assayed more than once during the course of a pregnancy.

### 3.2 Isolation of cfRNA

Cell-free RNA can be isolated from a maternal sample using techniques well known in the art. See Example 1 below. Isolation of cfRNA from blood or blood fractions is described in Qin et al., BMC Res. Notes., 26;6:380 (2013) and Mersy et al., Clin. Chem., 61(12)1515-23 (2015). Kits for isolating cfRNA from blood are known and are commercially available (e.g., PaxGene Blood RNA kit (Qiagen, Catalog No. 762164). Kits for isolating cfRNA from plasma/serum are known and are commercially available (e.g., Plasma/Serum RNA Purification Kit from Norgen Biotek Corporation, Canada, Catalog No.: 56900 and Quick-cfRNA^{™} Serum & Plasma from Zymo Research, Catalog No.: R1059; NextPrep Magnazol cfRNA Isolation Kit (Bioo Scientific); Quick-cfRNA^{™} Serum & Plasma Kit (Zymo Research), and the QIAamp^{®} Circulating Nucleic Acid Kit (Qiagen).

Isolation of cfRNA from urine has been described (see, e.g., Zhao et al., 2015, Int. J. Cancer, 1;136(11):2610-5 describing use of cfRNA for identification of biomarkers and monitoring disease status). Kits for isolating cfRNA from urine are known and are commercially available (e.g., Urine Cell Free Circulating RNA Purification Kit from Norgen Biotek Corporation, Canada, Catalog No.: 56900).

### 3.3 Quantification of cfRNA Transcripts

Quantification of specific transcripts from a cell free RNA sample can be accomplished in a variety of ways including, but not limited to, array-based methods, amplification-based methods (e.g., RT-qPCR), and high-throughput sequencing (RNA-Seq). The methods of the invention are not limited to a particular method of quantitation.

### 3.3.1 RT-qPCR Assays

RT-qPCR assays are described in Example 1, below. Briefly, RNA is transcribed into complementary DNA (cDNA) by reverse transcriptase from total RNA or messenger RNA (mRNA). Alternatively, cDNA is generated using template-specific primers specific for selected RNA transcripts (e.g., one of more of SEQ ID NOS:1-19). The cDNA is then used as the template for the qPCR reaction.

RT-qPCR can be performed in a one-step or a two-step assay. One-step assays combine reverse transcription and PCR in a single tube and buffer, using a reverse transcriptase along with a DNA polymerase. One-step RT-qPCR only utilizes sequence-specific primers. In two-step assays, the reverse transcription and PCR steps are performed in separate tubes, with different optimized buffers, reaction conditions, and priming strategies (such as random primers, oligo-(dT) or sequence specific primers in the reverse transcription followed by sequence specific primers in the qPCR step. As described above, it will be apparent that reference to RT-qPCR herein includes either a one or two step RT-qPCR assay.

RT-qPCR can be performed using various buffers and optimizations. See Example 1 below. Isolation of cfRNA from blood and subsequent analysis by RT-qPCR is known in the art (for example, see US Patent Publication No.: 20140199681). Kits for performing one step RT-qPCR are known and are commercially available (e.g., TaqPath^{™} 1-step RT-qPCR Master Mix, CG (Thermo Fisher Scientific, Catalog No. A15299). Kits for performing two step RT-qPCR are known and are commercially available (e.g., Maxima First Strand cDNA Synthesis Kit for RT-qPCR (Thermo Fisher Scientific, Catalog No. K1641).

### 3.3.2 RNA-Seq Assays

RNA-Seq (RNA-sequencing) assays also known as whole transcriptome shotgun sequencing uses next-generation sequencing (NGS) to reveal the presence and quantity of RNA in a sample at a given point in time (see, Zhong et al. Not. Rev. Gen. 10 (1): 57-63 (2009*)*)*.* RNA-Seq assays are described in Example 1, below. RNA-Seq facilitates the ability to look at changes in gene expression over time or differences in gene expression in different groups or treatments (see, Maher et al. Nature. 458 (7234): 97-101 (2009*)*)*.*

The following sets forth an exemplary method to analyze cfRNAs isolated from a maternal body fluid sample. Briefly, cfRNAs are isolated from a maternal sample, for example using sequence specific primers, oligo(dT) or random primers to generate cDNA molecules. In one approach cDNA is generated using template-specific primers specific for selected RNA transcripts (e.g., corresponding to genes listed in TABLES 1 and 2; one of more of SEQ ID NOS:1-19). The cDNA molecules can be fragmented and optimized such that sequencing linkers are added to the 3' and 5' ends of the cDNA molecules to produce a sequencing library. Fragmentation is typically not needed for cfRNA. The optimized cDNAs are then sequenced using an NGS sequencing platform. Suitable kits for amplifying cDNA and analyzing sequencing products in accordance with the methods of the invention include, for example, the Ovation^{™} RNA-Seq System (NuGen). Other methods for preparing RNA-Seq libraries for use with a sequencing platform are known such as Podnar et al., 2014, "Next-Generation Sequencing RNA-Seq Library Construction" Curr Protoc Mol Biol. 2014 Apr 14;106:4.21.1-19. doi: 10.1002/0471142727.mb0421s106; Schuierer et al., 2017, "A comprehensive assessment of RNA-Seq protocols for degraded and low-quantity samples. BMC Genomics. 2017 Jun 5;18(1):442. doi: 10.1186/s12864-017-3827-y; Hrdlickova R, 2017, RNA-Seq methods for transcriptome analysis, Wiley Interdiscip Rev RAM. 2017 Jan;8(1). doi: 10.1002/wrna.1364).

Sequencing libraries suitable for use with RNA-Seq assays can include cDNAs derived from cfRNAs isolated from a maternal sample. It will also be apparent that the sequencing libraries can include cDNAs derived from other RNA species (e.g., miRNAs) that may have been collected during total RNA isolation rather than a cfRNA isolation procedure. Accordingly, either a partial or complete transcriptome analysis can be performed on the RNA content obtained from the maternal sample. In one embodiment, it is preferred that only cfRNAs obtained from the maternal sample are used as the input material for preparing cDNAs suitable for RNA-Seq.

### 3.4 Profile Panels

The inventors have discovered that certain combinations of gene products are of particular use in practicing the invention. That is, certain combinations of gene products have been identified as sufficient or preferred for providing accurate estimates of gestational age (not claimed), time to delivery (not claimed) or predicting likelihood of preterm delivery. For example, as described in Example 4, a subset of 9 placental genes provided more predictive power for estimating gestational age or time to delivery than a larger gene panel.

It will be appreciated that, although certain features of panels are discussed in this section, the invention is not limited to these particular described embodiments. It also will be understood that although this section describes panels by reference to cfRNA transcript expression, panels based on expression levels of circulating proteins encoded by the those gene subsets may also be used to determine gestational age or time to delivery and identify women at risk of preterm delivery. See Section 4, below.

In some approaches, multiple different profile panels are used during the course of a woman's pregnancy. For example, a first profile panel may be used in the second trimester and a different profile panel may be used in the third trimester.

### 3.4.1 Profile Panels For Determining Gestational Age Or Time To Delivery (not claimed)

A method for estimating gestational age or time to delivery of a fetus by analyzing a maternal sample to determine an expression profile of placental genes (e.g., cfRNA or protein encoded by a placental gene) is described. Suitable panels may be selected based on the information provided in this disclosure. The panel may include one, at least 2, or at least 3 placental genes. The profile panel can include at least 1, 2, 3, 4, 5, 6, 7, 8, 9, 10, 11, 12, 13, 14, 15, 16, 17, 18, 19, or 20 placental genes. The profile panel can include exactly 1, 2, 3, 4, 5, 6, 7, 8, 9, 10, 11, 12, 13, 14, 15, 16, 17, 18, 19, or 20 placental genes. The profile panel may include fewer than 100 genes, e.g., fewer than 100 placental genes, sometimes fewer than 50 placental genes, sometimes fewer than 20 placental genes, sometimes fewer than 15 placental genes, sometimes fewer than 10 placental genes, and sometimes fewer than 5 placental genes.

The expression level of each of the placental genes in the profile panel may change during the course of pregnancy. See Examples below. Thus, the expression level of at least one placental gene in the panel may be higher in the first trimester compared to the third trimester. The expression levels of most or all placental genes in the panel may be higher in the first trimester compared to the third trimester. The expression level of at least one placental gene may be lower in the first trimester compared to the third trimester. The expression levels of most or all placental genes in the panel may be lower in the first trimester compared to the third trimester

At least one placental gene may be selected from genes in TABLE 1. All of the placental genes in a profile panel may be genes listed TABLE 1.

The expression profile may include at least 1, at least 2, at least 3, at least 4, at least 5, at least 6, at least 7, at least 8, or 9 genes selected from CGA [SEQ ID NO:1], CAPN6 [SEQ ID NO:2], CGB [SEQ ID NO:3], ALPP [SEQ ID NO:4], CSHL1 [SEQ ID NO:5], PLAC4 [SEQ ID NO:6], PSG7 [SEQ ID NO:7], PAPPA [SEQ ID NO:8], and LGALS14 [SEQ ID NO:9]. The expression profile may include 1, 2, 3, 4, 5, 6, 7, 8, or 9 genes selected from CGA [SEQ ID NO:1], CAPN6 [SEQ ID NO:2], CGB [SEQ ID NO:3], ALPP [SEQ ID NO:4], CSHL1 [SEQ ID NO:5], PLAC4 [SEQ ID NO:6], PSG7 [SEQ ID NO:7], PAPPA [SEQ ID NO:8], and LGALS14 [SEQ ID NO:9]. In one approach the set of placental genes includes at least one gene other than CGA and CGB. In one approach, the profile panel comprises from three (3) to nine (9) cfRNAs selected from SEQ ID NOS:1-9.

Gestational age may be determined using a profile panel profile of 9 genes: CGA, CAPN6, CGB, ALPP, CSHL1, PLAC4, PSG7, PAPPA, and LGALS14. We trained several distinct models on subpopulations of women (i.e., nulliparous or multiparous women, women carrying male or female fetuses) to determine the importance of the 9 genes that compose the transcriptomic signature identified. Training 4 distinct models for women carrying male or female fetuses and nulliparous or multiparous women revealed that 2 of the 9 genes identified in the main text were sufficient to (CGA, CSHL1) or female (CGA, CAPN6) fetuses and multiparous (CGA, CSHL1) women. However, all 9 genes were necessary to optimally predict time until delivery for nulliparous women, highlighting the importance of the transcriptomic signature identified. In some embodiments of the invention the panel comprises CGA and CSHL1 or CGA and CAPN6.

The nine transcripts used to predict gestational age were weighted by the model in the following order of importance (from most to least): CGA, CAPN6, CGB, ALPP, CSHL1, PLAC4, PSG7, PAPPA, and LGALS14. Thus, the determined level of expression for individual genes may be given different weights (or coefficients) when compared to expression in a reference profile. For example, when all 9, or a subset comprising fewer than 9 genes in this group (e.g., 2, 3, 4, 5, 6, 7 or 8) expression values for each gene are ranked CGA > CAPN6 > CGB > ALPP > CSHL1 > PLAC4 > PSG7 > PAPPA > LGALS14.

The panel may include one, at least 2, or at least 3 genes from TABLE 1. The profile panel can include at least 1, 2, 3, 4, 5, 6, 7, 8, 9, 10, 11, 12, 13, 14, 15, 16, 17, 18, 19, or 20 genes from TABLE 1. The profile panel can include exactly 1, 2, 3, 4, 5, 6, 7, 8, 9, 10, 11, 12, 13, 14, 15, 16, 17, 18, 19, or 20 genes from TABLE 1. The profile panel may include fewer than 100 genes, sometimes fewer than 50 genes, sometimes fewer than 20 genes, sometimes fewer than 15 genes, sometimes fewer than 10 genes, and sometimes fewer than 5 genes. In certain approaches the profile panel comprises a number of genes in the range 1-100 genes, 1-50 genes, 1-25 genes, 3-100 genes, 3-50 genes, 3-25 genes, or 3-10 genes.

In some versions the placental genes are selected from genes in TABLE 1. The placental genes may be selected from CGA, CAPN6, CGB, ALPP, CSHL1, PLAC4, PSG7, PAPPA, and LGALS14. The genes may include at least one gene other than CGA. The genes may include at least two, three, four, five, six, seven or eight genes other than CGA. In the genes may include at least one gene other than CGB. The genes may include at least two, three, four, five, six, seven or eight genes other than CGB. The genes may include at least one gene other than CGA and CGB. The method may include determining the expression profile for three (3) to nine placental genes.

### 3.4.2 Profile Panels For Determining Risk of Preterm Delivery

In one aspect, the invention provides a method for estimating risk of preterm delivery by analyzing a maternal sample to determine an expression profile. In one embodiment, the profile panel used for such a determination comprises one or more cfRNA transcripts with higher expression levels in a preterm population than in a term population. In one embodiment, a preterm population refers to a set of women who delivered a fetus prior to 37 weeks gestational age. In another embodiment, a preterm population refers to women who delivered a fetus prior to 33 weeks gestational age. In another embodiment, a preterm population refers to women who delivered a fetus prior to 29 weeks gestational age. In yet another embodiment, a preterm population refers to women who delivered a fetus between 12 and 33 weeks gestational age. In another embodiment, a preterm population refers to a set of women who delivered a fetus between 16 and 29 weeks gestational age. In an embodiment, a preterm population refers to a set of women who delivered a fetus between 16 and 33 weeks gestational age. As noted above, one preterm population used in the Examples consisted of women who delivered a fetus prior to 29 weeks gestational age and this population (or subpopulations thereof) is preferred for making reference profiles characteristic of high risk of prematurity. The Examples also show that biomarkers discovered in a population of women who delivered a fetus prior to 29 weeks are applicable in a population of women who delivered a fetus prior to 33 weeks gestational age.

The profile panel includes 3 or more, genes listed in TABLE 2, as defined in claim 1.

In one approach the profile panel includes three (3) or more genes are selected from the ten transcript panel, comprising RAB27b [SEQ ID NO: 17] and two or more genes selected from CLCN3 [SEQ ID NO:10], DAPP1 [SEQ ID NO:11], POLE2 [SEQ ID NO:12], PPBP [SEQ ID NO:13], LYPLAL1 [SEQ ID NO:14], MAP3K7CL [SEQ ID NO:15], MOB1B [SEQ ID NO:16], RGS18 [SEQ ID NO:18], and TBC1D15 [SEQ ID NO:19]. The profile panel comprises three (3) or more genes selected from SEQ ID NO: 17 and two or more genes selected from SEQ ID NOS:10-16 and SEQ ID NOS: 18-19. In one approach the profile panel comprises exactly three (3) genes including SEQ ID NO: 17 and two or more genes selected from SEQ ID NOS:10-16 and SEQ ID NOS: 18-19. In some embodiments the panel comprises only SEQ ID NO: 17 and two or more genes selected from SEQ ID NOS:10-16 and SEQ ID NOS: 18-19. For example, in various embodiments, the profile panel will comprise the following combinations: (i) MAP3K7CL, MOB1B, RAB27B; (ii) MOB1B, RAB27B, RGS18; and (iii) RAB27B, RGS18, TBC1D15. It will be appreciated that the full list of combinations of 3 genes selected from SEQ ID NOS:10-19 is easily generated, and this paragraph is intended to convey possession of each said combination of 3 genes.

In one approach the profile panel includes three (3) or more genes are selected from the seven transcript panel and include RAB27B [SEQ ID NO: 17] and two or more genes selected from CLCN3 [SEQ ID NO:10], DAPP1 [SEQ ID NO:11], PPBP [SEQ ID NO:13], MAP3K7CL [SEQ ID NO:15], MOB1B [SEQ ID NO:16 and RGS18 [SEQ ID NO:18] . In one approach the profile panel comprises three (3) or more genes including SEQ ID NO: 17 and two or more genes selected from SEQ ID NOS: 10, 11, 13, 15, 16, and-18. In one approach the profile panel comprises exactly three (3) genes including SEQ ID NO: 17 and two or more genes selected from SEQ ID NOS: 10, 11, 13, 15, 16, and-18. In some embodiments the panel comprises only SEQ ID NO: 17 and two or more genes selected from SEQ ID NOS: 10, 11, 13, 15, 16, and-18.

In one approach the profile panel comprises exactly three genes including SEQ ID NO: 17 and two or more genes selected from SEQ ID NOS:10-16 and SEQ ID NOS: 18-19. In one approach the profile panel comprises exactly three genes including SEQ ID NO: 17 and two or more genes selected from SEQ ID NOS: 10, 11, 13, 15, 16, and-18.

The seven transcripts used to identify women at elevated risk or preterm delivery were weighted by the model in the following order of importance (from highest to lowest): RAB27B > PPBP > DAPP1 > RGS18 > (MOB1B, MAP3K7CL, and CLCN3), where MOB1B, MAP3K7CL, and CLCN3 are equally ranked. Thus, in some embodiments the determined level of expression for individual genes are given different weights (or coefficients) when compared to expression in a reference profile. For example, when all 7, or a subset comprising fewer than 7 genes in this group (e.g., 2, 3, 4, 5, 6) expression values for each gene are ranked ): RAB27B > PPBP > DAPP1 > RGS18 > (MOB1B, MAP3K7CL, and CLCN3).

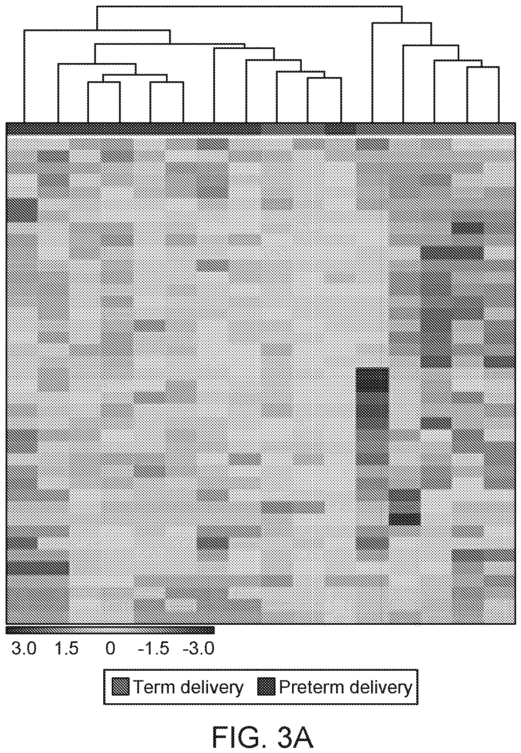

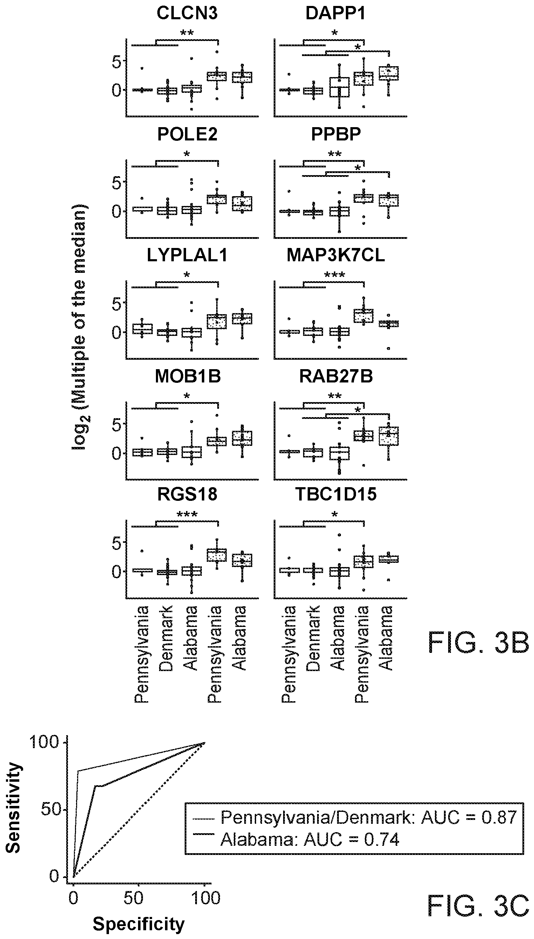

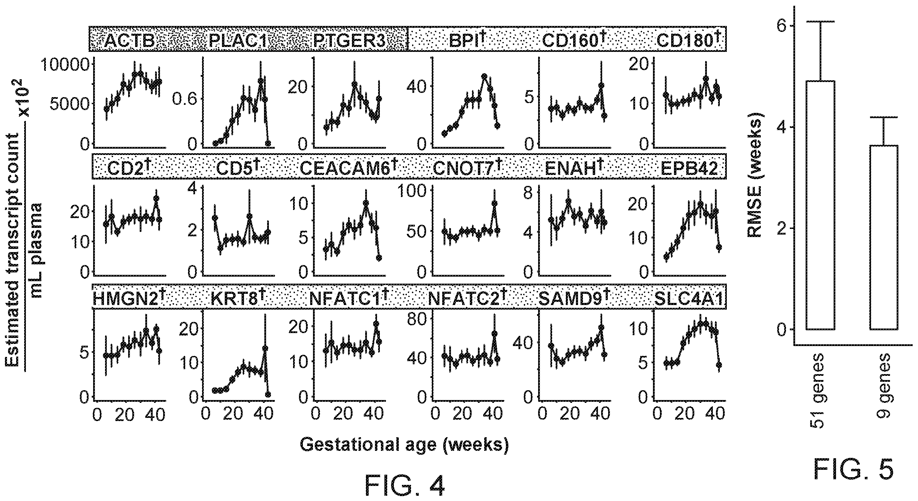

In one aspect, the invention provides a method for determining risk of preterm delivery by analyzing a maternal sample to determine an expression profile of a set of genes (*e.g*., cfRNA or protein) listed in TABLE 2, comprising SEQ ID NO: 17 and two or more genes selected from SEQ ID NOS: 10, 11, 13, 15, 16, and-18 . In one embodiment the panel includes at least three genes including SEQ ID NO: 17 and two or more genes selected from SEQ ID NOS:10-16 and SEQ ID NOS: 18-19 and one, at least 2, or at least 3 genes from TABLE 2. In some embodiments, the profile panel can include at least three genes including SEQ ID NO: 17 and two or more genes selected from SEQ ID NOS:10-16 and SEQ ID NOS: 18-19 and at least 1, 2, 3, 4, 5, 6, 7, 8, 9, 10, 11, 12, 13, 14, 15, 16, 17, 18, 19, or 20 genes from TABLE 2. In some embodiments, the profile panel can include exactly three genes including SEQ ID NO: 17 and two or more genes SEQ ID NOS:10-16 and SEQ ID NOS: 18-19 and 1, 2, 3, 4, 5, 6, 7, 8, 9, 10, 11, 12, 13, 14, 15, 16, 17, 18, 19, or 20 genes from TABLE 2. In some embodiments the profile panel includes fewer than 100 genes, sometimes fewer than 50 genes, sometimes fewer than 20 genes, sometimes fewer than 15 genes, sometimes fewer than 10 genes, and sometimes fewer than 5 genes. In certain approaches the profile panel comprises a number of genes in the range 1-100 genes, 1-50 genes, 1-25 genes, 3-100 genes, 3-50 genes, 3-25 genes, or 3-10 genes. In one approach at least one of the genes in the profile panel does not listed in FIG. 3A and/or FIG. 3B and/or FIG. 4 of US Patent Publication No. 2013/0252835.

In one approach a maternal sample is obtained at a specified week of pregnancy and the maternal expression profile is compared to a time matched reference profile, wherein the time matched reference profile is characteristic of a full-term pregnancy profile at the specified week of pregnancy. In one approach a maternal sample is obtained at a specified trimester (e.g, first, second or third trimester) of pregnancy and the maternal expression profile is compared to a time matched reference profile, wherein the time matched reference profile is characteristic of a full-term pregnancy profile at the specified trimester of pregnancy. Significant deviations of the maternal profile from the reference profile is indicative that the woman as at elevated risk of preterm delivery. It will be immediately apparent that, in an alternative approach, a maternal sample is obtained at a specified week of pregnancy and the maternal expression profile is compared to a time matched reference profile, wherein the time matched reference profile is characteristic of a preterm pregnancy profile at the specified week of pregnancy. Significant similarities between the maternal profile and the reference profile is indicative that the woman as at elevated risk of preterm delivery. In one approach a machine learning model is used to compare the maternal profile and the reference profile.

### 4. ILLUSTRATIVE METHODS AND EMBODIMENTS USING CIRCULATING PROTEIN EXPRESSION

### 4.1 Isolation Of Proteins From Maternal Blood or Urine

Proteins can be isolated from a maternal sample using methods well known in the art. In one appropach total protein is from a maternal blood fraction or urine and assayed for the presence and/or quantity of particular proteins. In one approach an assay is carried out using a protein fraction (e.g., a fraction enriched for protein(s) of interest. In one approach an assay is carried out using one or more purified proteins. Isolation and fractionation of proteins can be performed using fractionation by molecular weight, protein charge, solubility/hydrophobicity, protein isoelectric point (pl), affinity purification (e.g., using a an antiligand, such as an antibody or aptamer, specific from a protein among other methods. Kits for isolating proteins from blood are known and are commercially available (e.g., Total Protein Assay Kit from ITSIBiosciences, Catalog No.: K-0014-20). Kits for isolating proteins from plasma/serum are known and are commercially available (e.g., Antibody Serum Purification Kit (Protein A) from Abcam, Catalog No.: ab109209). Kits for isolating protein and RNA from the sample are also known (e.g., Protein and RNA Isolation System (PARIS) from Thermo Fisher Scientific, Catalog No. AM1921).

### 4.2 Detecting Proteins from a Maternal Sample

Specific proteins from a maternal sample can be identifed and/or quantified using well know methods, including enzyme-linked immunoadsorbent assay (ELISA); radioimmunoassay (RA) (see, e.g., Anthony et al., Ann. Clin. Biochem., 34:276-280 (1997) describing detection of low levels of protein undetectable using comparable ELISA conditions); proximity ligation and proximity extension assays (see, e.g., US Pat. Pub. Nos. 20170211133; 20160376642; 20160369321; 20160289750: 20140194311; 20140170654; 20130323729; and 20020064779), protein binding arrays (e.g., antibody or aptamer arrays), mass spectroscopy (see, e.g., Han, X. et al.(2008). Mass Spectrometry for Proteomics. Current Opinion in Chemical Biology, 12(5), 483-490. http://doi.org/10.1016/j.cbpa.2008.07.024; Serang, O et al (2012). A review of statistical methods for protein identification using tandem mass spectrometry. Statistics and Its Interface, 5(1), 3-20). Any suitable method may be used.

Protein binding arrays may be used to detect and quantitate proteins, including but not limited to antibody based arrays and aptamer based arrays (see, e.g., Gold L, et al. (2010) Aptamer-Based Multiplexed Proteomic Technology for Biomarker Discovery. PLoS ONE5(12): e15004. https://doi.org/10.1371/journal.pone.0015004). An antibody array (also known as antibody microarray) is a specific form of protein array. In this technology, a collection of capture antibodies are fixed on a solid surface such as glass, plastic, membrane, or silicon chip, and the interaction between the antibody and its target antigen is detected (see, e.g., US Patent Nos.: 4,591,570; 4,829,010; and 5,100,777). Antibody arrays can be used to detect protein expression from various biological fluids including serum, plasma, urine and cell or tissue lysates (see, Knickerbocker T., MacBeath G. Detecting and Quantifying Multiple Proteins in Clinical Samples in High-Throughput Using Antibody Microarrays. In: Wu C. (eds) Protein Microarray for Disease Analysis. Methods in Molecular Biology (Methods and Protocols), vol 723. Humana Press (2011)).

Kits for performing antibody arrays are known and are commercially available (e.g., custom designed antibody arrays or predetermined antibody arrays from RayBiotech, Norcross, GA).

### 5. STATISTICAL ANALYSIS

A maternal expression profile may be compared with a reference profile(s) in a variety of ways. In one approach, a comparison between two data sets is performed to determine whether one data set differs or is similar to another data set, e.g., to within statistical significance. In one embodiment, a first data set can comprise a maternal expression profile, and a second data set comprises a reference profile, where the first and second data sets include one or more data points (for example, median values) for gene expression data for one or more genes, collected over one or more time points during pregnancy (e.g., once a week or once a trimester during the course of the pregnancy). In some embodiments, the second data set comprises a plurality of data points from a preterm maternal sample or a maternal sample having a known gestational age.

Accordingly, a maternal data set can be a measured value of an expression level of one or more genes, where the expression level can be determined from individual expression values for each of the genes, e.g., as an average, weighted average, or median of the individual expression levels. In other embodiments, the individual expression levels can be treated as different dimensions of a multi-dimensional data point, e.g., for use in clustering. For determining a gestational age or time to delivery, the comparison can be between a measured expression level(s) of a maternal sample and the reference expression level(s) of each of a plurality of reference having different known gestational ages, thereby identifying a group or representative data point that is closest (e.g., least difference in a distance between the measured expression level(s) and the reference expression level(s)). The known gestational age of the closest reference sample (or representative data point of a group of reference samples all having a same gestational age) can be used as the gestational age or time to delivery of the maternal sample. Such a comparison can be performed by comprising the measured expression level(s) to a gestational function that is determined from the reference samples, e.g., a linear function that defines a functional relationship between the expression level(s) (e.g., in a multi-dimensional space when individual expression levels correspond to different dimensions or in a 2D-plot when individual expression levels are combined to provide a single metric).

In embodiments where a discrimination is made between term and preterm samples, the comparison can involve determining whether the measured expression level(s) are more similar to preterm reference level(s) or term reference level(s). Such a comparison can involve determining which cluster of reference levels is closest to the measured expression level(s). One or more values may be used for determining whether the measured expression level(s) are sufficiently close (e.g., as measured by a distance or a weight distance where differences along one dimension are weighted differently) for the measured level(s) to be considered part of either cluster of term or preterm samples. An indeterminate classification may result if the expression level(s) are not sufficiently close. A threshold can be used to determine whether the measured expression levels are sufficiently close to reference expression levels of a term or preterm population. A threshold can be selected based on a desired sensitivity and specificity, as will be apparent to one skilled in the art.

To determine the reference level(s), a set of training samples can be labeled with different classifications, e.g., term or preterm. Then, the reference levels can be chosen as being representative of a classification or as values that separate the different classifications, e.g., as cutoffs for assigning different classifications to a new sample. A machine learning technique can analyze different expression levels of different genes to determine which set of expression levels (features) provide the best discrimination for an optimized set of reference levels. A tradeoff between specificity and sensitivity can be optimized, e.g., by a ROC (receiver operating characteristic) curve. In some embodiments, a plurality of training samples, each labeled as preterm or full-term, can be obtained. In some embodiments, training samples are labeled as nulliparous, multiparous women, carrying male fetus, carrying female fetus, or the like. One or more measured expression levels for the panel of genes can be obtained for each of the plurality of training samples. Using the machine learning technique (e.g., by optimizing a cost function as defined by the model), the one or more reference expression levels can be iteratively adjusted to increase a number of the training samples that are classified correctly as a result of comparing the one or more measured expression levels to the one or more reference expression levels.

In some aspects, the first and second data sets can be analyzed to establish relative differences or similarities (e.g., fold increase or fold decrease) between the data sets (e.g., the expression level(s) of the data sets). Such a procedure can be performed when a single expression level is determine for a panel of genes. In another aspect, a pairwise comparison of expression level(s) at each time point for each gene across the duration of pregnancy can be used to identify which reference level(s) are most similar, where each set of reference level(s) can correspond to a different gestational age. In some embodiments, the pairwise comparison (e.g., pairwise between expression levels of different genes and/or between reference level(s) at different times) can include statistical analysis via a range of statistical methodologies, including but not limited to Fisher's exact test, Wilcox rank test, permutation test, linear regression, generalized linear models and quasi-likelihood tests coupled with the appropriate multiple hypothesis correction (e.g., Benjamini Hochberg).

In one embodiment, differentiating gene activity (e.g., between preterm and term maternal samples, see Example 1 and FIGS 11A-11D) across the pregnancy can include using a quantile adjusted conditional maximum likelihood method, a generalized linear model (GLM) likelihood ratio test, and/or a quasi-likelihood F-test implemented in R using the edgeR software (Bioconductor, available at https://bioconductor.org/ packages/release/ bioc/html/edgeR.html).

In another aspect, a sample data set can be analyzed using a random forest model (see, e.g., Chen and Ishwaran, Genomics, 99:323-329 (2012)) that was generated using the second data set. See Examples. Random forest is a form of machine learning that selects training sets randomly for building multiple models (e.g., decision trees or regression models) and uses the outputs of this ensemble of models to determine a final output (e.g., via majority voting for a term/preterm classification or an average when determining gestational age or time to delivery). Each model can have the same or different features (e.g., expression levels of genes), but have different reference levels as determined from the different training sets that are randomly selected. It will be recognized that other techniques of machine learning can be used to compare two data sets, including but not limited to, support vector machines, elastic net, lasso or neural networks. It will also be apparent that machine learning models (e.g., supervised machine learning; see, for example Mohri et al. (2012) Foundations of Machine Learning, The MIT Press) can be developed to account for particular attributes of a population such as ethnicity and that multiple models can be prepared based on different needs (e.g., an Eastern European model versus a North African model).

In one aspect, a machine learning model (e.g., to predict gestational age or time to delivery) can be prepared as follows:
(1) Curate a labeled training set (e.g., where gestational age of each sample is known);
(2) Iterate through selecting features of interest (e.g., recursive feature selection);
(3) Build a regression model (e.g., random forest) based on the selected features; and
(4) Select a regression model and feature subset using cross validation data (e.g., by withholding part of the training set and determining how accurately the regression model evaluated the withheld data).

In one embodiment, once the regression model is prepared, it can be saved and used for future data interpretations. In other embodiments, a single regression model can be determined, e.g., by fitting a line or a curve to a set of measured expression level(s) that are measured at known gestational ages. The regression model can be considered a gestational function, e.g., when a model (e.g., a linear or non-linear function) is fit to expression levels of a plurality of calibration samples having measured expression levels and of which a gestational age is known. Accordingly, the comparison of the maternal expression profile to the reference profile can be performed by comparing the maternal expression profile to a gestational function that provides a gestational age based on an input of one or more expression levels.

In another aspect, the first and second data sets can be analyzed using SAMS (Scoring Algorithm of Molecular Subphenotypes) available at http://statweb.stanford.edu/~tibs/SAM/ (see, Tusher et al., PNAS, 98:5116-5121 (2001)). SAMS is a classification algorithm of gene expression data generated from the calculation of two scores (e.g., an up score and a down score). In one embodiment, a maternal expression profile data set of the instant invention (e.g., cfRNAs) can be compared to a reference expression profile data set and a maternal sample having an up score above the median value (as compared to the reference expression profile) and a down score above the median value (as compared to the reference expression profile) can be classified as statistically significant (see., e.g., Herazo-Maya, Lancet Respir Med, September 20, (2017) doi:org/10.1016/52213-2600(17)30349-1 and Dinu et al., BMC Bioinformatics, 8:242 (2007)). Other evaluations of a first data set and a second data set using SAMS can be performed according to the SAMS user manual (available at http://www-stat.stanford. edu/~tibs/SAM/sam.pdf).

Various additional statistical analyses exist for the comparison of a first and second data set directed to gene expression data (e.g., preterm data set versus a maternal sample) including for example, methods set forth by Efron and Tibshirani (On Testing the Significance of Sets of Genes. Ann Appl. Stat., 1. 107-129 (2007) and Zhao et al. (Gene expression profiling predicts survival in conventional renal cell carcinoma, PLOS Medicine, 3. E13. 13. 10.1371/journal.pmed.0030013. (2006)).

As discussed above, maternal expression profiles may be compared to reference profiles and a measure of similarity or difference may be made. In one approach, comparing a maternal expression profile to a reference profile includes compiling gene expression data (e.g., the number or relative number of transcripts of a specified cfRNA sequence on a computer-readable medium) and processing said data on said computer to identify degrees of similarity and difference between said profiles.

### 6. MEDICAL INTERVENTIONS FOR WOMEN AT RISK OF PRETERM DELIVERY

Women identified as at risk for preterm delivery may elect medical interventions (e.g., progesterone supplementation, cervical cerclage), behavioral changes (smoking cessation), or ultrasound imaging to monitor and reduce the likelihood of preterm delivery or to extend the pregnancy for as long as possible. See Newnham et al. "Strategies to Prevent Preterm Birth." Frontiers in Immunology 5 (2014):584. Progesterone may be used to treat and/or prevent the onset of preterm labor in women identified as at risk for preterm delivery. A pregnant woman may be administered an amount of progesterone, e.g., as a vaginal gel, that is sufficient to prolong gestation by delaying the shortening or effacing of cervix. The administration can be as infrequent as weekly, or as often as 4 times daily. Antibiotic treatment (amoxicillin, ampicillin, erythromycin, azithromycin, and cephalosporin) is indicated in some women with premature rupture of the membranes (PROM), a precursor of premature delivery, and may be administered to women identified as at risk for preterm delivery. When a woman is identified as at risk of preterm delivery the medical provider may recommend an ultrasound examination at least once per four week period, biweekely, or weekly.

### 7. THERANOSTIC AND PROGNOSTIC USES OF THE INVENTION FOR WOMEN AT RISK OF PRETERM DELIVERY

In some embodiments, the methods described herein are used for theranosis. In one approach a first maternal expression profile is obtained from a woman at risk of preterm delivery at a first point in time, medically appropriate steps (e.g., medical interventions) are initiated or carried out, and then a second maternal expression profile is obtained from the woman at a second point in time. Each maternal expression profile is compared to an appropriate reference profile (e.g., time matched, population matched, etc.). If the difference between the second maternal expression profile and the appropriate corresponding reference profile is less than the difference between the first maternal expression profile and its appropriate corresponding reference profile this is an indication that the steps carried out have a beneficial therapeutic effect. In some cases, the first and second maternal expression profiles are compared to the same reference profile. In one approach the process is carried out without any medical intervention, in which case a spontaneous improvement may be observed.

In some embodiments, the methods described herein are used for prognosis. It is believed that certain maternal expression profiles are indicative of particular prognoses. For example, certain maternal expression profiles may be used to estimate time until preterm delivery (absent intervention). Reference profiles for this purpose can be generated from sub-populations grouped by specific pregnancy outcomes (dates of prematurity), by genetic risk, or by phenotypic factors such as age and previous pregnancy history. The methods disclosed herein may also be used for identifying and monitoring fetuses having congenital defects; in some cases the methods may be used to inform decisions about in utero treatment. Maternal expression profiles can be used to estimate time to delivery and gestational age for the fetus, and the results used for providing advice or treatment for either the mother or the fetus. Similarly, with appropriately chosen genes such profiles can be used to estimate the risk of adverse events such as preterm delivery.

### 8. COMPUTER IMPLEMENTED METHODS & DATABASE OF REFERENCE VALUES

Methods of the invention may be implemented using a computer-based system. As used herein, "a computer-based system" refers to the hardware means, software means, and data storage means used to analyze the information of the present invention. The minimum hardware of the computer-based systems of the present invention comprises a central processing unit (CPU), input means, output means, and data storage means. A skilled artisan can readily appreciate that any one of the currently available computer-based system are suitable for use in the present invention. The data storage means may comprise any manufacture comprising a recording of the present information as described above, or a memory access means that can access such a manufacture.

In some embodiments, a database comprising reference profiles is used in methods of the invention. In some embodiments, a database comprising expression data from a plurality of women, and optionally different subpopulations of women, is provided. Accordingly, aspects of the invention provide systems and methods for the use and development of a database. In some approaches the database is used in combination with an algorithm that enables generation of new reference profiles selected based on characteristics of an individual woman.

Any of the computer systems mentioned herein may utilize any suitable number of subsystems. In some embodiments, a computer system includes a single computer apparatus, where the subsystems can be the components of the computer apparatus. In other embodiments, a computer system can include multiple computer apparatuses, each being a subsystem, with internal components. A computer system can include desktop and laptop computers, tablets, mobile phones and other mobile devices.

A computer system can include a plurality of the same components or subsystems, e.g., connected together by external interface, by an internal interface, or via removable storage devices that can be connected and removed from one component to another component. In some embodiments, computer systems, subsystem, or apparatuses can communicate over a network. In such instances, one computer can be considered a client and another computer a server, where each can be part of a same computer system. A client and a server can each include multiple systems, subsystems, or components.

Aspects of embodiments can be implemented in the form of control logic using hardware circuitry (e.g. an application specific integrated circuit or field programmable gate array) and/or using computer software with a generally programmable processor in a modular or integrated manner. As used herein, a processor can include a single-core processor, multi-core processor on a same integrated chip, or multiple processing units on a single circuit board or networked, as well as dedicated hardware. Based on the disclosure and teachings provided herein, a person of ordinary skill in the art will know and appreciate other ways and/or methods to implement embodiments of the present invention using hardware and a combination of hardware and software.

Any of the software components or functions described in this application may be implemented as software code to be executed by a processor using any suitable computer language such as, for example, Java, C, C++, C#, Objective-C, Swift, or scripting language such as Perl or Python using, for example, conventional or object-oriented techniques. The software code may be stored as a series of instructions or commands on a computer readable medium for storage and/or transmission. A suitable non-transitory computer readable medium can include random access memory (RAM), a read only memory (ROM), a magnetic medium such as a hard-drive or a floppy disk, or an optical medium such as a compact disk (CD) or DVD (digital versatile disk), flash memory, and the like. The computer readable medium may be any combination of such storage or transmission devices.

The databases may be provided in a variety of forms or media to facilitate their use. "Media" refers to a manufacture that contains the expression information of the present invention. The databases of the present invention can be recorded on computer readable media, e.g. any medium that can be read and accessed directly by a computer (e.g., an internet database). Such media include, but are not limited to: magnetic storage media, such as floppy discs, hard disc storage medium, and magnetic tape; optical storage media such as CD-ROM; electrical storage media such as RAM and ROM; and hybrids of these categories such as magnetic/optical storage media. One of skill in the art can readily appreciate how any of the presently known computer readable media can be used to create a manufacture comprising a recording of the present database information. "Recorded" refers to a process for storing information on computer readable medium, using any such methods as known in the art. Any convenient data storage structure may be chosen, based on the means used to access the stored information. A variety of data processor programs and formats can be used for storage, e.g. word processing text file, database format, etc.

Such programs may also be encoded and transmitted using carrier signals adapted for transmission via wired, optical, and/or wireless networks conforming to a variety of protocols, including the Internet. As such, a computer readable medium may be created using a data signal encoded with such programs. Computer readable media encoded with the program code may be packaged with a compatible device or provided separately from other devices (e.g., via Internet download). Any such computer readable medium may reside on or within a single computer product (e.g. a hard drive, a CD, or an entire computer system), and may be present on or within different computer products within a system or network. A computer system may include a monitor, printer, or other suitable display for providing any of the results mentioned herein to a user.

Any of the methods described herein may be totally or partially performed with a computer system including one or more processors, which can be configured to perform the steps. Thus, embodiments can be directed to computer systems configured to perform the steps of any of the methods described herein, potentially with different components performing a respective step or a respective group of steps. Although presented as numbered steps, steps of methods herein can be performed at a same time or at different times or in a different order. Additionally, portions of these steps may be used with portions of other steps from other methods. Also, all or portions of a step may be optional. Additionally, any of the steps of any of the methods can be performed with modules, units, circuits, or other means of a system for performing these steps.

### 9. PRIMERS, PROBES, AND COMPOSITIONS

Primers and probes that specifically hybridize to or amplify cfRNA from placental genes (including genes in TABLE 1) and other informative genes (including genes in TABLE 1 and TABLE 2) may be used in the practice of aspects of the invention. In particular, useful primers and probes include those that specifically hybridize to or amplify SEQ ID NOS: 1-19. These primers and probes are used for amplification (including multiplex PCR, multiplex RT-qPCR, or other amplification methods), for reverse transcription, for construction of sequencing libraries (e.g., RNA-seq libraries), for addition of adaptor sequences, for hybrid capture of RNAs of interest, for construction nucleic acid arrays, for primer extension and for other uses known to the practitioner with knowledge of the art. It is well within the ability of persons of ordinary skill in the art to design probes and primers for their intended uses, taking into account methods of amplification (e.g., addition of adaptors or universal primers), target sequence composition, base composition, avoiding artifacts such as primer dimer formation, as well as the fragmented nature of cfRNA.

For example, it is within the ability of persons of ordinary skill in the art to use SEQ ID NOS:1-19 to design primers, primers pairs, and probes that are specific for each gene and work for their intended purposes (e.g., use in a multiplex reaction). It will be appreciated that for each RNA transcript there are many different primers and combinations of primers that can amplify at least a portion of the transcript. A person of skill in the art can therefore design primer combinations to amplify informative sequences of any of SEQ ID NOS:1-19 or any combination thereof, as well as other gene sequences identified in TABLES 1 and 2. Exemplary primers and probes are described in TABLES 3-5. Probes may be nucleic acid probes, such as RNA or DNA probes. Primers or probes may be immobilized (e.g., for capture based enrichment) or detectably labeled (e.g., with fluorescent, enzymatic, or chemiluminescent moieties or the like).

### 9.1 Gestational Age Or Time To Delivery Compositions (not claimed)

Primers for multiplex amplification of at least 3 and not more than 50, optionally no more than 25, optionally no more than 10 genes, selected from genes in TABLE 1 are described. Primers for multiplex amplification of at least 3 mRNA transcripts provided in TABLE 1 are described. Primers for multiplex amplification of any combination of at least 3 mRNA transcripts selected from SEQ ID NOS:1-9 are described. The primers may by for multiplex amplification, wherein the primers may comprise at least one pair, and optionally three or more primer pairs. Exemplary primer pairs are provided in TABLE 3. The primers for multiplex amplification may comprise at least three and no more than 100 primer pairs, optionally no more than 50, optionally no more than 25, optionally no more than 10 primer pairs selected from any of the primer pairs provided in TABLE 3.

Compositions comprising primer(s) or primer pair(s) as described above are described. The composition may be an admixture. The composition may be a solution. The composition may additionally contain one or more of (a) maternal cfRNA, (b) buffer, (c) enzymes (e.g., one or a combination of reverse transcriptase, DNA polymerase, RNA or DNA ligase), (d) dNTPs.

In one aspect a composition is described, comprising (1) cfRNAs with cfRNA sequences corresponding to at least 2 genes in TABLE 1, or amplicons of, or cDNAs from, said cfRNA sequences and (2) primers for amplifying said cfRNA sequences or amplicons or cDNAs, or probes for detecting said cfRNA sequences or amplicons or cDNAs, with the proviso that the composition does not comprise primers for amplifying more than a threshold number of different genes, amplicons or cDNAs; and does not comprise probes for detecting more than the threshold number of different cfRNA sequences or amplicons or cDNAs. The composition may not comprise cfRNAs with cfRNA sequences corresponding to more than the a threshold number of different genes from the human genome, or amplicons of, or cDNAs from more than the threshold number of different genes. The threshold number may be 200. The threshold number may be 150. Tthe threshold number may be 100. The threshold number may be 50. The threshold number may be 25.

Nucleic acid arrays comprising primer(s), primer pair(s), or probes as described above are provided.

### 9.2 Preterm Risk Compositions

Primers for multiplex amplification of at least 3 and no more than 100 genes, optionally no more than 50, optionally no more than 25, optionally no more than 10 genes, selected from genes in TABLE 2 are described. Primers for multiplex amplification of at least 3 mRNA transcripts provided in TABLE 2 (i.e., RefSeq identifiers) are described. Primers for multiplex amplification of any combination of at least 3 mRNA transcripts selected from SEQ ID NOS:10-19, or, alternatively at least 3 mRNA transcripts selected from SEQ ID NOS: 10, 11, 13, and 15-18 are described. The primers may be for multiplex amplification, wherein the primers comprise at least one pair, and optionally three or more primer pairs. Exemplary primer pairs are provided in TABLE 3. The primers for multiplex amplification may comprise at least three and no more than 100 primer pairs, optionally no more than 50, optionally no more than 25, optionally no more than 10 pairs selected from any of the primer pairs provided in TABLE 3.

Compositions comprising primer(s) or primer pair(s) as described above are described. The composition may be an admixture. The composition may be a solution. The composition may additionally contain one or more of (a) maternal cfRNA, (b) buffer, (c) enzymes (e.g., reverse transcriptase, DNA polymerase, RNA or DNA ligase), (d) dNTPs.

Kits comprising primer(s) or primer pair(s) as described above packaged together are described. In one approach, a mixture of different primers are combined in a single mixture. In another approach, primers specific for individual cfRNAs are packaged together in separate vials. The kit may additionally contain one or more of (a) maternal cfRNA, (b) buffer, (c) enzymes (e.g., reverse transcriptase, DNA polymerase, RNA or DNA ligase), (d) dNTPs.

In one aspect a composition is provided, comprising (1) cfRNAs with cfRNA sequences corresponding to at least 2 genes in TABLE 2, or amplicons of, or cDNAs from, said cfRNA sequences and (2) primers for amplifying said cfRNA sequences or amplicons or cDNAs, or probes for detecting said cfRNA sequences or amplicons or cDNAs, with the proviso that the composition does not comprise primers for amplifying more than a threshold number of different genes, amplicons or cDNAs; and does not comprise probes for detecting more than the threshold number of different cfRNA sequences or amplicons or cDNAs. Tthe composition may not comprise cfRNAs with cfRNA sequences corresponding to more than the a threshold number of different genes from the human genome, or amplicons of, or cDNAs from more than the threshold number of different genes. The threshold number may be 200. The threshold number may be 150. The threshold number may be 100. The threshold number may be 50. The threshold number may be 25.

Nucleic acid arrays comprising primer(s) or primer pair(s) as described above are described.

### 10. METHODS

This section describes implementation of the methods for determination of gestational age and risk of preterm delivery. Examples in this section are intended as illustrations and are in no sense limiting.

In one approach a maternal sample(s) is collected, frozen, and shipped to a centralized laboratory for analysis. In one approach methods of the invention are carried out in a local medical facility (e.g., hospital lab) optionally using a kit for isolation of cfRNA, production of cDNA, qPCR and/or sequencing. In one approach the kit includes reagent for cfRNA isolation. The use of a standardized kit is advantageous in ensuring uniformity of sample collection, cfRNA isolation, and analysis by qPCR or transcriptome sequencing. The kit may contain reagents for cfRNA, production of cDNA, qPCR and/or sequencing as well as primers or probes described herein for determining expression levels of cfRNA transcripts or combinations of transcripts described herein. In one approach cfRNA, cDNA, or a library is produced and shipped to a centralized laboratory for analysis.

In one approach a maternal sample(s) is collected and an expression profile is determined using a distributed system including client systems and server systems communicating over a computer network server-client, frozen, and shipped to a centralized laboratory for analysis. The server system may comprise databases of reference profiles and may receive data (e.g., expression profile information) from a client system. The expression profile information from the patient is compared to the reference profile using a computer product, e.g., comprising a computer readable medium storing a plurality of instructions for controlling a computer system to perform a method of the invention. the method of any one of the preceding claims. The databases of reference profiles may be produced using the machine learning approaches described herein. Advantageously, as expression profiles from individual patients is collected that information may be used as training data. This may be particularly useful when training and validation data are collected from demographically distinct patient populations (e.g., populations identified by age, race or ethnicity, geographical location, or other criteria).

Patient expression profiles will be most useful when they are tied to particular outcomes (e.g., term delivery or preterm delivery) or gestational age at birth. Thus, in one aspect the invention involves (1) collecting cfRNA from a pregnant woman one or multiple times during pregnancy, determining an expression profile using the cfRNA (i.e., an expression profile corresponding to a set of genes identified herein, e.g., genes from TABLE 1, TABLE 2, or TABLE 6 or combinations or subsets described herein); and recording the expression profile, e.g., on a suitable non-transitory computer readable medium; and then (2) determining the delivery date for the woman, categorizing the delivery as term or preterm (and if preterm, by how many days) or otherwise characterizing the outcome of the pregnancy, and (3) associating the information in (2) with the expression profiles in (1), e.g., by linking the information and expression profile(s) in the computer readable medium.

### Determination of gestational age (not claimed)

In one approach a method performed using a computer for estimating gestational age of a fetus is provided comprising: (a) obtaining one or more expression profiles from a maternal sample of a pregnant woman carrying a fetus, wherein the expression profile(s) corresponds to the expression of cfRNA transcripts from a first panel of genes; (b) comparing, using a computer system, the expression profile(s) to one or more reference profile(s) characteristic of a defined gestational age(s) to estimate the gestational age of the fetus, wherein the reference profile(s) characteristic of the defined gestational age(s) are determined using a machine learning model that analyzes first training samples that are cfRNA expression profiles labeled with a defined gestational age; (c) updating, using the computer system, the reference profile(s) by: (1) receiving second training samples, wherein the second training samples are cfRNA expression profiles labeled with a defined gestational age, and (2) iteratively adjusting the reference profile(s) via a machine learning model to increase the number of the first and second training samples that are classified correctly. The reference profiles can form a line or curve or be discrete values. the first panel of genes may comprise any combination of genes disclosed herein as predictive of gestational age, including placental genes, placental genes listed in Table 1, and at least 1, at least 2, at least 3, at least 4, at least 5, at least 6, at least 7, at least 8, or 9 genes selected from CGA [SEQ ID NO:1], CAPN6 [SEQ ID NO:2], CGB [SEQ ID NO:3], ALPP [SEQ ID NO:4], CSHL1 [SEQ ID NO:5], PLAC4 [SEQ ID NO:6], PSG7 [SEQ ID NO:7], PAPPA [SEQ ID NO:8], and LGALS14 [SEQ ID NO:9].

Also provided is a computer system comprising: (a) a database comprising reference profile(s), each including a level of expression in a population of pregnant women of cfRNA transcripts corresponding to a first panel of genes and corresponding to a defined gestational age; (b) a user interface configured to interact with a client computer over a network and to receive expression profile(s) including the level of expression in a pregnant woman carrying a fetus of cfRNA transcripts corresponding to the first panel of genes; and (c) one or more processors configured to analyze the reference profile and expression profile, including comparing the reference profile(s) and expression profile(s) to determine gestational age of the fetus; and (d) a network interface that transmits the gestational age of the fetus to the client computer. The reference profile(s) and expression profile(s) may comprise expression levels of a panel of cfRNAs in any combination disclosed herein, including transcripts from placental genes; placental genes listed in Table 1; and at least 1, at least 2, at least 3, at least 4, at least 5, at least 6, at least 7, at least 8, or 9 genes selected from CGA [SEQ ID NO:1], CAPN6 [SEQ ID NO:2], CGB [SEQ ID NO:3], ALPP [SEQ ID NO:4], CSHL1 [SEQ ID NO:5], PLAC4 [SEQ ID NO:6], PSG7 [SEQ ID NO:7], PAPPA [SEQ ID NO:8], and LGALS14 [SEQ ID NO:9].

### Risk of preterm delivery

In one approach a method performed using a computer for assessing risk of preterm delivery by a pregnant woman is provided comprising: (a) obtaining one or more expression profiles from a maternal sample of a pregnant woman, wherein the expression profile(s) corresponds to the expression of a plurality of cfRNA transcripts from a first panel of genes; (b) comparing, using a computer system, the expression profile(s) to one or more reference profile(s) characteristic of a woman with (a) a high risk of preterm delivery or (b) a low risk of preterm delivery, or characteristic of a woman with a defined length of pregnancy, wherein the one or more reference profiles are determined using a machine learning model that analyzes first training samples that are cfRNA expression profiles preterm or full-term, or labeled with a length of pregnancy; and (c) updating, using the computer system, the one or more reference profile) by: (1) receiving second training samples, wherein the second training samples are cfRNA expression profiles labeled as preterm or full-term or labeled with a length of pregnancy, and (2) iteratively adjusting the one or more reference profile( via a machine learning model to increase the number of the first and second training samples that are classified correctly, wherein the first panel of genes comprises RAB27B and two or more genes selected from PPBP, DAPP1, RGS18, CLCN3, MAP3K7CL, MOB1B, POLE2, LYPLAL1, and TBC1D15. The reference profiles can form a line or curve or be discrete values. In some embodiments the first panel of genes comprises RAB27B and two or more genes selected from PPBP, DAPP1, RGS18, CLCN3, MAP3K7CL, MOB1B, POLE2, LYPLAL1, and TBC1D15 and any combination of any combination of genes disclosed herein as predictive of risk of premature delivery, including genes listed in Table 1, and at least 2, at least 3, at least 4, at least 5, at least 6, at least 7, at least 8, or 9 genes selected from CGA [SEQ ID NO:1], CAPN6 [SEQ ID NO:2], CGB [SEQ ID NO:3], ALPP [SEQ ID NO:4], CSHL1 [SEQ ID NO:5], PLAC4 [SEQ ID NO:6], PSG7 [SEQ ID NO:7], PAPPA [SEQ ID NO:8], and LGALS14 [SEQ ID NO:9] or at least least 2, at least 3, at least 4, at least 5, at least 6, or 7 genes selected from CLCN3 [SEQ ID NO:10], DAPP1 [SEQ ID NO:11], PPBP [SEQ ID NO:13], MAP3K7CL [SEQ ID NO:15], MOB1B [SEQ ID NO:16], RAB27B [SEQ ID NO:17], and RGS18 [SEQ ID NO:18]. In some embodiments the first panel of genes comprises at least one combination selected from (1) RGS18; RAB27B; PPBP; (2) DAPP1; RAB27B; PPBP; (3) RAB27B; MOB1B; PPBP; (4) RAB27B; PPBP; MAP3K7CL; (5) RAB27B; PPBP; CLCN3.

For determining risk of preterm delivery maternal samples can be labeled "preterm" and "term"; or with the gestational age of the child at birth; or with the length of the pregnancy (e.g., week of delivery), combinations of these, or labels suitable for quantitatively or qualitatively distinguishing a full-term delivery from a preterm delivery.

Also provided is a computer system comprising: (a) a database comprising one or more reference profiles, each including (i) a level of expression in a population of pregnant women of cfRNA transcripts corresponding to a first panel of genes and (ii) a risk of preterm delivery; (b) a user interface configured to interact with a client computer over a network and to receive one or more expression profiles including the level of expression in a pregnant woman of cfRNA transcripts corresponding to the first panel of genes; and (c) one or more processors configured to analyze the one or more reference profile and the one or more expression profile, including comparing the one or more reference profiles and one or more expression profiles to determine the risk of preterm delivery; and (d) a network interface that transmits the risk of preterm delivery to the client computer, wherein the first panel of genes comprises RAB27B and two or more genes selected from PPBP, DAPP1, RGS18, CLCN3, MAP3K7CL, MOB1B, POLE2, LYPLAL1, and TBC1D15. In some embodiments the reference profile(s) and expression profile(s) comprise expression levels of a panel of cfRNAs in any combination disclosed herein, including genes listed in Table 1 and at least 2, at least 3, at least 4, at least 5, at least 6, at least 7, at least 8, or 9 genes selected from CGA [SEQ ID NO:1], CAPN6 [SEQ ID NO:2], CGB [SEQ ID NO:3], ALPP [SEQ ID NO:4], CSHL1 [SEQ ID NO:5], PLAC4 [SEQ ID NO:6], PSG7 [SEQ ID NO:7], PAPPA [SEQ ID NO:8], and LGALS14 [SEQ ID NO:9] or at least least 2, at least 3, at least 4, at least 5, at least 6, or 7 genes selected from CLCN3 [SEQ ID NO:10], DAPP1 [SEQ ID NO:11], PPBP [SEQ ID NO:13], MAP3K7CL [SEQ ID NO:15], MOB1B [SEQ ID NO:16], RAB27B [SEQ ID NO:17], and RGS18 [SEQ ID NO:18].

### 11. EXAMPLES

### 12.1 Example 1 - Materials and Experimental Methods

### Sample collection

Blood samples from pregnant Danish women were collected weekly (high-resolution cohort) and at one time point during the second or third trimester from the University of Pennsylvania (preterm discovery cohort) and the University of Alabama at Birmingham (preterm validation cohort) under an Institutional Review Board-approved protocol. Women who participated in the study in Pennsylvania and Alabama were at elevated risk for spontaneous premature delivery. All women who delivered preterm except one patient from Pennsylvania (preeclampsia) experienced spontaneous preterm birth. As per the standard of care, all women with a history of preterm delivery received weekly progesterone injections. The blood samples were collected into EDTA-coated Vacutainer tubes (Becton Dickinson, NJ). Plasma was separated from blood using standard clinical blood centrifugation protocol.

### Cell-free RNA (cfRNA) Isolation

Cell-free RNA was extracted from 0.75 - 2 mL of plasma using Plasma/Serum Circulating RNA and Exosomal Purification kit (Norgen Biotek Corp, Canada, Catalog No. 42800). The residue of DNA was digested using Baseline-ZERO DNase (Epicentre, WI) and then cleaned by RNA Clean and Concentrator^{™}-5 kit (Zymo Research, CA). The resulting RNA was eluted to 12 µl in elution buffer.

### RT-qPCR Assay

RT-qPCR assays consist of two main reactions: reverse transcription/preamplification of extracted cfRNA and qPCR of pre-amplified cDNA. The primers for our gene panels were designed and synthesized by Fluidigm Corporation, CA (TABLE 3). Either 1-2 µl or 10 µl out of the 12 µl of total purified RNA was used for reverse transcription/preamplification reaction using the CellsDirect^{™} One-Step RT-qPCR Kit (Invitrogen, CA, Catalog No. 11753-100) and a pool of 96 primer pairs from TABLE 3. Preamplification was performed for 20 cycles and residual primers of the reaction were digested using exonuclease I treatment. Multiplex qPCR reactions of 96 samples for the 96 primer pairs were performed using 96x96 Dynamic Array Chip on BioMark System (Fluidigm Corp., CA). The BioMark Dynamic Array Chip loads individual samples (cDNA) and individual reagents (primer pairs) separately into wells on the Dynamic Array chip. The integrated fluidics circuit controllers push samples and reagents through channels until full; then coordinated releasing and closing of fluidic values allows mixing of samples and reagents into individual compartments within the chip. The 96x96 Dynamic Array Chip can simultaneously analyze up to 9,216 reactions. Threshold cycles (Ct values) of qPCR reactions were extracted using Fluidigm real-time PCR analysis software.

### cfRNA-Seq library preparation

A cell-free RNA sequencing library was prepared by SMARTer Stranded Total RNAseq - Pico Input Mammalian kit (Clontech, CA, Catalog No. 634413) from 6 µl of eluted cfRNA according to the manufacturer's manual. Short read sequencing was performed on Illumina NextSeq^{™} (2x75 bp) platform (Illumina, CA) to the depth of more than 10 million reads per samples.

### Statistical Analysis

### cfRNA-Seq differential expression analysis

28 samples (14 term and 14 preterm) cfRNA samples of the preterm discovery cohort were sequenced. The sequencing reads were mapped to human reference genome (hg38) using STAR aligner. Duplicates were removed by Picard and then unique reads were quantified using htseq-count. After preprocessing, 16 samples containing sequencing reads that mapped to more than 3000 genes were used for subsequent statistical analyses. Differentiating genes between term and preterm samples were identified using a quantile-adjusted conditional maximum likelihood method, a generalized linear model (GLM) likelihood ratio test, and a quasi-likelihood F-test implemented in R using the edgeR package.

### RT-qPCR sample analysis

Raw Cₜ values were quantified in absolute terms. Absolute quantification estimated the transcript counts contained in each sample based on cycle thresholds for known quantities of ERCC (FIG. 9). Estimated transcript counts were then adjusted for dilution, sample volume, and normalized by the volume of processed plasma.

### Multivariate random forest modeling

Recursive feature selection and model construction were performed in R using the caret package. Longitudinal data was smoothed using a 3-week centered moving average and divided into a 21 patient training set and a 10 patient validation set. Model selection was performed using 10-fold cross validation repeated 10 times.

### Expected delivery date estimation

Expected delivery dates were derived from random forest model predictions. Longitudinal data for this application were not smoothed using a centered moving average. For any given sampling period (second trimester (T2), third trimester (T3), or both (T2&T3), time to delivery estimates were shifted to a specified reference time point and then averaged using the median to establish an expected delivery date.

### Preterm biomarker candidate selection and validation

Absolute RT-qPCR values were normalized using a modified multiple of the median approach as applied in Rose and Mennuti (Fetal Medicine, West J Med., 1993; 159:312-317that is both time and epidemiologically invariant, allowing for consistent comparisons across cohorts of different ethnicities. At-term patient medians were quantified by trimester on a cohort level for each gene. Biomarker discovery was performed using the combined criterion of an effect size and significance value threshold calculated using Hedges' g and the Fisher exact test, respectively, as described in Sweeney et al. (*J. Pediatric Infect. Dis. Soc.,* 2017, doi: 10.1093/jpids/pix021). Genes were considered significantly different between cohorts using an effect size threshold of 0.8 and a false discovery rate (FDR) of 5%. Candidate gene biomarkers were then tested in unique combinations of 3 to estimate their ability to detect both true and false positives. Combinations with a true positive rate of greater than 0.75 and a false positive rate less than 0.05 were selected for further validation using an independent cohort. The ROC curve was based on the fraction of biomarker combinations where all genes showed a fold increase of at least 2.5 over median expression.

### 11.2 Example 2 - Longitudinal Data of Due Dates From Three Distinct Populations

We performed a high time-resolution study of normal human development by measuring cfRNA in blood from pregnant women longitudinally during each week of pregnancy. cfRNA provides a window into the phenotypic state of the pregnancy by providing information about gene expression in fetal, placental and maternal tissues. Koh et al. described using tissue-specific genes for direct measurement of tissue health and physiology, and that these measurements are concordant with the known physiology of pregnancy and fetal development at low time resolution (Koh et al. PNAS, Vol. 111, 20:7361-7366, (2014)). Analysis of tissue-specific transcripts in the instant samples enabled us to follow fetal and placental development with high resolution and sensitivity, and also to detect gene-specific response of the maternal immune system to pregnancy. The data from the present study establishes a "clock" for normal human development and enables a direct molecular approach to establish time to delivery and gestational age using nine placental genes. We demonstrate that cfRNA samples from both the second and third trimesters of pregnancy can predict expected delivery date with comparable accuracy to ultrasound, creating the basis for a portable, inexpensive dating method.

We recruited 31 pregnant Danish women from the Danish National Biobank, each of whom agreed to give blood on a weekly basis, resulting in 521 total plasma samples to analyze (FIG. 1A). All women delivered normally at term, defined as a gestational age at delivery of or greater than 37 weeks, and their medical records showed no unusual health changes during pregnancy (TABLE 8). Each sample was analyzed by highly multiplexed real time PCR using a panel of genes that were chosen to be specific to the placenta, fetal tissue, or the immune system.

**TABLE 8**

| **Demographics** | **Denmark (n=31)** | **Pennsylvania (n=16)** | | **Alabama (n=26)** | |
|---|---|---|---|---|---|
| | | *Preterm (n=9)* | *At-term (n*=*7)* | *Preterm (n=8)* | *At-term (n=18)* |
| Age (years ± SD) | 29.9 ± 3.2 | | | 23.9 ± 2.8 | 25.8 ± 4.4 |
| Parity (% nulliparous) | 19 (61.3) | | | 0 (0) | 0(0) |
| BMI (kg/m². mean ± SD) | 22.1 ± 3.6 | | | 28.9 ± 10.5 | 28.6 ± 7.0 |
| Ethnicity (% Hispanic) | 0(0) | | | 0(0) | 0(0) |
| Caucasian (%) | 31 (100) | | | 0(0) | 1 (6) |
| African-American (%) | 0(0) | | | 8 (100) | 17 (94) |
| Gestational age at delivery (weeks, mean ± SD) | 40 ± 1.2 | 26.7 ± 2.3 | 39.4 ± 0.5 | 30.8 ± 2.5 | 38.7 ± 1.2 |
| Mode of delivery | | | | | |
| Spontaneous | 67.7 | | | 7 (88) | 16 (29) |
| Cesarean section | 12.9 | | | 1 (12) | 2(11) |
| Gender (% male) | 14 (45.2) | | | 5 (63) | 10 (56) |
| Birth weight (kg, mean ± SD) | 3.6 ± 0.6 | | | 1.7 ± 0.7 | 3.1 ± 0.4 |

### 11.3 Example 3 - Gene Expression Of Maternal, Placental And Fetal-Tissue Specific Genes In Maternal Plasma Samples From Normal Due Date Deliveries

Cell-free RNA was isolated from each of the Denmark cohort individuals blood samples as set forth in Example 1. RT-qPCR assays were performed on the isolated cfRNA essentially as set forth in Example 1. A primer pair for each of the genes set forth in FIG. 9 was added to aliquots of the cfRNA samples and Ct values were calculated using appropriate controls.

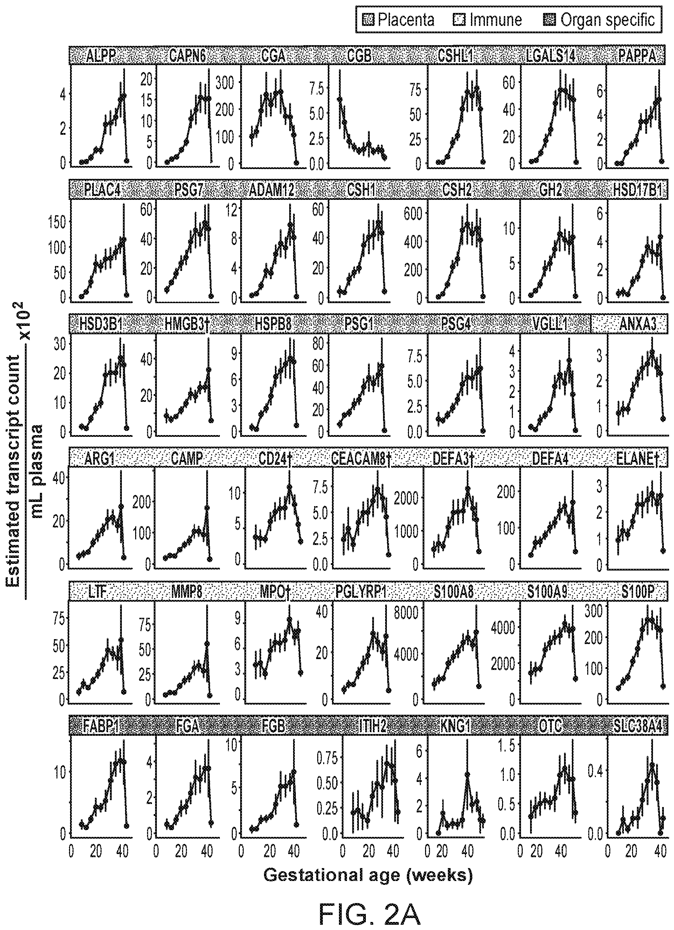

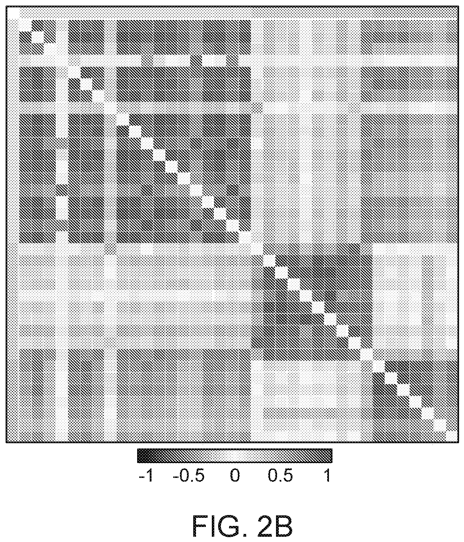

Gene-specific inter-patient monthly averages ± standard error of the mean (SEM) were plotted over the course of gestation (FIG. 2A). The average time course of gene expression highlighted interesting behavior that differed by gene function (FIGS. 2A and 4). Placental and fetal genes (blue and yellow) show a clear increase through the course of pregnancy with slightly different trajectories depending on the gene. Some of these genes plateau before delivery and one of them (CGB) decreases from a peak in the first trimester. Immune genes, which are dominated by the maternal immune system but may also include a fetal contribution, have a more complex interpretation but in general show changes in time with measurable baselines early in pregnancy and after delivery. We then calculated the correlation between gene values across all genes and all pregnancies (FIG. 2B) and discovered that genes within each set (i.e. placental, immune, fetal) were highly correlated with each other. Moreover, we found that placental and fetal genes also showed a moderate degree of cross correlation, suggesting that placental cfRNA may provide an accurate estimate of fetal development and gestational age throughout pregnancy.

### 11.4 Example 4 - Model for Prediction Of Time To Delivery & Comparison With Gold Standard (not claimed)

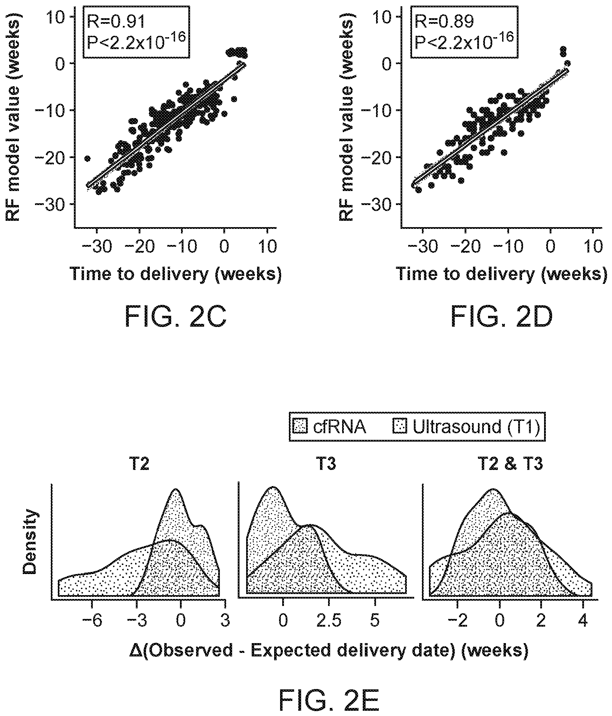

The results of the gene expression assays motivated us to apply a machine learning approach in order to build a model, which would predict gestational age or time to delivery from cfRNA measurements. We used a random forest model and were able to show that a subset of nine placental genes provided more predictive power than using the full panel of measured genes (FIG. 5). Using these 9 genes (CGA, CAPN6, CGB, ALPP, CSHL1, PLAC4, PSG7, PAPPA, and LGALS14) we accurately predicted the time from sample collection until delivery (Pearson correlation r=0.91, P < 2.2×10⁻¹⁶), which is an objective criterion independent of ultrasound-estimated gestational age (FIG. 2C). Our model's performance improved significantly over the course of gestation (root mean squared error (RMSE) = 6.0 (T1), 3.9 (T2), 3.3 (T3), 3.7 (PP) weeks). Remarkably, our model performed equally well (r=0.89, P < 2.2×10⁻¹⁶) on a withheld cohort of 10 women during the validation stage (RMSE = 5.4 (T1), 4.2 (T2), 3.8 (T3), 2.7 (PP) weeks) (FIG. 2D).

We also built a separate model to predict gestational age (as estimated by ultrasound) and using the same nine placental genes, the model performed comparably well both on training (r=0.91, P < 2.2×10⁻¹⁶) and validation data (r=0.90, P < 2.2×10⁻¹⁶) (FIGS. 6A and 6B).

The random forest model selects placental genes as most predictive of time from sample collection until delivery and gestational age. Although several of these genes show similar time trajectories, their detection rate early on pregnancy varies, suggesting that redundancy may improve accuracy at early time points, when both placental and fetal cfRNA are low and lead to drop-out effects. As cfRNA increases during gestation, the accuracy of the model improves. This is in contrast with the efficacy of ultrasound dating, which relies on a constant fetal growth rate, an assumption that deteriorates over time (Savitz et al. 2002; Papageorghiou et al. 2016).

Further investigating drivers of the model reveals markers with known roles during pregnancy. CGA and CGB, the two main model drivers together with CAPN6, behave differently from other genes in the model. CGA and CGB are the two subunits of HCG, known to play a major role in pregnancy initiation and progression and involved in trophoblast differentiation (Jaffe et al. 1969). The trend observed for these two genes is compatible with what is known from protein levels during pregnancy (Cocquebert et al. 2012). Free CGB and PAPPA are also used as biochemical markers for at risk of Down Syndrome in the first trimester (Wald and Hackshaw 1997), and other genes selected by the model are related to trophoblast development (e.g., LGALS14, PAPPA).

We then used our model to estimate expected delivery date from samples taken during the second, third, or both trimesters (FIG. 2E). We found that 32% (T2), 23% (T3), 45% (T2&T3), and 48% (T1 Ultrasound) of patients delivered within one week of their expected delivery dates (TABLE 9).

**TABLE 9**

| Method | Δ(Observed - Expected delivery date) (%) | | | | |
|---|---|---|---|---|---|
| | < -2 weeks | -1 to -2 weeks | ± 1 week | +1 to +2 weeks | > +2 weeks |
| cfRNA (T2) | 50 | 18 | 32 | 0 | 0 |
| cfRNA (T3) | 0 | 6 | 23 | 29 | 42 |
| cfRNA (T2 & T3) | 19 | 6 | 45 | 10 | 20 |
| Ultrasound (T1) | 0 | 26 | 48 | 23 | 3 |

Prior studies report that under normal circumstances it is possible to determine the week in which a woman may deliver with 57.8% accuracy using ultrasound and 48.1% using LMP (Savitz et al. 2002). Our results are not only comparable to ultrasound measurements at a fraction of the cost but also use a method that is more easily ported to resource challenged settings.

For gestational age prediction, we trained several distinct models on subpopulations of women (i.e., nulliparous or multiparous women, women carrying male or female fetuses) to determine the importance of the 9 genes that compose the transcriptomic signature identified. Training 4 distinct models for women carrying male or female fetuses and nulliparous or multiparous women revealed that 2 of the 9 genes identified in the main text were sufficient to predict time to delivery for women carrying male (CGA, CSHL1) (Root mean squared error (RMSE) of 5.43 and 4.80 in the second and third trimesters respectively) or female (CGA, CAPN6) fetuses (RMSE of 5.58 and 4.60 in the second and third trimesters respectively) and multiparous (CGA, CSHL1) women (RMSE of 5.22 and 4.56 in the second and third trimesters respectively). However, all 9 genes were necessary to predict time until delivery for nulliparous women (RMSE of 5.09 and 4.50 in the second and third trimesters respectively), highlighting the importance of the transcriptomic signature identified. The nine transcripts used to predict gestational age were weighted by the model in the following order of importance (from most to least): CGA, CAPN6, CGB, ALPP, CSHL1, PLAC4, PSG7, PAPPA, and LGALS14. See TABLE 10.

**TABLE 10**

| |
|---|
| 7.70 (T1 - multiparous), |
| 5.09 (T2 - nulliparous) vs 5.22 (T2 - multiparous), |
| 4.50 (T3 - nulliparous) vs 4.56 (T3 - multiparous), and |
| 3.13 (PP - nulliparous) vs 4.24 (PP - multiparous) weeks. |
| 5.58 (T2 - female) vs 5.43 (T2 - male), |
| 4.60 (T3 - female) vs 4.80 (T3 - male), and |
| 2.57 (PP - female) vs 2.83 (PP - male) weeks. |

In summary, we have discovered a molecular clock of fetal development which reflects the roadmap of developmental gene expression in the placenta and fetus, and enables prediction of time to delivery, gestational age, and expected delivery date with comparable accuracy to ultrasound. Our method has several advantages to ultrasound, namely cost and applicability later during pregnancy. At a fraction of the cost of ultrasound, cfRNA measurements can be easily ported to resource challenged settings. Even in countries that regularly use ultrasound, cfRNA presents an attractive, accurate alternative to ultrasound, especially during the second and third trimesters, when ultrasound predictions deteriorate to 15 (T2) or 27 (T3) day estimates of delivery (Altman and Chitty 1997). We expect that this clock will also be useful for discovering and monitoring fetuses having congenital defects that can be treated in utero, which represents a rapidly growing part of maternal-fetal medicine.

### 11.5 Example 5 - Identification Of Differentially Expressed Genes Between Normal And Preterm Deliveries

While the first generation "clock" model is able to predict gestational age and time of delivery for a normal pregnancy, we were also interested in testing its performance on preterm delivery. We therefore used two separately recruited cohorts from communities at high risk for premature delivery recruited at the University of Pennsylvania and the University of Alabama at Birmingham to test performance on preterm pregnancies (see, FIG. 1 and TABLE 1). We discovered that while the model validated performance on normal pregnancy (RMSE = 4.3 weeks), it generally failed to predict time until delivery in preterm samples (RMSE = 10.5 weeks) (FIG. 7). This suggests that the model's content is reflective of the normal developmental program and may not account for the various outlier physiological events which may lead to preterm birth. In other words, from a molecular perspective, the premature fetus does not appear to have reached full gestation and therefore preterm birth is likely not caused by overmaturation signals from the fetus or placenta, which give the illusion of reaching full-term. This conclusion is supported by the observation that pharmacological agents designed to stop or slow down uterine contractions prevent a small number of preterm deliveries (Romero et al. 2014; Conde-Agudelo and Romero 2016).

To further investigate this question and develop a second generation "clock" model capable of predicting preterm delivery, we performed RNAseq, essentially as set forth in Example 1, on cfRNA obtained from plasma samples from term (n=7) and preterm (n=9) women collected from one of the preterm-enriched cohorts (Pennsylvania) (see, FIG. 1 and TABLE 1) for genes, which may discriminate preterm from normal delivery.

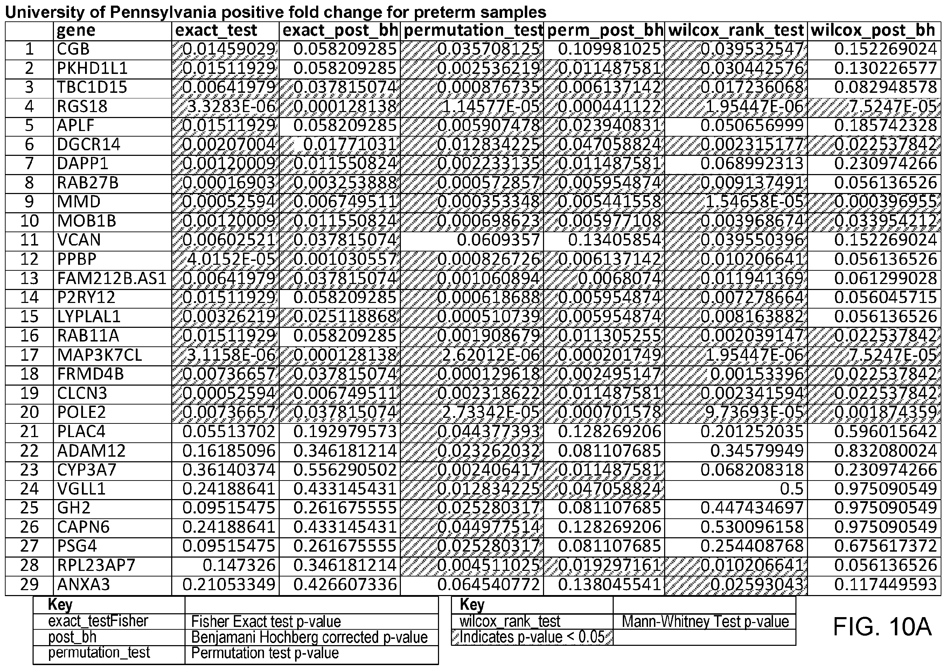

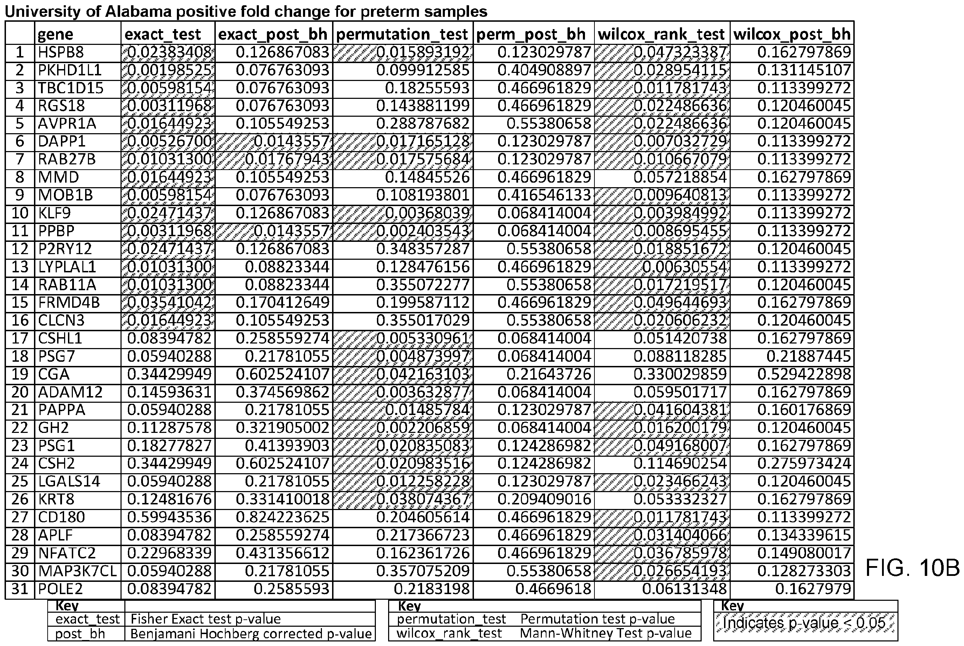

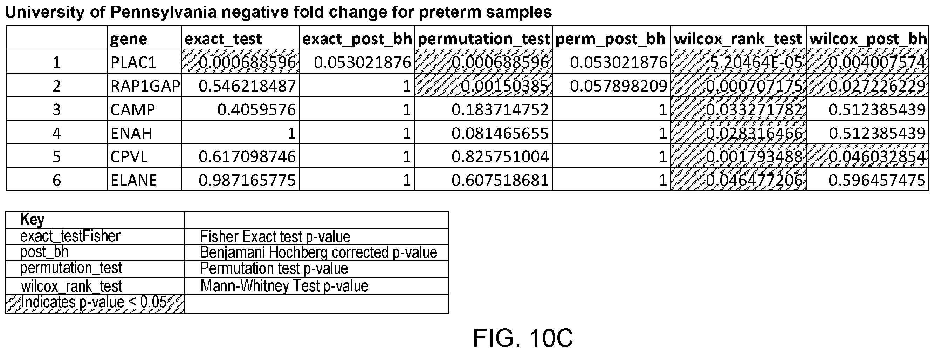

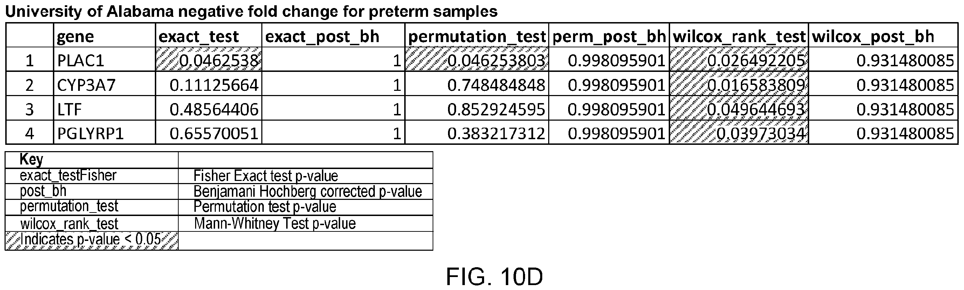

Analysis of this RNAseq data suggested that nearly 40 genes could separate term from preterm with statistical significance (p<0.001) (see, FIG. 3A and FIGS. 10A-10D). When recalculated to exclude one preeclamptic woman (see Examples) it was determined that 37 genes could separate term from preterm with statistical significance.

We then created a PCR panel with the highest scoring candidate preterm biomarkers and other immune and placental genes. We confirmed that the differential expression observed in RNAseq was also observed with this qPCR panel (FIG. 8).

### 11.6 Example 6 - Model for Prediction Of Preterm Delivery

The top ten genes from this panel (CLCN3, DAPP1, POLE2, PPBP, LYPLAL1, MAP3K7CL, MOB1B, RAB27B, RGS18, TBC1D15) (FDR ≤ 5%, Hedge's g ≥ 0.8) (FIG. 3B), accurately classify 7 out of 9 preterm samples (78%) and misclassify only 1 of 26 at-term samples (4%) from both Pennsylvania and Denmark with a mean AUC of 0.87 (FIG. 3C).

When used in combination, these ten genes also showed successful validation in an independent preterm-enriched cohort from Alabama, accurately classifying 4 out of 6 preterm samples (66%) and misclassifying 3 out of 18 at-term samples (17%) (see, FIG. 1).

Moreover, this independent validation cohort shows that it is possible to discriminate preterm from term pregnancy up to 2 months in advance of labor with an AUC of 0.74 (FIG. 3C). Several of the genes in the response signature were individually significantly more highly expressed in women who delivered preterm (FDR ≤ 5%, Hedge's g ≥ 0.8), demonstrating the robustness of their effect (FIG. 3B). Our data suggests that the genes associated with spontaneous preterm birth are distinct from those found to be most predictive for gestational age and normal time to delivery.

In subsequent refinements we determined that one woman in the cohort experienced induced preterm birth due to preeclampsia rather than spontaneous preterm birth We removed the data points associated with her plasma sample. Rerunning the analysis with this sample removed yielded 7 transcripts (CLCN3, DAPP1, PPBP, MAP3K7CL, MOB1B, RAB27B, RGS18) as opposed to 10, that when used in combinations of 3 produced a true positive rate of greater than 75% and misclassified less than 5%.

As described in Example 7, below, we identified several subcombinations of the 7 transcripts that may be used to determine a woman's likelihood or risk of preterm delivery. Thus, in some approaches one or more of the following panels is used to assess the likelihood of full-term, or preterm, delivery: (1) RGS18; DAPP1; PPBP; (2) RGS18; RAB27B; PPBP; (3) RGS18; MOB1B; PPBP; (4) RGS18; PPBP; MAP3K7CL; (5) RGS18; PPBP; CLCN3; (6) DAPP1; RAB27B; PPBP; (7) DAPP1; MOB1B; PPBP; (8) DAPP1; PPBP; CLCN3; (9) RAB27B; MOB1B; PPBP; (10) RAB27B; PPBP; MAP3K7CL; (11) RAB27B; PPBP; CLCN3; (12) MOB1B; PPBP; MAP3K7CL; and (13) MOB1B; PPBP; CLCN3.

We found that PPBP, DAPP1, and RAB27B were all individually elevated in women who delivered preterm in both the Pennsylvania and Alabama cohorts (FDR ≤ 5%, Hedge's g ≥ 0.8), demonstrating the robustness of their effect. The ranking the weight order (from highest to lowest) is RAB27B > PPBP > DAPP1 > RGS18 > (MOB1B, MAP3K7CL, and CLCN3).

In summary, we have discovered and validated a set of biomarkers which enables prediction of time to delivery for patients at risk of preterm delivery. Furthermore, our preterm delivery model suggests that the physiology of preterm delivery is distinct from normal development, forming the basis for the first screening or diagnostic test for risk of prematurity.

### 11.7 Example 7 - Gene combinations meeting the criterion of 75% true positive rate and less than 5% false positive rate

Seven transcripts of interest RAB27B, PPBP, DAPP1, RGS18, MOB1B, MAP3K7CL, CLCN37 can be grouped in 35 unique combinations of genes. We filtered those combinations using the criterion of 75% true positive rate and less than 5% false positive rate. This yielded 13 combinations shown in TABLE 11. We generated an ROC curve to determine the which combinations predict risk of delivering preterm.

**TABLE 11**

| Combination | Gene 1 | Gene 2 | Gene 3 |
|---|---|---|---|
| 1 | RGS18 | DAPP1 | PPBP |
| 2 | RGS18 | RAB27B | PPBP |
| 3 | RGS18 | MOB1B | PPBP |
| 4 | RGS18 | PPBP | MAP3K7CL |
| 5 | RGS18 | PPBP | CLCN3 |
| 6 | DAPP1 | RAB27B | PPBP |
| 7 | DAPP1 | MOB1B | PPBP |
| 8 | DAPP1 | PPBP | CLCN3 |
| 9 | RAB27B | MOB1B | PPBP |
| 10 | RAB27B | PPBP | MAP3K7CL |
| 11 | RAB27B | PPBP | CLCN3 |
| 12 | MOB1B | PPBP | MAP3K7CL |
| 13 | MOB1B | PPBP | CLCN3 |

Each of these 13 combinations of 3 genes may be used as a panel for assessing risk of preterm delivery. Thus, in some embodiments a panel comprising one or more of the following combination of genes is used to determine of the following panels Thus, in some approaches a panel comprising one or more of the following combinations of genes is used to assess the likelihood of full-term, or preterm, delivery: (1) RGS18; RAB27B; PPBP; (2) DAPP1; RAB27B; PPBP; (3) RAB27B; MOB1B; PPBP; (4) RAB27B; PPBP; MAP3K7CL; (5) RAB27B; PPBP; CLCN3.

### 11.8 Example 8 - Body mass index (BMI) does not affect cell-free RNA (cfRNA) levels

We have tested for the effect of BMI on circulating cfRNA levels using estimated transcript counts of GAPDH per milliliter of plasma and found no significant difference between underweight (BMI < 18.5), normal weight (18.5 ≤ BMI < 25), overweight (25 ≤ BMI < 30), and obese (BMI ≥ 30) individuals both before and after Bonferroni correction using a Wilcoxon rank sum test.

P-values for distinct tests of GAPDH levels before and after Bonferroni correction, respectively, were as follows: (1) underweight versus normal weight (P = 0.58,1), underweight versus overweight (P = 0.12, 0.80), underweight versus obese (P = 0.26, 1), normal weight versus overweight (P = 0.06, 0.35), normal weight versus obese (P = 0.16, 0.95), and overweight versus obese (P = 0.72, 1). Similar results were obtained for placental-specific cfRNAs such as CAPN6, CGA, and CGB.

All comparisons were done within cohorts so that differences in BMI distribution between cohorts were not confounding.

### 12. Selected References

Altman, D. G., & Chitty, L. S. (1997). New charts for ultrasound dating of pregnancy. Ultrasound in Obstetrics & Gynecology, 10(3), 174-191. doi:10.1046/j.1469-0705.1997. 10030174.x
Barr, W. B., & Pecci, C. C. (2004). Last menstrual period versus ultrasound for pregnancy dating. International Journal of Gynaecology and Obstetrics, 87(1), 38-39. doi:10.1016 /j.ijgo.2004.06.008
Bennett, K. A., Crane, J. M. G., O'shea, P., Lacelle, J., Hutchens, D., & Copel, J. A. (2004). First trimester ultrasound screening is effective in reducing postterm labor induction rates: a randomized controlled trial. American Journal of Obstetrics and Gynecology, 190(4), 1077-1081. doi:10.1016/j.ajog.2003.09.065
Blencowe, H., Cousens, S., Chou, D., Oestergaard, M., Say, L., Moller, A.-B., ... Born Too Soon Preterm Birth Action Group. (2013). Born too soon: the global epidemiology of 15 million preterm births. Reproductive Health, 10 Suppl 1, S2. doi:10.1186/1742-4755-10-S1-S2
Cocquebert, M., Berndt, S., Segond, N., Guibourdenche, J., Murthi, P., Aldaz-Carroll, L., ... Fournier, T. (2012). Comparative expression of hCG β-genes in human trophoblast from early and late first-trimester placentas. American Journal of Physiology. Endocrinology and Metabolism, 303(8), E950-8. doi:10.1152/ajpendo.00087.2012
Conde-Agudelo, A., & Romero, R. (2016). Vaginal progesterone to prevent preterm birth in pregnant women with a sonographic short cervix: clinical and public health implications. American Journal of Obstetrics and Gynecology, 214(2), 235-242. doi:10.1016/j.ajog.2015.09.102
Dugoff, L., Hobbins, J. C., Malone, F. D., Vidaver, J., Sullivan, L., Canick, J. A., ... FASTER Trial Research Consortium. (2005). Quad screen as a predictor of adverse pregnancy outcome. Obstetrics and Gynecology, 106(2), 260-267. doi:10.1097/01.AOG.0000172419.37410.eb
Hanson, A. E. (1987). The Eight Months' Child and the Etiquette of Birth: Obsit Omen! Bulletin of the History of Medicine.
Hanson, A. E. (1995). Paidopoiia: Metaphors for conception, abortion, and gestation in the Hippocratic Corpus. Clio Medica (Amsterdam, Netherlands).
Institute of Medicine (US) Committee on Understanding Premature Birth and Assuring Healthy Outcomes. (2007). Preterm Birth: Causes, Consequences, and Prevention. (R. E. Behrman & A. S. Butler, Eds.). Washington (DC): National Academies Press (US).
Jaffe, R. B., Lee, P. A., & Midgley, A. R. (1969). Serum gonadotropins before, at the inception of, and following human pregnancy. The Journal of Clinical Endocrinology and Metabolism, 29(9), 1281-1283. doi:10.1210/jcem-29-9-1281
Koh, W., Pan, W., Gawad, C., Fan, H. C., Kerchner, G. A., Wyss-Coray, T., ... Quake, S. R. (2014). Noninvasive in vivo monitoring of tissue-specific global gene expression in humans. Proceedings of the National Academy of Sciences of the United States of America, 111(20), 7361-7366. doi:10.1073/pnas.1405528111
Liu, L., Johnson, H. L., Cousens, S., Perin, J., Scott, S., Lawn, J. E., ... Child Health Epidemiology Reference Group of WHO and UNICEF. (2012). Global, regional, and national causes of child mortality: an updated systematic analysis for 2010 with time trends since 2000. The Lancet, 379(9832), 2151-2161. doi:10.1016/50140-6736(12)60560-1
Lund, S. P., Nettleton, D., McCarthy, D. J., & Smyth, G. K. (2012). Detecting differential expression in RNA-sequence data using quasi-likelihood with shrunken dispersion estimates. Statistical Applications in Genetics and Molecular Biology, 11(5). doi:10.1515/1544-6115.1826
McCarthy, D. J., Chen, Y., & Smyth, G. K. (2012). Differential expression analysis of multifactor RNA-Seq experiments with respect to biological variation. Nucleic Acids Research, 40(10), 4288-4297. doi:10.1093/nar/gks042
Muglia, L. J., & Katz, M. (2010). The enigma of spontaneous preterm birth. The New England Journal of Medicine, 362(6), 529-535. doi:10.1056/NEJMra0904308
Murray, C. J. L., Vos, T., Lozano, R., Naghavi, M., Flaxman, A. D., Michaud, C., ... et al. (2012). Disability-adjusted life years (DALYs) for 291 diseases and injuries in 21 regions, 1990-2010: a systematic analysis for the Global Burden of Disease Study 2010. The Lancet, 380(9859), 2197-2223. doi:10.1016/50140-6736(12)61689-4
Papageorghiou, A. T., Kemp, B., Stones, W., Ohuma, E. O., Kennedy, S. H., Purwar, M., ... International Fetal and Newborn Growth Consortium for the 21st Century (INTERGROWTH-21st). (2016). Ultrasound-based gestational-age estimation in late pregnancy. Ultrasound in Obstetrics & Gynecology, 48(6), 719-726. doi:10.1002/uog.15894
Parker, H. (1999). Greek Embryological Calendars and a Fragment from the Lost Work of Damastes, on the Care of Pregnant Women and of Infants. The Classical Quarterly.
Robinson, M. D., McCarthy, D. J., & Smyth, G. K. (2010). edgeR: a Bioconductor package for differential expression analysis of digital gene expression data. Bioinformatics, 26(1), 139-140. doi:10.1093/bioinformatics/btp616
   Robinson, M. D., & Smyth, G. K. (2008). Small-sample estimation of negative binomial dispersion, with applications to SAGE data. Biostatistics, 9(2), 321-332. doi:10.1093/biostatistics/kxm030
Romero, R., Dey, S. K., & Fisher, S. J. (2014). Preterm labor: one syndrome, many causes. Science, 345(6198), 760-765. doi:10.1126/science.1251816
Rose, N. C., & Mennuti, M. T. (1993). Maternal serum screening for neural tube defects and fetal chromosome abnormalities. The Western Journal of Medicine, 159(3), 312-317.
Savitz, D. A., Terry, J. W., Dole, N., Thorp, J. M., Siega-Riz, A. M., & Herring, A. H. (2002). Comparison of pregnancy dating by last menstrual period, ultrasound scanning, and their combination. American Journal of Obstetrics and Gynecology, 187(6), 1660-1666. doi:10.1067/mob.2002.127601
Sweeney, T. E., Haynes, W. A., Vallania, F., Ioannidis, J. P., & Khatri, P. (2017). Methods to increase reproducibility in differential gene expression via meta-analysis. Nucleic Acids Research, 45(1), e1. doi:10.1093/nar/gkw797
Wald, N. J., & Hackshaw, A. K. (1997). Combining ultrasound and biochemistry in first-trimester screening for Down's syndrome. Prenatal Diagnosis, 17(9), 821-829. doi:10.1002/(SICI)1097-0223(199709)17:9<821::AID-PD154>3.0.CO;2-5
Ward, K., Argyle, V., Meade, M., & Nelson, L. (2005). The heritability of preterm delivery. Obstetrics and Gynecology, 106(6), 1235-1239. doi:10.1097/01.AOG.0000189091.35982.85
Whitworth, M., Bricker, L., & Mullan, C. (2015). Ultrasound for fetal assessment in early pregnancy. Cochrane Database of Systematic Reviews, (7), CD007058. doi:10.1002/ 14651858.CD007058.pub3
Yefet, E., Kuzmin, O., Schwartz, N., Basson, F., & Nachum, Z. (2017). Predictive Value of Second-Trimester Biomarkers and Maternal Features for Adverse Pregnancy Outcomes. Fetal Diagnosis and Therapy. doi:10.1159/000458409
York, T. P., Strauss, J. F., Neale, M. C., & Eaves, L. J. (2009). Estimating fetal and maternal genetic contributions to premature birth from multiparous pregnancy histories of twins using MCMC and maximum-likelihood approaches. Twin Research and Human Genetics, 12(4), 333-342. doi:10.1375/twin.12.4.333
Zhang, G., et al. (2017). Genetic Associations with Gestational Duration and Spontaneous Preterm Birth. The New England Journal of Medicine, 377(12), 1156-1167. doi:10.1056/ NEJMoa1612665
Rose and Mennuti (Fetal Medicine, West J Med., 1993; 159:312-317)
Sweeney et al. (J. Pediatric Infect. Dis. Soc., 2017, doi: 10.1093/jpids/pix021.)

### 13. TABLES 1-5

**TABLE 1: PREDICTING TIME TO DELIVERY**

| Gene | RefSeq | Gene ID | Tissue Specificity | Tissue | Function |
|---|---|---|---|---|---|
| CGA | NM_001252383.1 | 1081 | Yes | Placenta | Subunit of HCG |
| CAPN6 | NM_014289.3 | 827 | Yes | Placenta | Calcium-dependent cysteine protease |
| CGB | NM_000737.3 | 1082 | Yes | Placenta | Subunit of HCG |
| LGALS14 | NM_020129.2 | 56891 | Yes | Placenta | Carbohydrate recognition |
| PSG7 | NM_002783.2 | 5676 | Yes | Placenta | Immunoglobin-like proteins, known to be released into maternal circulation |
| ALPP | NM_001632.3 | 250 | Yes | Placenta | Alkaline phosphatase |
| CSHL1 | NM_001318.2 | 1444 | Yes | Placenta | Growth control, located at growth hormone locus, expressed in placental villi |
| PAPPA | NM_002581.3 | 5069 | Yes | Placenta | Metalloproteinase which cleaves insulin growth factors that can then bind IGF receptors |
| PLAC4 | NM_182832.2 | 191585 | Yes | Placenta | Expressed in placental syncytiotrophoblasts, associated with preeclampsia and trisomy 21 |
| ACTB | NM_001101.3 | 60 | No | | |
| HSD3B1 | NM_000862.2 | 3283 | Yes | Placenta | |
| S100A8 | NM_002964.4 | 6279 | Yes | Immune | Immune indicates bone marrow specificity |
| HAL | NM_002108.2 | 15109 | No | | |
| HSPB8 | NM_014365.2 | 26353 | No | | |
| VGLL1 | NM_016267.3 | 51442 | Yes | Placenta | |
| S100A9 | NM_002965.3 | 6280 | Yes | Immune | Immune indicates bone marrow specificity |
| ITIH2 | NM_002216.2 | 3698 | Yes | Liver | |
| ANXA3 | NM_005139.2 | 306 | Yes | Immune | |
| S100P | NM_005980.2 | 6286 | No | | |
| KNG1 | NM_000893.3 | 3827 | Yes | Liver | |
| CYP3A7 | NM_000765.3 | 1551 | Yes | Liver | |
| CSH1 | NM_001317.5 | 1442 | Yes | Placenta | |
| CAMP | NM_004345.4 | 820 | Yes | Immune | Immune indicates bone marrow specificity |
| OTC | NM_000531.5 | 5009 | Yes | Liver | |
| DCX | NM_000555.3 | 1641 | Yes | Brain | |
| FSTL3 | NM_005860.2 | 10272 | Yes | Placenta | |
| CSH2 | NM_022644.3 | 1443 | Yes | Placenta | |
| PLAC1 | NM_021796.3 | 10761 | Yes | Placenta | |
| DEFA4 | NM_001925.1 | 1669 | Yes | Immune | Immune indicates bone marrow specificity |
| FABP1 | NM_001443.1 | 2168 | Yes | Liver | |
| SERPINA7 | NM_000354.5 | 6906 | Yes | Liver | |
| FRZB | NM_001463.3 | 2487 | No | | |
| SLC2A2 | NM_000340.1 | 6514 | Yes | Liver | |
| LTF | NM_001199149.1 | 4057 | Yes | Immune | Immune indicates bone marrow specificity |
| FGA | NM_000508.3 | 2243 | Yes | Liver | |
| SLC4A1 | NM_000342.3 | 6521 | Yes | Immune | Immune indicates bone marrow specificity |
| GNAZ | NM_002073.2 | 2781 | No | | |
| ADAM 12 | NM_003474.4 | 8038 | Yes | Placenta | |
| GH2 | NM_022557.3 | 2689 | Yes | Placenta | |
| PSG 1 | NM_006905.2 | 5669 | Yes | Placenta | |
| MMP8 | NM_002424.2 | 4317 | Yes | Immune | Immune indicates bone marrow specificity |
| FGB | NM_005141.4 | 2244 | Yes | Liver | |
| ARG1 | NM_001244438.1 | 383 | Yes | Liver | |
| MEF2C | NM_001131005.2 | 4208 | No | | |
| HSD17B1 | NM_000413.2 | 3292 | Yes | Placenta | |
| PSG4 | NM_002780.4 | 5672 | Yes | Placenta | |
| PGLYRP1 | NM_005091.2 | 8993 | Yes | Immune | Immune indicates bone marrow specificity |
| SLC38A4 | NM_018018.4 | 55089 | Yes | Liver | |
| EPB42 | NM_000119.2 | 2038 | Yes | Immune | Immune indicates bone marrow specificity |
| PTGER3 | NM_198717.1 | 5733 | No | | |

**TABLE 2: PREDICTING PRETERM DELIVERY**

| **Gene** | **RefSeq** | **Gene ID** | **Tissue Specifici ty** | **Tissue** | **"Druggable?"** | **Function** |
|---|---|---|---|---|---|---|
| TBC1D15 | NM_00114621 4 | 64786 | No | | Yes - involved in signalling | Encodes Ras-like protein. Regulator of intracellular traffic |
| RGS18 | NM_130782 | 64407 | No | | Yes - involved in signalling | Regulator of G-protein signaling |
| DAPP1 | NM_00130615 1 | 27071 | No | | Yes - involved in signalling | B-cell receptor signaling pathway |
| RAB27B | NM_004163 | 5874 | No | | Yes - involved in signalling | Prenylated, membrane bound proteins involved in vesicular fusion and trafficking |
| MOB1B | NM_00124476 6 | 92597 | No | | Yes - involved in cell cycle | Kinase essential for spindle pole body duplicaiton and mitotic checkpoint regulation |
| PPBP | NM_002704 | 5473 | Yes | Immune | Unclear | Platelet dereived growth factor |
| LYPLAL1 | NM_138794 | 127018 | No | | Unclear | Unknown, links to childhood obesity and hypertension |
| MAP3K7CL | NM_00128661 7 | 56911 | No | | Unclear | Unknown |
| CLCN3 | NM_173872 | 1182 | No | | Probably not given its ubiquitous nature across cell types | Voltage-gated chloride channel present in all cell types |
| POLE2 | NM_002692 | 5427 | No | | Yes- involved in cell cycle | Involved in DNA repair and replication |
| CGB | NM_000737.3 | 1082 | Yes | Placenta | | |
| PKHD1L1 | NM_177531 | 93035 | Yes | Thyroid | | |
| APLF | NM_173545 | 200558 | No | | | |
| DGCR14 | NR_134304 | 8220 | Yes | Testis | | |
| MMD | NM_012329 | 23531 | Yes | Fat | | |
| VCAN | NM_004385 | 1462 | No | | | |
| P2RY12 | NM_022788 | 64805 | Yes | Brain | | |
| RAB11A | NM_004663 | 8766 | No | | | |
| FRMD4B | NM_015123 | 23150 | No | | | |
| PLAC4 | NM_182832.2 | 191585 | Yes | Placenta | | |
| ADAM 12 | NM_003474.4 | 8038 | Yes | Placenta | | |
| CYP3A7 | NM_000765.3 | 1551 | Yes | Liver | | |
| VGLL1 | NM_016267.3 | 51442 | Yes | Placenta | | |
| GH2 | NM_022557.3 | 2689 | Yes | Placenta | | |
| CAPN6 | NM_014289.3 | 827 | Yes | Placenta | | |
| PSG4 | NM_002780.4 | 5672 | Yes | Placenta | | |
| RPL23AP7 | NR_024528 | 118433 | No | | | |
| ANXA3 | NM_005139.2 | 306 | Yes | Immune | | |
| HSPB8 | NM_014365.2 | 26353 | No | | | |
| PKHD1L1 | NM_177531 | 93035 | Yes | Thyroid | | |
| AVPR1A | NM_000706 | 552 | No | | | |
| KLF9 | NM_001206 | 687 | No | | | |
| CSHL1 | NM_001318.2 | 1444 | Yes | Placenta | | |
| PSG7 | NM_002783.2 | 5676 | Yes | Placenta | | |
| CGA | NM_00125238 3.1 | 1081 | Yes | Placenta | | |
| PAPPA | NM_002581.3 | 5069 | Yes | Placenta | | |
| PSG1 | NM_006905.2 | 5669 | Yes | Placenta | | |
| CSH2 | NM_022644.3 | 1443 | Yes | Placenta | | |
| LGALS14 | NM_020129.2 | 56891 | Yes | Placenta | | |
| KRT8 | NR_045962 | 3856 | No | | | |
| CD180 | NM_005582 | 4064 | No | | | |
| NFATC2 | NM_012340 | 4773 | No | | | |
| PLAC1 | NM_021796.3 | 10761 | Yes | Placenta | | |
| RAP1GAP | NM_00114565 7 | 5909 | No | | | |
| CAMP | NM_004345.4 | 820 | Yes | Immune | | |
| ENAH | NM_00100849 3 | 55740 | No | | | |
| CPVL | NM_019029 | 54504 | No | | | |
| ELANE | NM_001972 | 1991 | Yes | Immune | | |
| LTF | NM_00119914 9.1 | 4057 | Yes | Immune | | |
| PGLYRP1 | NM_005091.2 | 8993 | Yes | Immune | | |
| FAM212B-AS1 | NR_038951 | 1005063 43 | No | | | |
| Immune | indicates | bone | marrow | specificit y | | |

**TABLE 3: Exemplary primer pairs.**

| **Gene** | **SEQ ID NO:** | **Forward Primer** | **Reverse Primer** | **SEQ ID NO:** |
|---|---|---|---|---|
| ACTB | 20 | CCAACCGCGAGAAGATGAC | TAGCACAGCCTGGATAGCAA | 21 |
| ADAM 12 | 22 | TGAGAAAGGAGGCTGCATCA | CTGCTGCAACTGCTGAACA | 23 |
| AFP | 24 | GCCTCTTCCAGAAACTAGGAGAA | GGGGCTTTCTTTGTGTAAGCAA | 25 |
| ALPP | 26 | GACAGCTGCCAGGATCCTAA | GTCTGGCACATGTTTGTCTACA | 27 |
| ANXA1 | 28 | AAGTGCGCCACAAGCAAA | TGCCTTATGGCGAGTTCCA | 29 |
| ANXA3 | 30 | CAGCGGCAGCTGATTGTTAA | CAGAGAGATCACCCTTCAAGTCA | 31 |
| APLF | 32 | ACCCAGATGACTCCCACAAA | CAAGGATTGGCTGCTGCTTA | 33 |
| APOA4 | 34 | AAGGCCGTGGTCCTGAC | TCAGCTGGCTGAAGTAGTCC | 35 |
| ARG1 | 36 | GCAAGGTGGCAGAAGTCAA | ATGGCCAGAGATGCTTCCA | 37 |
| AVPR1A | 38 | GCGCCTTTCTTCATCATCCA | GATGGTGATGGTAGGGTTTTCC | 39 |
| BPI | 40 | TCCTGGAACTGAAGCACTCA | GCAGCACAAGAATGGGTACA | 41 |
| CALCB | 42 | CCCCTTCCTGGCTCTCAGTA | GGTCTGGGCTGCTCTCCA | 43 |
| CAMP | 44 | GGACAGTGACCCTCAACCA | CAGCAGGGCAAATCTCTTGTTA | 45 |
| CAPN6 | 46 | TGGAAAGGTGGTGTGGAAAC | GTCAG CTG GTG GTTG CTAA | 47 |
| CCL20 | 48 | TGATGTCAGTGCTGCTACTCC | CTGTGTATCCAAGACAGCAGTCA | 49 |
| CD160 | 50 | CTCAGTTCAGGCTTCCTACA | TCTTTTGGCACAAGGCTTAC | 51 |
| CD180 | 52 | CACAATAGAACCTTCAGCAGAC | GAAAAGTGTCTTCATGTATCCAGTTA | 53 |
| CD2 | 54 | ATTCCAGCTTCAACCCCTCA | ATGACTAGGTGCCTGGGAAC | 55 |
| CD24 | 56 | CCAACTAATGCCACCACCAA | CGAAGAGACTGGCTGTTGAC | 57 |
| CD5 | 58 | CCCCTTGCCTACAAGAAGCTA | TCCCGTTGGGCCAATCC | 59 |
| CDK5R1 | 60 | AGCAAGAACGCCAAGGACAA | CGGCCACGATTCTCTTCCAA | 61 |
| CEACAM6 | 62 | AGATTGCATGTCCCCTGGAA | GGGTGGGTTCCAGAAGGTTA | 63 |
| CEACAM8 | 64 | TATGCCTGCCACACCACTAA | GCCAGGAGAACTTCCTTGTACTA | 65 |
| CGA | 66 | TCAACCGCCCTGAACACA | ACACCGACAATGTGACCAGAA | 67 |
| CGB | 68 | AG CCTTCCAAG CCCATCC | TGCGGATTGAGAAGCCTTTA | 69 |
| CLCN3 | 70 | CGTGGTCAGGATGGCTAGTA | CCAATCGGCAGCAATGTCTA | 71 |
| CNOT7 | 72 | GTCCTCTGTGAAGGGGTCAAA | TCTTCAGGCAAGTTAGAGTTGGTTA | 73 |
| COL17A1 | 74 | TGACAACCCAGAGCTCATCC | GGACGCCATGTTGTTTGGAA | 75 |
| COL21A1 | 76 | CGTCCAGGTGTCAGAGGATTA | ACCTTGTTCTCCAGGATACCC | 77 |
| CPVL | 78 | TGAAGTGGCTGGTTACATCC | AGAGGCTGGTCATAGGGTAA | 79 |
| CRP | 80 | GTCTTGACCAGCCTCTCTCA | ACGGTGCTTTGAGGGATACA | 81 |
| CSH1 | 82 | ACAAGAGACCGGCTCTAGGA | TTGCCACTAGGTGAGCTGTC | 83 |
| CSH2 | 84 | CGTTCCGTTATCCAGGCTTTT | ACTCCTGGTAGGTGTCAATGG | 85 |
| CSHL1 | 86 | TTAGAGCTGCTCCACATCTCC | ACCAGGTTGTTGGTGAAGGTA | 87 |
| CUX2 | 88 | TCCATCACCAAGAGGGTGAA | CAGGATGCTTTCCCCAAACA | 89 |
| CYP3A7 | 90 | ACGTGCATTGTGCTCTCTCA | CAGCACTGATTTGGTCATCTCC | 91 |
| DAPP1 | 92 | TGGGCACCAAAGAAGGTTA | TTCCTGTGCAGAGTAAACCA | 93 |
| DCX | 94 | ATCTCTACG CCCACCAGTCC | AGCGAGTCCGAGTCATCCAA | 95 |
| DEFA3 | 96 | GACGAAAGCTTGGCTCCAAA | GTTCCATAGCGACGTTCTCC | 97 |
| DEFA4 | 98 | TGGGATAAAAGCTCTGCTCTTCA | TGTTCGCCGGCAGAATACTA | 99 |
| DGCR14 | 100 | ACAAGGCCAAGAATTCCCTCA | TGCCGGGGCTTCTTAAACA | 101 |
| DLX2 | 102 | TTCGTCCCCAGCCAACAA | TGGCTTCCCGTTCACTATCC | 103 |
| EGFR | 104 | GCAGTGACTTTCTCAGCAACA | TTGGGACAGCTTGGATCACA | 105 |
| ELANE | 106 | CTCTGCCGTCGCAGCAA | TGGATTAGCCCGTTGCAGAC | 107 |
| ENAH | 108 | GCCGGAGCAAAACTTAGGAAA | AGGCGGAGTTCACACCAATA | 109 |
| EPB42 | 110 | GCCAAGCTCTGGAGGAAGAA | GAGAAGAACAGGCCGATGGTTA | 111 |
| EPOR | 112 | ATCCTGGTGCTGCTGAC | GGCCAGATCTTCTGCTTCA | 113 |
| EPX | 114 | AGTTCAGAAGAGCCCGAGAC | GCGCTGTCTTTTGGTGAAAAC | 115 |
| EVX1 | 116 | TACCGGGAGAACTACGTATCCA | ATGCGCCGGTTCTGGAA | 117 |
| FABP1 | 118 | AGGAATGTGAGCTGGAGACA | TTGTCACCTTCCAACTGAACC | 119 |
| FABP7 | 120 | GCTACCTGGAAGCTGACCAA | CCACCTGCCTAGTGGCAAA | 121 |
| FAM212B- | 122 | GGAAAGGGGTGGATGTGTCA | CACCCAGGATGTCCTTGTTCTA | 123 |
| FGA | 124 | ATGTTAGAGCTCAGTTGGTTGATA | TACTGCATGACCCTCGACAA | 125 |
| FGB | 126 | ATATTGTCGCACCCCATGCA | ACCTCCTTTCCTGATAATTTCCTCAC | 127 |
| FOXG1 | 128 | GCCAGCAGCACTTTGAGTTA | TGAGTCAACACGGAGCTGTA | 129 |
| FRMD4B | 130 | GAAACCCAGCCAGAAAGCAA | AGGTGGTGGTGTCAGACAAA | 131 |
| FRZB | 132 | CCTCTGCCCTCCACTTAATGTTA | CAGCTATAGAGCCTTCCACCAA | 133 |
| FSTL3 | 134 | CCGGACCTGAGCGTCATGTA | GCACACCACGTGCTCACA | 135 |
| GAPDH | 136 | GAACGGGAAGCTTGTCATCAA | ATCGCCCCACTTGATTTTGG | 137 |
| GCA | 138 | TCAGTTTGGAAACCTGCAGAA | GCTGCCCATAGCTCTTTGAA | 139 |
| GH2 | 140 | CCCGTCGCCTGTACCA | TGTTGGAATAGACTCTGAGAAGCA | 141 |
| GNAZ | 142 | CGGCTACGACCTGAAACTCTA | TGAGTGAGGTGTTGATGAACCA | 143 |
| GPR116 | 144 | CCAGAGGCAGTGCAAACATAA | AGAAATTGGGTCCGGGGTTA | 145 |
| GRHL2 | 146 | ACTCCGGACAGCACATACA | CCAACTGAAG CACTCCGAAA | 147 |
| GSN | 148 | AAGACCTGGCAACGGATGAC | TTGAGAATCCTTTCCAACCCAGAC | 149 |
| GYPB | 150 | ACAACTTGTCCATCGTTTCAC | ACCAGCCATCACACACAA | 151 |
| HAL | 152 | AGAACTGAACAGCGCAACA | GCTGGGTATTCACCATGGAA | 153 |
| HBG2 | 154 | GGTGACCGTTTTGGCAATCC | CACTGGCCACTCCAGTCAC | 155 |
| HIST1H2BM | 156 | GCCTGGCGCATTACAACAA | CAATTCCCCGGGTAGCAGTA | 157 |
| HMGB3 | 158 | CGGCAAAGCTGAAGGAGAAGTA | CAGGACCCTTTGCACCATCA | 159 |
| HMGN2 | 160 | ACACAGTGCTAGGTGCAGTTA | TCCATACTCCCAGCCTTTCAC | 161 |
| HS6ST1 | 162 | AAGTTCATCCGGCCCTTCA | GGTGTCTTCATCCACCTCCA | 163 |
| HSD17B1 | 164 | TGGACGTAAGGGACTCAAAATCC | CCCAGGCCTGCGTTACA | 165 |
| HSD3B1 | 166 | TGTGCCTTACGACCCATGTA | GTTGTTCAGGGCCTCGTTTA | 167 |
| HSPB8 | 168 | GCAAGAAGGTGGCATTGTTTCTA | TCTGGGGAAAGTGAGGCAAA | 169 |
| ITIH2 | 170 | AGAGAAGAGAAGGCTGGTGAAC | TCCAGGTTGTCAGGAGCAAA | 171 |
| KLF9 | 172 | TCCCATCTCAAAGCCCATTACA | CTCGTCTGAGCGGGAGAA | 173 |
| KNG1 | 174 | CTGGCAGGACTGTGAGTACAA | ATTTCGTACTGCTCCTCTTCCC | 175 |
| KRT8 | 176 | TGACCGACGAGATCAACTTCC | TGTGCCTTGACCTCAGCAA | 177 |
| KRT81 | 178 | TGAAGGCATTGGGGCTGTG | AGCCTGACACGCAGAGGT | 179 |
| LGALS14 | 180 | TGTG CATCTATGTG CGTCAC | GGAATCGATGGGCAAAGTTGTA | 181 |
| LHX2 | 182 | CAAAAGACGGGCCTCACCAA | CGTAAGAGGTTGCGCCTGAA | 183 |
| LIPC | 184 | CATCGGTGGAACGCACAA | GGGCACTTCCCTCAAACAAA | 185 |
| LRRN3 | 186 | GCCTTGGTTGGACTGGAAAA | TTTGAAGAGCAACATGGGGTAC | 187 |
| LTF | 188 | CTCCCAGGAACCGTACTTCA | CTCTGATAAAAGCCACGTCTCC | 189 |
| LYPLAL1 | 190 | CATCAAGATGTGGCAGGAGTA | TGCAGTACCATGACACTGAAATA | 191 |
| MAP3K7CL | 192 | GACTCCATTCCTTTGGTCTTTCC | CCATGGATTCCTCGGAGTCA | 193 |
| MEF2C | 194 | TGGTCTGATGGGTGGAGACC | TGAGTTTCGGGGATTGCCATAC | 195 |
| MMD | 196 | TCTCACAATGGGATTCTCTCCA | CAGGCAAGTTCCTGAAGTCC | 197 |
| MMP8 | 198 | TGCCGAAGAAACATGGACCAA | AGCCCCAAAGAATGGCCAAA | 199 |
| MN1 | 200 | AGAAGGCCAAACCCCAGAA | ATGCTGAGGCCTTGTTTGC | 201 |
| MOB1B | 202 | GAGAGTTGTCCAGTGATGTCA | GTCCTGAACCCAAGTCATCA | 203 |
| MPO | 204 | CATCGGTACCCAGTTCAGGAA | TGCTGCATGCTGAACACAC | 205 |
| NFATC1 | 206 | TCCTCTCCAACACCAAAGTCC | AGGATTCCGGCACAGTCAA | 207 |
| NFATC2 | 208 | TGGAAGCCACGGTGGATAA | TGTGCGGATATGCTTGTTCC | 209 |
| NPY1R | 210 | TCTGCTCCCTTCCATTCCC | GAATTCTTCATTCCCTTGAACTGAAC | 211 |
| NTSR1 | 212 | CGCCTCATGTTCTGCTACA | TAGAAGAGTGCGTTGGTCAC | 213 |
| OAZ1 | 214 | CGAGCCGACCATGTCTTCA | AAGCTGAAGGTTCGGAGCAA | 215 |
| OTC | 216 | CCAGGCTTTCCAAGGTTACCA | TGGCTTTCTGGGCAAGCA | 217 |
| P2RY12 | 218 | ACTGGATACATTCAAACCCTCCA | TGGTGCACAGACTGGTGTTA | 219 |
| PAPPA | 220 | GTACTGTGGCGATGGCATTATAC | AGAAAAGGGAGCAGCCATCA | 221 |
| PAPPA2 | 222 | ACAGTGGAAGCCTGGGTTAA | ACAGTGTGGGAGCAGTTATCA | 223 |
| PCDH11X | 224 | CTGGCATCCAGTTGACGAAA | CATCAGGGCCTAGCAGGTAA | 225 |
| PGLYRP1 | 226 | GTGCAGCACTACCACATGAA | TATACGAGCCCGTCTTCTCC | 227 |
| PKHD1L1 | 228 | GCCAGCTGCTATATCACACAAA | AAACCCAGGGCTACTTCCAA | 229 |
| PLAC1 | 230 | GCCACATTTCAAAGGAAACTGAC | TCCCTGCAGCCAATCAGATA | 231 |
| PLAC4 | 232 | CCACCAAGAAGCCACTTTCC | TACCAGCAATGCCAGGGTTA | 233 |
| POLE2 | 234 | AGAAACTGCGTCCGTTTTCC | GGAGTCAGATGTCCTTGGGATAA | 235 |
| POU3F2 | 236 | CGGATCAAACTGGGATTTACCC | CGAGAACACGTTGCCATACA | 237 |
| PPBP | 238 | TCTGGCTTCCTCCACCAAA | CAGCGGAGTTCAGCATACAA | 239 |
| PRDX5 | 240 | GTTCG GCTCCTG GCTGAT | CAAAGATGGACACCAGCGAATC | 241 |
| PRG2 | 242 | GGGGCAGTTTCTGCTCTTCA | TCATCCTCAGGCAGCGTCTTA | 243 |
| PSG 1 | 244 | GCAGGATCCTACACCTTACACA | TGCTGGAGATGGAGGGCTTA | 245 |
| PSG2 | 246 | CTGGCGAGGAAAGCTCCA | CAGAAATGACATCACAGCTGCTA | 247 |
| PSG4 | 248 | CTCCCCAGCATTTACCCTTCA | GGTTAGACTCGGCGAAGCA | 249 |
| PSG7 | 250 | ACCCAGTCACCCTGAATGTC | GCAGGACAAGTAGAGGTTTTGTC | 251 |
| PTGER3 | 252 | GTCGGTCTGCTGGTCTCC | TGTGTCTTGCAGTGCTCAAC | 253 |
| RAB11A | 254 | AGGCACAGATATGGGACACA | ATAAGGCACCTACAGCTCCA | 255 |
| RAB27B | 256 | ACCAGATCAGAGGGAAGTCA | CAGTTGCTGCACTTGTTTCA | 257 |
| RAP1GAP | 258 | GGAAGCAGGATGGATGAACA | CTCGGGTATGGAATGTAGTCC | 259 |
| RGS18 | 260 | TGAAGACACCCGCTCCAGTA | CCCCATTTCACTG CCTCTTCA | 261 |
| RHCE | 262 | TGGGAAGGTGGTCATCACAC | CAGCACCCGCTGAGATCA | 263 |
| RNASE2 | 264 | GCCAAGATCCCATCTCTCCA | AGGCACTTCAGCTCAGGAAA | 265 |
| RPL23AP7 | 266 | CTGGCTGTGGGTGTGGTACT | CGCTCCACTCCCTCTAGGC | 267 |
| S100A8 | 268 | GCTAGAGACCGAGTGTCCTCA | CCAGAATGAGGAACTCCTGGAA | 269 |
| S100A9 | 270 | TCAAAGAGCTGGTGCGAAAA | ATTTGTGTCCAG GTCCTCCA | 271 |
| S100P | 272 | GAAGGAGCTACCAGGCTTCC | AGCAATTTATCCACGGCATCC | 273 |
| SAM D9 | 274 | CTTCGAGAAGTCTTGCAACC | GCCAGAATAAGAGGGAAGCTA | 275 |
| SATB2 | 276 | TTTGCCAAAGTGGCTGCAAA | TTTCTGGGCTTGGGTTCTCC | 277 |
| SEMA3B | 278 | TGCACCAGTGGGTGTCATA | GTGGAACTGAAGGTGCCAAA | 279 |
| SERPINA7 | 280 | AGAAGTGGAACCGCTTACTACA | AGTGTGGCTCCAAGGTCATA | 281 |
| SLC12A8 | 282 | GCTGCCATCGTGTATTTCTACA | AGACCTCATCCACCGGAAAA | 283 |
| SLC2A2 | 284 | GGGAGCACTTGGCACTTTTCA | GCAGGATGTGCCACAGATCA | 285 |
| SLC38A4 | 286 | GGTCCTTCCCATCTACAGTGAA | AGCATCCCCGTGATGGAAATA | 287 |
| SLC4A1 | 288 | TGCTGCCGCTCATCTTCA | CAAAGGTTGCCTTGGCATCA | 289 |
| SLITRK3 | 290 | GACCTGGCGCTCCAGTTTA | CCTCTGTGAAGCATCTCAGCTA | 291 |
| TBC1D15 | 292 | AAGACGGCTTGATTTCAGGAA | GCATCATCCAATGGTCTCCA | 293 |
| TFIP11 | 294 | TGTTAAGCAGGACGACTTTCC | CCTTTCTGGCTGGGCTTAAA | 295 |
| VCAN | 296 | GGTGCCTCTGCCTTCCAA | TTGTGCCAGCCATAGTCACA | 297 |
| VGLL1 | 298 | AGAGTGAAGGTGTGATGCTGAA | GCACGGTTTGTGACAGGTAC | 299 |

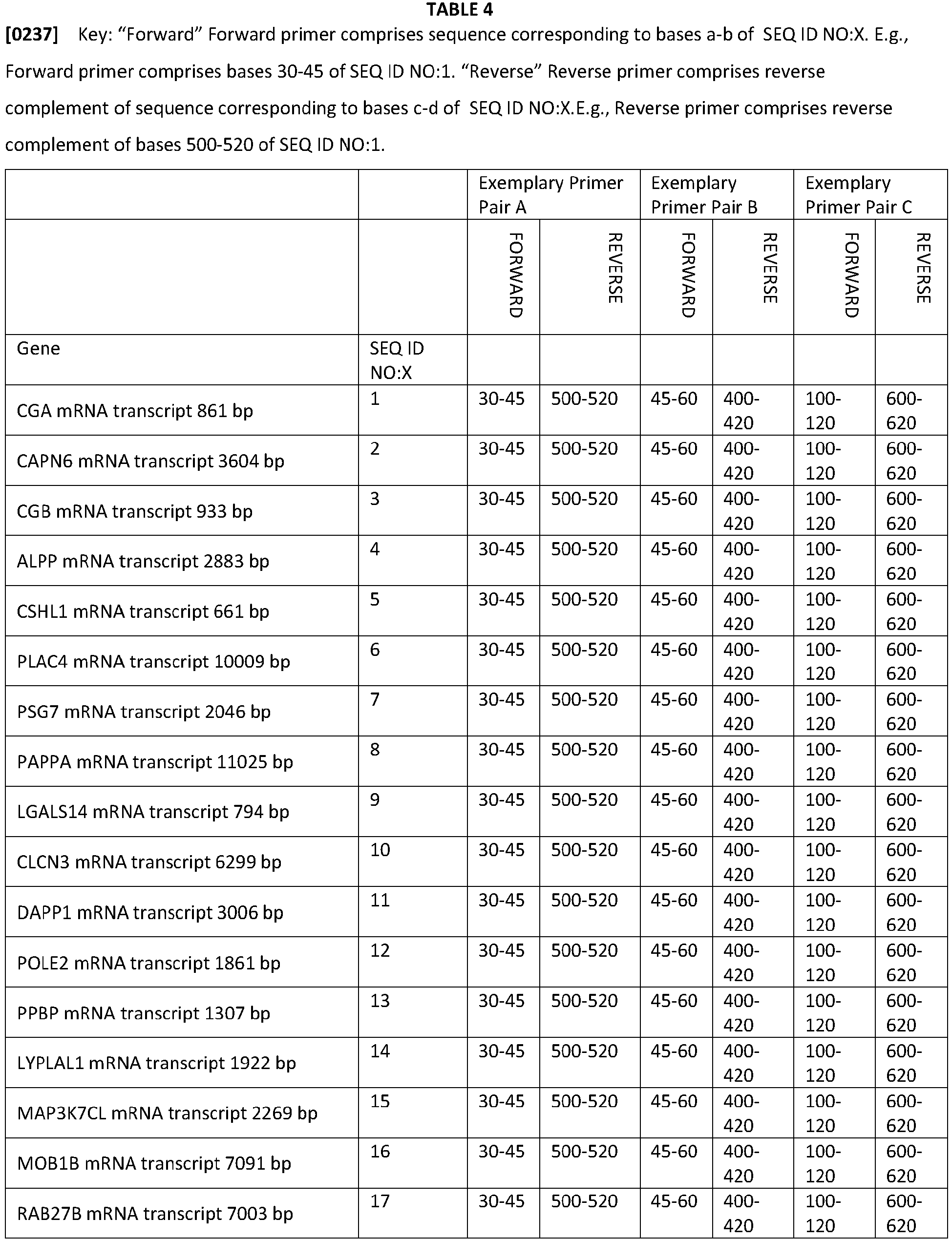

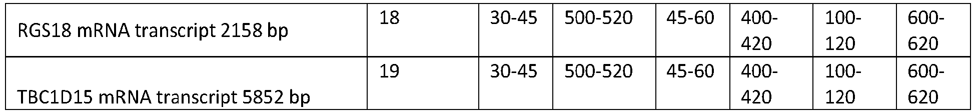

**TABLE 5**

| Key: Probe comprises sequence corresponding to bases a-b of SEQ ID NO:X. or the complement thereof | | | | |
|---|---|---|---|---|
| | | Exemplary Probe A | Exemplary Probe B | Exemplary Probe C |
| Gene | SEQ ID NO:X | | | |
| CGA mRNA transcript 861 bp | 1 | 100-140 | 200-240 | 300-340 |
| CAPN6 mRNA transcript 3604 bp | 2 | 100-140 | 200-240 | 300-340 |
| CGB mRNA transcript 933 bp | 3 | 100-140 | 200-240 | 300-340 |
| ALPP mRNA transcript 2883 bp | 4 | 100-140 | 200-240 | 300-340 |
| CSHL1 mRNA transcript 661 bp | 5 | 100-140 | 200-240 | 300-340 |
| PLAC4 mRNA transcript 10009 bp | 6 | 100-140 | 200-240 | 300-340 |
| PSG7 mRNA transcript 2046 bp | 7 | 100-140 | 200-240 | 300-340 |
| PAPPA mRNA transcript 11025 bp | 8 | 100-140 | 200-240 | 300-340 |
| LGALS14 mRNA transcript 794 bp | 9 | 100-140 | 200-240 | 300-340 |
| CLCN3 mRNA transcript 6299 bp | 10 | 100-140 | 200-240 | 300-340 |
| DAPP1 mRNA transcript 3006 bp | 11 | 100-140 | 200-240 | 300-340 |
| POLE2 mRNA transcript 1861 bp | 12 | 100-140 | 200-240 | 300-340 |
| PPBP mRNA transcript 1307 bp | 13 | 100-140 | 200-240 | 300-340 |
| LYPLAL1 mRNA transcript 1922 bp | 14 | 100-140 | 200-240 | 300-340 |
| MAP3K7CL mRNA transcript 2269 bp | 15 | 100-140 | 200-240 | 300-340 |
| MOB1B mRNA transcript 7091 bp | 16 | 100-140 | 200-240 | 300-340 |
| RAB27B mRNA transcript 7003 bp | 17 | 100-140 | 200-240 | 300-340 |
| RGS18 mRNA transcript 2158 bp | 18 | 100-140 | 200-240 | 300-340 |
| TBC1D15 mRNA transcript 5852 bp | 19 | 100-140 | 200-240 | 300-340 |

**TABLE 6: LIST OF EXEMPLARY mRNA TRANSCRIPTS:**

| **SEQ ID NO:** | **Specification Identity** | **Accession No.** |
|---|---|---|
| 1 | CGA mRNA transcript 861 bp | NM_001252383.1 |
| 2 | CAPN6 mRNA transcript 3604 bp | NM_014289.3 |
| 3 | CGB mRNA transcript 933 bp | NM_000737.3 |
| 4 | ALPP mRNA transcript 2883 bp | NM_001632.3 |
| 5 | CSHL1 mRNA transcript 661 bp | NM_001318.2 |
| 6 | PLAC4 mRNA transcript 10009 bp | NM_182832.2 |
| 7 | PSG7 mRNA transcript 2046 bp | NM_002783.2 |
| 8 | PAPPA mRNA transcript 11025 bp | NM_002581.3 |
| 9 | LGALS14 mRNA transcript 794 bp | NM_020129.2 |
| 10 | CLCN3 mRNA transcript 6299 bp | NM_173872 |
| 11 | DAPP1 mRNA transcript 3006 bp | NM_001306151 |
| 12 | POLE2 mRNA transcript 1861 bp | NM_002692 |
| 13 | PPBP mRNA transcript 1307 bp | NM_002704 |
| 14 | LYPLAL1 mRNA transcript 1922 bp | NM_138794 |
| 15 | MAP3K7CL mRNA transcript 2269 bp | NM_001286617 |
| 16 | MOB1B mRNA transcript 7091 bp | NM_001244766 |
| 17 | RAB27B mRNA transcript 7003 bp | NM_004163 |
| 18 | RGS18 mRNA transcript 2158 bp | NM_130782 |
| 19 | TBC1D15 mRNA transcript 5852 bp | NM_001146214 |

**TABLE 7: SEQUENCES OF EXEMPLARY mRNA TRANSCRIPTS:**

| |
|---|
| **SEQ ID NO:1 CGA mRNA transcript 861 bp** |
| 1 acactctgct ggtataaaag caggtgagga cttcattaac tgcagttact gagaactcat 61 aagacgaagc taaaatccct cttcggatcc acagtcaacc gccctgaaca catcctgcaa 121 aaagcccaga gaaaggagcg ccatggatta ctacagaaaa tatgcagcta tctttctggt 181 cacattgtcg gtgtttctgc atgttctcca ttccgctcct gatgtgcagg agacagggtt 241 tcaccatgtt gcccaggctg ctctcaaact cctgagctca agcaatccac ccactaaggc 301 ctcccaaagt gctaggatta cagattgccc agaatgcacg ctacaggaaa acccattctt 361 ctcccagccg ggtgccccaa tacttcagtg catgggctgc tgcttctcta gagcatatcc 421 cactccacta aggtccaaga agacgatgtt ggtccaaaag aacgtcacct cagagtccac 481 ttgctgtgta gctaaatcat ataacagggt cacagtaatg gggggtttca aagtggagaa 541 ccacacggcg tgccactgca gtacttgtta ttatcacaaa tcttaaatgt tttaccaagt 601 gctgtcttga tgactgctga ttttctggaa tggaaaatta agttgtttag tgtttatggc 661 tttgtgagat aaaactctcc ttttccttac cataccactt tgacacgctt caaggatata 721 ctgcagcttt actgccttcc tccttatcct acagtacaat cagcagtcta gttcttttca 781 tttggaatga atacagcatt tagcttgttc cactgcaaat aaagcctttt aaatcatcat 841 tcaaaaaaaa aaaaaaaaaa a |
| SEQ ID NO:2 CAPN6 mRNA transcript 3604 bp |
| 1 gagcagagct tggtacagcc caaatagttt tcaggttaag aaagccagaa tctttgttca 61 gccacactga ctgaacagac ttttagtggg gttacctggc taacagcagc agcggcaacg 121 gcagcagcag cagcagcagc agcagcagca gcagcagggc tcctgggata actcaggcat 181 agttcaacac tatgggtcct cctctgaagc tcttcaaaaa ccagaaatac caggaactga 241 agcaggaatg catcaaagac agcagacttt tctgtgatcc aacatttctg cctgagaatg 301 attctctttt ctacaaccga ctgcttcctg gaaaggtggt gtggaaacgt ccccaggaca 361 tctgtgatga cccccatctg attgtgggca acattagcaa ccaccagctg acccaaggga 421 gactggggca caagccaatg gtttctgcat tttcctgttt ggctgttcag gagtctcatt 481 ggacaaagac aattcccaac cataaggaac aggaatggga ccctcaaaaa acagaaaaat 541 acgctgggat atttcacttt cgtttctggc attttggaga atggactgaa gtggtgattg 601 atgacttgtt gcccaccatt aacggagatc tggtcttctc tttctccact tccatgaatg 661 agttttggaa tgctctgctg gaaaaagctt atgcaaagct gctaggctgt tatgaggccc 721 tggatggttt gaccatcact gatattattg tggacttcac gggcacattg gctgaaactg 781 ttgacatgca gaaaggaaga tacactgagc ttgttgagga gaagtacaag ctattcggag 841 aactgtacaa aacatttacc aaaggtggtc tgatctgctg ttccattgag tctcccaatc 901 aggaggagca agaagttgaa actgattggg gtctgctgaa gggccatacc tataccatga 961 ctgatattcg caaaattcgt cttggagaga gacttgtgga agtcttcagt gctgagaagg 1021 tgtatatggt tcgcctgaga aaccccttgg gaagacagga atggagtggc ccctggagtg 1081 aaatttctga agagtggcag caactgactg catcagatcg caagaacctg gggcttgtta 1141 tgtctgatga tggagagttt tggatgagct tggaggactt ttgccgcaac tttcacaaac 1201 tgaatgtctg ccgcaatgtg aacaacccta tttttggccg aaaggagctg gaatcggtgt 1261 tgggatgctg gactgtggat gatgatcccc tgatgaaccg ctcaggaggc tgctataaca 1321 accgtgatac cttcctgcag aatccccagt acatcttcac tgtgcctgag gatgggcaca 1381 aggtcattat gtcactgcag cagaaggacc tgcgcactta ccgccgaatg ggaagacctg 1441 acaattacat cattggcttt gagctcttca aggtggagat gaaccgcaaa ttccgcctcc 1501 accacctcta catccaggag cgtgctggga cttccaccta tattgacacc cgcacagtgt 1561 ttctgagcaa gtacctgaag aagggcaact atgtgcttgt cccaaccatg ttccagcatg 1621 gtcgcaccag cgagtttctc ctgagaatct tctctgaagt gcctgtccag ctcagggaac 1681 tgactctgga catgcccaaa atgtcctgct ggaacctggc tcgtggctac ccgaaagtag 1741 ttactcagat cactgttcac agtgctgagg acctggagaa gaagtatgcc aatgaaactg 1801 taaacccata tttggtcatc aaatgtggaa aggaggaagt ccgttctcct gtccagaaga |
| 1861 atacagttca tgccattttt gacacccagg ccattttcta cagaaggacc actgacattc 1921 ctattatagt acaggtctgg aacagccgaa aattctgtga tcagttcttg gggcaggtta 1981 ctctggatgc tgaccccagc gactgccgtg atctgaagtc tctgtacctg cgtaagaagg 2041 gtggtccaac tgccaaagtc aagcaaggcc acatcagctt caaggttatt tccagcgatg 2101 atctcactga gctctaaatc tgcaatccca gagaatcctg acaaagcgtg ccaccctttt 2161 attttccgtc aggtgccagg tcttagttaa gattcacaat ctttagaaag aatgagattc 2221 acaataatta actcttcctc tcttctgata aattccccat acctcccaat ccaagtagca 2281 tctgtagcta cataacctat atacctccag cagctggaca tgggaggcga acagtcctat 2341 ctagacatca tacacatttg ccaagaaagg atctctgggg cttccggggg tgagattcaa 2401 gcaggacaat aacaagaggc tggacaccct acagatgtct ttgatgtttt cagttgtttg 2461 atatatctcc cctgtagggc atgttgagga aggaggaggg ctgatcaagg ccaagctggt 2521 ctagcctgac atcctagctc ctgactgaac actatagact tcccagcagc atttcaccca 2581 gcagccagag ccggctttaa gtccccaacc cttacagaca ccactgccac caccaccaac 2641 cacgaccacc accaccacca ccactcacca ccatcatcac ctccggaaag tgtagtcctg 2701 ccctaaccca agtcaccccc gacagtaaat tttaccttca tgttgagaaa gcttcctggt 2761 gcttaatcaa gagctggagt tcaatgagtc ctagacagtg agaggggcct gagcttcagc 2821 tcaatggaag cctgctgtgt gccacaagac ggaaaagtgg aagaagctgc agtgggagac 2881 aaagcctcgg tcccccaccc atccacacac acctacactc acacacgcgc acatgggcgc 2941 gcacgaacta ccattcaggc agtcagtggg caagaggaaa gataagtaag taccatacac 3001 acctaaaaga tgagagaatt catccagaca tattacagcc agtttggggc ccctgactgc 3061 aatgtgaaac ctctcgctgc tgctaggttt acaaacaagc ccattgtcct gtgcctccta 3121 atatcatttg tactgaagac cccatctggg gacttgagac tttggtccca gcccagactc 3181 ctcagacttt tctctcagtt gggatgcttc actcgctggg ggtgtttgtt tgccctctca 3241 tttttcagta cttctacaga attttctcta gagtcagtca ttatgaaatg tacttccctc 3301 catcttaacc tatcaacttt ctgcccctcc ttcaaggccc agtataaatg ccacctcctc 3361 catgaagcct tccctaattc caccccaaac ccccaccttc aacaatattt caacgcttct 3421 gcaatgatga aaaagaaaca tagttgtagt acttagccta cctagaccag caagcattca 3481 tttttagctc gctcattttt taccatgttt tccagtctgt ttaacttctg cagtgccttc 3541 actacactgc cttacataaa ccaaatcaca ataaagttca tattcagtac attgaaaaaa 3601 aaaa |
| **SEQ ID NO:3 CGB mRNA transcript 933 bp** |
| 1 tgcaggaaag cctcaagtag aggagggttg aggcttcagt ccagcacctt tctcgggtca 61 cggcctcctc ctggctccca ggaccccacc ataggcagag gcaggccttc ctacacccta 121 ctccctgtgc ctccagcctc gactagtccc tagcactcga cgactgagtc tctgaggtca 181 cttcaccgtg gtctccgcct cacccttggc gctggaccag tgagaggaga gggctggggc 241 gctccgctga gccactcctg cgcccccctg gccttgtcta cctcttgccc cccgaggggt 301 tagtgtcgag ctcaccccag catcctatca cctcctggtg gccttgccgc ccccacaacc 361 ccgaggtata aagccaggta cacgaggcag gggacgcacc aaggatggag atgttccagg 421 ggctgctgct gttgctgctg ctgagcatgg gcgggacatg ggcatccaag gagccgcttc 481 ggccacggtg ccgccccatc aatgccaccc tggctgtgga gaaggagggc tgccccgtgt 541 gcatcaccgt caacaccacc atctgtgccg gctactgccc caccatgacc cgcgtgctgc 601 agggggtcct gccggccctg cctcaggtgg tgtgcaacta ccgcgatgtg cgcttcgagt 661 ccatccggct ccctggctgc ccgcgcggcg tgaaccccgt ggtctcctac gccgtggctc 721 tcagctgtca atgtgcactc tgccgccgca gcaccactga ctgcgggggt cccaaggacc 781 accccttgac ctgtgatgac ccccgcttcc aggactcctc ttcctcaaag gcccctcccc 841 ccagccttcc aagcccatcc cgactcccgg ggccctcgga caccccgatc ctcccacaat 901 aaaggcttct caatccgcaa aaaaaaaaaa aaa |
| **SEQ ID NO:4 ALPP mRNA transcript 2883 bp** |
| 1 tcagccagtg tggcttcagg tcaagaggct gggcagggtc aaggtggcaa cgagggagaa 61 agccgggaca cagttctccc tgatttaaac ccgggcagcc tggagtgcag ctcatactcc 121 atgcccagaa ttcctgcctc gccactgtcc tgctgccctc cagacatgct ggggccctgc 181 atgctgctgc tgctgctgct gctgggcctg aggctacagc tctccctggg catcatccca 241 gttgaggagg agaacccgga cttctggaac cgcgaggcag ccgaggccct gggtgccgcc 301 aagaagctgc agcctgcaca gacagccgcc aagaacctca tcatcttcct gggcgatggg 361 atgggggtgt ctacggtgac agctgccagg atcctaaaag ggcagaagaa ggacaaactg 421 gggcctgaga tacccctggc catggaccgc ttcccatatg tggctctgtc caagacatac 481 aatgtagaca aacatgtgcc agacagtgga gccacagcca cggcctacct gtgcggggtc 541 aagggcaact tccagaccat tggcttgagt gcagccgccc gctttaacca gtgcaacacg 601 acacgcggca acgaggtcat ctccgtgatg aatcgggcca agaaagcagg gaagtcagtg |
| 661 ggagtggtaa ccaccacacg agtgcagcac gcctcgccag ccggcaccta cgcccacacg 721 gtgaaccgca actggtactc ggacgccgac gtgcctgcct ccgcccgcca ggaggggtgc 781 caggacatcg ctacgcagct catctccaac atggacattg acgtgatcct aggtggaggc 841 cgaaagtaca tgtttcgcat gggaacccca gaccctgagt acccagatga ctacagccaa 901 ggtgggacca ggctggacgg gaagaatctg gtgcaggaat ggctggcgaa gcgccagggt 961 gcccggtatg tgtggaaccg cactgagctc atgcaggctt ccctggaccc gtctgtgacc 1021 catctcatgg gtctctttga gcctggagac atgaaatacg agatccaccg agactccaca 1081 ctggacccct ccctgatgga gatgacagag gctgccctgc gcctgctgag caggaacccc 1141 cgcggcttct tcctcttcgt ggagggtggt cgcatcgacc atggtcatca tgaaagcagg 1201 gcttaccggg cactgactga gacgatcatg ttcgacgacg ccattgagag ggcgggccag 1261 ctcaccagcg aggaggacac gctgagcctc gtcactgccg accactccca cgtcttctcc 1321 ttcggaggct accccctgcg agggagctcc atcttcgggc tggcccctgg caaggcccgg 1381 gacaggaagg cctacacggt cctcctatac ggaaacggtc caggctatgt gctcaaggac 1441 ggcgcccggc cggatgttac cgagagcgag agcgggagcc ccgagtatcg gcagcagtca 1501 gcagtgcccc tggacgaaga gacccacgca ggcgaggacg tggcggtgtt cgcgcgcggc 1561 ccgcaggcgc acctggttca cggcgtgcag gagcagacct tcatagcgca cgtcatggcc 1621 ttcgccgcct gcctggagcc ctacaccgcc tgcgacctgg cgccccccgc cggcaccacc 1681 gacgccgcgc acccggggcg gtccgtggtc cccgcgttgc ttcctctgct ggccgggacc 1741 ctgctgctgc tggagacggc cactgctccc tgagtgtccc gtccctgggg ctcctgcttc 1801 cccatcccgg agttctcctg ctccccacct cctgtcgtcc tgcctggcct ccagcccgag 1861 tcgtcatccc cggagtccct atacagaggt cctgccatgg aaccttcccc tccccgtgcg 1921 ctctggggac tgagcccatg acaccaaacc tgccccttgg ctgctctcgg actccctacc 1981 ccaaccccag ggactgcagg ttgtgccctg tggctgcctg caccccagga aaggaggggg 2041 ctcaggccat ccagccacca cctacagccc agtgggtacc aggcaggctc ccttcctggg 2101 gaaaagaagc acccagaccc cgcgccccgc tgatctttgc ttcagtcctt gaatcacctg 2161 tgggacttga ggactcggga tcttcaggac gcctggagaa gggtggtttc ctgccaccct 2221 gctggccaag gaggctcctg gggtggggat caccaggggg attttgacac agccttcggc 2281 tgccccccac taagctaatt ccacacccct gtaccccccc agggggccct ctgcctcatg 2341 gcaaaggctt gccccaaatc tcaacttctc agacgttcca tacccccaca tgccaatttc 2401 agcacccaac tgagatccga ggagctcctg ggaagccctg ggtgcaggac actggtcgag 2461 agccaaaggt ccctccccag acatctggac actgggcata gatttctcaa gaaggaagac 2521 tcccctgcct ccccagggcc tctgctctcc tgggagacaa agcaataata aaaggaagtg 2581 tttgtaatcc cagcactttg ggaggccgag gtgggcggat cacgaggtca ggagatggag 2641 accatcctgg ctaacacggt gaaacccctt atctatgcgc ctgtagtccc agctacccag 2701 gaggctgaag caggataatc gcttgaaccc gggcggcgga gattgcagtg agccgaggtc 2761 atgccactgc actgcagcct gggcgacaga gcgagattct gcctcaaaaa taaacaaata 2821 aattttaaaa ataaataaat aataaaagga agtgttagac aatgtaaaaa aaaaaaaaaa 2881 aaa |
| **SEQ ID NO:5 CSHL1 mRNA transcript 661 bp** |
| 1 agcatcccaa ggcccgactc cccgcaccac tcagggtcct gtggacagct cacctagcgg 61 caatggctgc aggaagaagc ctatatcaca aaggaacaga agtattcatt cctgcatgac 121 tcccagacct ccttctgctt ctcagactct attccgacat cctccaacat ggaggaaacg 181 cagcagaaat ccaacttaga gctgctccac atctccctgc tgctcatcga gtcgcggctg 241 gagcccgtgc ggttcctcag gagtaccttc accaacaacc tggtgtatga cacctcggac 301 agcgatgact atcacctcct aaaggaccta gaggaaggca tccaaatgct gatggggagg 361 ctggaagacg gcagccacct gactgggcag accctcaagc agacctacag caagtttgac 421 acaaactcgc acaaccatga cgcactgctc aagaactacg ggctgctcca ctgcttcagg 481 aaggacatgg acaaggtcga gacattcctg cgcatggtgc agtgccgctc tgtggagggc 541 agctgtggct tctaggggcc cgcgtggcat cctgtgaccc ctccccagtg cctctcctgg 601 ccctgaaggt gccactccag tgcccaccag ccttgtctta ataaaattaa gttgtattgt 661 t |
| **SEQ ID NO:6 PLAC4 mRNA transcript 10009 bp** |
| 1 cgtagctcat aatccatttt tataacacct tgctatctat atttacacct ttaaagaaca 61 cgggaattta agagggaaga gtaactaggc ttttgctaaa cttgggctaa taaaaccctc 121 tgtagagaga tccttaatat aggcatgggg acaacaagga gtatcccaag ggactcgccg 181 ctagggtgtc ttttaagcta ttggagcaaa ttcaaatttg gcttaaagaa aaagaaactc 241 attttgtatt gcaacaccat ttgggttaaa tacaagttag atgacgaata tatctggcct 301 aaacatggtt ctatatacta tagtgatatt ttacgattag gcttattttg taaaagagaa 361 ggaaaatggg aagagatccc ttatgtacag gcttttatgg ctctatactg gatcacgtta |
| 421 cttccaggca ttagaatgcc atgcataagg gatccccacc tagctgctcc ccatagaaag 481 ttcataagcc tccccagagt ctcttcagtc ccccagtcct gagtgggggt tctcgccaat 541 tccctaatga gattccaccc caatatcatc aggcaccttt cccccttatc caactagccc 601 tagcctatac cctctgctgc ccaagaaaat gagcccaacc agtacaccag gagtggggct 661 ccatatcagc ccctaaggtc aagcctgtgt ccactgtgga aagtagttga tggaaatgag 721 ggaacactca aagagtacat atgccacttt ccatgtctaa ttagacctta taaaaggaaa 781 gaattggcca gttttcagat aaaccagaaa agcttataca agagtttgtt acgttgacta 841 tgttcttcaa attgccacga tttacaaata ttgtcatccg cttgctgtgc tgtggggaaa 901 aaaaagtaga ggaaaaagtg tgtggttaag ccagtcaatt atgacaaggt taaagaagta 961 actcggggaa aagatgaaaa tcccgctctg tttcagggtc ttttagttga agcactcagg 1021 aaatatacta atgcaggccc agacacccca gaagggcaag ctctcctggg tatacatttt 1081 ctcattcaat cttctcctga cattaggagg aatctacaaa aagcagcaat gggaccttca 1141 agtcctatga aacgacgctt aaacatagcc tttaaagttt acaacaacag ggacagggca 1201 aaagagggga gtaaaaagaa atagccaaaa agtacaattg ttaacagtga ctttaagcct 1261 ccttgcccct caggattact catcttgaga aaatgttaca aaattagcat ctgggatgcc 1321 tagacaagac ttgatgcctg acttgctgac ccctgggcca gaatcactgc gcctactata 1381 cgcaaaaggg cccctggcaa tgcaaatgtc ctaactgctc tggtgagaga gaacaataac 1441 aacaaaaagc ttccatcaat actagagcta accttctcct actagcccca gtgagctgct 1501 tagctcaagt aagtttactg tcccagagga cagctttcca cagtggcaga taagcagccg 1561 cctgaacatt tttctttggt atttccacca ctgagtgtgc tctccagtgg cgtggggact 1621 ccagaatctc cttttgagca atgcagtttg cttcctcccc tttttagttg atgctatggg 1681 attccctgtc ctgccttttc ctgttttcca tacctatcgg ggcaaacaaa atttggccag 1741 gtagatgggt cccagttctg taaataactt gaatccagtt gtcttgtata ggtcatttta 1801 tttaatatgt ttttgggtat atgtacatgt attgtgatgt gtgttacatc tagcgtgctg 1861 tcaaactggc ttatagataa aagaacactc atacattcaa caaataagac tactgaaagc 1921 ttattagttt gaagagaatc ttgtatcttc taaaatttaa ctttaggatt tttacctagg 1981 taagtcactg atgttcatag gctttaaaat ggttaaaatg gctttaaatg gtgaccagct 2041 ttgcatggta ccttggttct cggtgatcta gataaagtta aaagtgaaat aattaaatac 2101 acgtaaatgg gatatgctta atgtgtggtt taaaatcata aaatggtaga atggttctca 2161 gttatagaat gacaatgtct agtgtgaagt tcatgacttc ttccttccta ggtttccata 2221 aaatgtgcta aagaaatgta ttctttattg agaaaaaatt ttttgtctaa tccggaagtt 2281 actaaatggg aggttcaaaa catgagtgaa ccagtgagta gaaaagagag atgtaaagaa 2341 tattatgaat agaaaatgta ttttttgttt gttttgcaag gaaggatata aagaaagagt 2401 aattttatat gtggaggaat cctgtatagt aaattcccta tcctagagta aaataacttt 2461 aagaaagagg tagtatagaa catgtcagga aattcagcta tgttgtagat ggtctgtgta 2521 agtcatctgc acagtgcatg agtgtggagg tgggcgggca ctcattggcc cttgaactcc 2581 ttttgagcag tatggaagcc aagaactaga agccaggaaa tggggttgta aaactgattt 2641 gtctatggat tttatgtgtt gagctgctgt ggtcttggct tgtagtaatt acctatatga 2701 accttccccc ctccccttta gaatttagga caggttcaaa aggccctcca atataaaaat 2761 aaaatactgt ccttccccac aaaggaaaaa atagctcccc ggttcaacca ggagacttag 2821 tcttgctaaa accttaaaga cagggtaaag acagggatac cccaagaatc aattacaatg 2881 aaatggaagg ggccttatca ggtattgtta agtaccccca ctgctgttaa acttcaggga 2941 acacctactt gggcacacag atccaggact aaacctgttt cttatgagtc acaggcacaa 3001 aggaagggca ctacaaccac aaccaatatc agtaaagctt tggaagacct ctgctaccta 3061 tttaaaataa tcaacactca gccagaagag gtaatgtaat gctgtagatg ggaataggag 3121 cattgatctt gctcttcttc ctgactgtag tacttccttt ctatggcttt aaccagccac 3181 ctcctcctgg gaaacatctc ctgtgggctt gttgggtata gaagctactc taagacccaa 3241 ccagatacca tgatgccact gttaattctg tttgctcttc taattaacct aagctagtgt 3301 gtatgtggac agggagggtg gacaaaattc tacagtaaat atttcaaaaa ttatagcatc 3361 atagaatcat ctttatggct gccagatttg tcatcaacac ccccaggata gacagtttca 3421 tcttccgacc tatctggaaa atctcaggac catgtcccca gacctcctaa ctaaccatag 3481 caccccaaaa tacccaaacc cctattgtga agtggaactc ttccccactt agtggatccc 3541 ccctggaccc tgctgtcccc ctgccctgac cactattatc ggaatctggg aagttgggca 3601 tctatatctc cagtgcactc ataactctaa catttgcatc cactcttgca ttaatgacac 3661 aaaagtggaa gcttccctgc gatgctctgg tccaactcta gttgccaagt ttccaagacc 3721 acggggaggt aaatgagatt ccatttgtga gtgaaaagac catatatggt accttctccc 3781 ggatgggaac atacaaagga aaaacaactg cctgatctgg gaaggtgaca gtactacctt 3841 cttctagaaa acaaagattg ttcaaccacc accatgagaa caggtggaaa atatctctat 3901 agacccaacc tggcaatgaa gtataaacat cgcaccccgc agggcttctc ttggtgccct 3961 agttgggttc atttttgttt gtgactatga atgggaagaa gtcacaccct gtaaccactc 4021 caactcccta aggagtcacc tcttctttaa ggaatagctt tcccttgtat ctaaaaaact |
| 4081 tggaactgac atgaatgaac gttggccact cttacccctc caggggtcac aatctataac 4141 gcctaggacc caagaatatc agaaataagt aagcaataaa actaattctg gcaggaatca 4201 gggtggcaat aggactagca gcaccctggg gtggctttgc ctaccatgag ttaacgctaa 4261 agaacttggc tcaaatccta gaatccttag ccaccaacgg agatcaggca ttaaagagaa 4321 ttcaagagtt ccccagactc tggaaaatgt agttgttgat aacagactag cattggatta 4381 tttactagct gaacaaggtg gggtcttgtg cagttattaa taaaacctgc tgcacatata 4441 ttaactctgg acaggttgag gttaacattc aaaagatcta tgagcaagct acctagttac 4501 atagatataa ccagggcact gcccccaact atatctggtc aaccatcaaa agtgccttcc 4561 caagtctcac ctgtttttca cctcttctag gacctttgac aactgtcttg ttacaaatgt 4621 ttggtccttg cttctttaac ctcttagtaa agtttgtgta ttctagatta ccacagttcc 4681 agagacaatg ctggcacaag gcttccagcc catcctgtcc actgacacgg agaatgaaat 4741 cgtcctgcct ctgggctcct tagatcaggt atccagagat ttttactcct ccagtgccag 4801 gcagggccta cgtccataaa ctcagcagga agtagttacg gaaaacagat ctccgccctt 4861 ctgcagcccc cttaagatta aggaggagta tctaatctct gaagggggaa tgaggtagga 4921 ggtgggactc aactctggaa gtggggctca ggcactcaga ccaaactgag cactagctaa 4981 aataggtcca gggcagatgc tagtttccat aggacacacc gacctgtgtc aagtcagttc 5041 accatggctc tggcagcacc cagaagttac caccctcacc ctggaaatgt ctgcataaac 5101 tgccccttca tttgcatata attaaaagtg gatacaaata ccactgcaga actgcctctg 5161 agctgctact gtgggcgcac agcctgtagg gcagccctgc tttgcaagga gcagcgcctc 5221 tgctgctgct gtgcacagcc ggccgcttca ataaaagttg ctaacaccac tggcttgccc 5281 ttgagttcct tcctgggcaa agctaagaac cctcccgggc tatgcttcaa tcttagggct 5341 cgcctgtcct gcatcactgg gatcatctcc cagtaaacta gccacactta catccatgtg 5401 tcagggacat ttctggagaa agcagcccag gacactgttg aataaaacac acaatagtct 5461 ctgtggtctt ctccacccca ccccacacca ggcaccctca gcttgattct cctttttaat 5521 tgcctgtaag cagggaagca caatgttttc acattctttg taaggccttt gttctactaa 5581 aatctaacct cagagcacaa ttttaaacta gatgaaagag ttgctgcgcc tgaagcactg 5641 caaacacctc ctcaccacac atgtgcactc accctggaca ccctcactca ccctgacacc 5701 ctcactcctc accctggaca ccctcactca ccccagacac cgtcactcct caccctggac 5761 acctcactct gcaccctgga caccctcact caccctggac acgttcactc accctgacac 5821 cctcactcac cctggacacc ctcactcacc ctggataccc tcactcctca ccctggacac 5881 cctcactcac cctggatacc ctcactcctc accctggaca ctctcactca ccctgacacc 5941 ctcaatcctc accctggact ccctcactcc tcaccctgga ctccctcact cctcaccctg 6001 gacaccctca ctcctcatcc tggacaccct cactcaacct ggacaccctc actcctcacc 6061 ctgacaccct cactcctcac cctggacacc ctcactcctc accctgacac cctcactcct 6121 caccctggca ccctcagtca ccctgacacc ctcactcctc accctgacac cctcaagtct 6181 tcacctccct ggctgcagcc tgggacacgc tttccctaac ttctgaaggc tcagtcctcc 6241 tcaagccaat ctcatctcaa attgcacctc ctcagagagg tcttccataa ccgcccttat 6301 aaagcaggat tctttcacca ataccccttc ccacatggca ctgtctcaca gcactcctct 6361 aaaagtctgt ttacttcctt gacaatctgt cttccttata aggggaggtt ctgtaaaagc 6421 caagactctc tctgtctagt tgactgttgc ataccagggc ttagaccaag gccctgacat 6481 gcagtaggtg cttaatatgt tttgaggcaa ggtcttgctc tgttgcacat gctggagtgc 6541 agtggcacaa tcgtaattca ttgcagcctt gaactcctga gctcaagtga tcctcctgcc 6601 tcagcctcct gagtagctgg gactacaggc atgcaccacc aagcttggct aatttaaaaa 6661 aaaaattata tagataggga cttgctatgt tgcctaggct gatcttgaac tcctaacctc 6721 aagcaatcct cccacctcgg ccttccaaag tgctgggata ataggcatgg agccgccaca 6781 cccagccaat gtgccgaaga aagaaagaaa aacatgctca tcctttgagt caggttcaaa 6841 ttttttctcc tctttaaccc ccagtcactc cagttataag tgatttttaa ctcttctcac 6901 actttaatgc atctggcaag aagatccacg tggtgttagg aacaatacag gaccttaagg 6961 atgggggaat cagcaggtgt cagcgtgccc tgtatgctca gggcagctgt ttccactgga 7021 cattctccct ttgcctctct gggcagcaac tcctaggcca gccgacctgc tgtgtcgagt 7081 aaccaggatt tctcaatctt ggcatggttg ccattttgga ccagatcgtt ctttgttgtg 7141 ggggctgccc tgtacggcaa agaatgccga gcagcacttc cagtctccac ccacaggacg 7201 ccagtagcac cctctaagtt gtgagaactc aaaatgtccc cagaggatgc cagatgtccc 7261 ctggggtggg gacacaatca ccccaggttg agatccatgg agccaggtct gtttgccacc 7321 aaggggtaaa gctccattcc caccttagga gggctaggag gcagcatcgt ggggccacag 7381 aaggcctggg tttgcagtca gaggacagga tgcacattcc ttcaagatac agacccagat 7441 tgttgggcat ctagttcttg ggttttctgt tgttgctgtt ccgttttgtc tgtcttccct 7501 cctttgttta ctagcagcct ggaatttgcc actttttcta aacgaagatt tatggaacac 7561 ttaccacacg gctgacgctg cgcgaggcta aggttctaat acaccgcagc tcacttaact 7621 ctcgcaatac cataaacgca cactgtttca tcttgaccct ttcttgggaa ggtgacagag 7681 aggtaggagg gcaaacatct tgtgtgcccc gtcccaaggg tattactggt ggaataatat |
| 7741 ccgcccccca ccccagtttc taatttgctg taggctgtga cgctgtgggg caagactagg 7801 agtcctgttg aaattaggaa taagtgtgct gtgagggaag ggctgcctta ttttagagca 7861 cagattttct gaatatctat tttgacaggt tcgatcctct ccccttcctg ccttccttct 7921 gtcgattttc aatgtcttga tggtgtccca cctgagtggc ctttagagat gtgagttgtg 7981 aggcactggg gaggcaggca cacgtcctcc agcccaagac tgcctaattt aacagggatt 8041 tctgcattct ggaacaagcc tccattttcc ccaagcagga ttactccaga gggcaaaaca 8101 cagcccaata gtatcacatt tcctttctgc tttagcaaaa ataaccactg tctcattcat 8161 gggaaaaggc cgccaaacaa atttgttact ggaaccattt gtaacaactt ctagtttgca 8221 ctgccttgga gcaagcacac tttgtagagg agggatttgc agttacttgg gcaacaaggt 8281 aaccactgat cattacagga agcttcagaa accgtgggac cagtgtagaa gaatggacta 8341 tctgtccaaa ctaagaataa aaagaatgac acttgtattt tgtatgtctt tttcactttg 8401 cctttctagt aattcatttt tcttgatatt tacaccttgt ggccctgtga tagactggaa 8461 atctcaaaaa cacacgttca gcaccaagat tttcagcagc accgcctcag aatgagaccc 8521 ctagaaaaaa ctgcgtgttt tccacttgcc caacacgagg agtttttgga acacgacctg 8581 cttgaggtgg agattttcta gatgggcaaa gagaaggaaa cacttaacct aggaagagta 8641 tttaggaaga agaaagaaca cagcctttct gcacaggaaa ccgccgagca gaggggcatc 8701 tggcctctgc agtggcctcc aaatagagtc caatggctgg ggccagcgtg gctgcttaaa 8761 ggggactcaa gggatataat aaaatgcaga ttctcaggtc ctagtgcaga caggctcacc 8821 caataagtct ggactgcata tgggaatctc tatttctagg cccttctgca aggtattcct 8881 gctctttcca ggaaccatcg gcagctggtt tggggaaaga agcaacgact ccaagtgtga 8941 cctgtgagct ggcagcagcc accctcagct ctgctctcgg tcactgaatc cgattctgca 9001 ttttaacagg accccaggtg ttgcacccac acaaagctga agcagattgg tctgggggca 9061 aaaaattaga gctatggaga ttctctcaaa tgaaatagat gatatcattg actgttagag 9121 cttctagaag gaatctgagg tcacttgttc aaattccctg atttacagat gaggaaacag 9181 aggctcagac agctcaaatg acttctctcc aatacccaac attcgacaag tagcagctct 9241 gggactagta cccaaagcac ctagctctcc aatcactgcg caagccacac aattctgtct 9301 gcttgtcagt ggcttttctg attcaaaaaa agcttaggaa tttccccagg aggcagcacg 9361 atgtagtggg aagggctctg gatgtctctc caaggcttct ggaattcatg cccacctcca 9421 ccaagaagcc actttcctgc cagctacagg tgctcacctg aaaagcaagc cagaccatat 9481 taaccctggc attgctggta cctggaagac tttctgattc aatgctttcc acctcctcct 9541 acccctcacc acccccgtgg catgaaatcc tgggggctgc tttagaaatt gttttctttg 9601 gctgctggtg ggggtgctgc tggtgggggt ttgcacagct ggcacactgc accagtctgg 9661 tgggggtttg cacagctggc acactgcacc agtctcctgc ctgctgccaa caaggccatt 9721 tcccaagcac tggctttgga gaagttgggg ctctgaagtg ggaacacaag gctgcctttt 9781 gcaggccagg tgtaaattct ccccctgcca ctttcagcct agcgtgaaac agatggagtg 9841 tgcattccca cttcccttta tggtaccctg gaatgatgga gctgcccagg gcatcgccac 9901 gttactctct agacagtctc tttgtcttcc tgcaatggca gcgccgaggt tgtatatttc 9961 taggtgcagg tatatgattg ccatataata aaaatctgaa aacatccca |
| SEQ ID NO:7 PSG7 mRNA transcript 2046 bp |
| 1 agtgcagaag gaggaaggac agcacagctg acagccgtgc tcaggaagat tctggatcct 61 aggctcatct ccacagagga gaacacgcag ggagcagaga ccatggggcc cctctcagcc 121 cctccctgca cacagcatat aacctggaaa gggctcctgc tcacagcatc acttttaaac 181 ttctggaacc cgcccaccac agcccaagtc acgattgaag cccagccacc aaaagtttcc 241 gaggggaagg atgttcttct acttgtccac aatttgcccc agaatcttac tggctacatc 301 tggtacaaag gacaaatcag ggacctctac cattatgtta catcatatat agtagacggt 361 caaataatta aatatgggcc tgcatacagt ggacgagaaa cagtatattc caatgcatcc 421 ctgctgatcc agaatgtcac ccaggaagac acaggatcct acactttaca catcataaag 481 cgaggtgatg ggactggagg agtaactgga cgtttcacct tcaccttata cctggagact 541 cccaaaccct ccatctccag cagcaatttc aaccccaggg aggccacgga ggctgtgatt 601 ttaacctgtg atcctgagac tccagatgca agctacctgt ggtggatgaa tggtcagagc 661 ctccctatga ctcacagctt gcagctgtct gaaaccaaca ggaccctcta cctatttggt 721 gtcacaaact atactgcagg accctatgaa tgtgaaatac ggaacccagt gagtgccagc 781 cgcagtgacc cagtcaccct gaatctcctc ccgaagctgc ccaagcccta catcaccatc 841 aataacttaa accccaggga gaataaggat gtctcaacct tcacctgtga acctaagagt 901 gagaactaca cctacatttg gtggctaaat ggtcagagcc tcccggtcag tcccagggta 961 aagcgacgca ttgaaaacag gatcctcatt ctacccagtg tcacgagaaa tgaaacagga 1021 ccctatcaat gtgaaatacg ggaccgatat ggtggcatcc gcagtgaccc agtcaccctg 1081 aatgtcctct atggtccaga cctccccaga atttaccctt cattcaccta ttaccattca 1141 ggacaaaacc tctacttgtc ctgctttgcg gactctaacc caccggcaca gtattcttgg 1201 acaattaatg ggaagtttca gctatcagga caaaagcttt ctatccccca gattactaca |
| 1261 aagcatagcg ggctctatgc ttgctctgtt cgtaactcag ccactggcaa ggaaagctcc 1321 aaatccgtga cagtcagagt ctctgactgg acattaccct gaattctact agttcctcca 1381 attccatctt ctcccatgga acctcaaaga gcaagaccca ctctgttcca gaagccctat 1441 aagtcagagt tggacaactc aatgtaaatt tcatgggaaa atccttgtac ctgatgtctg 1501 agccactcag aactcaccaa aatgttcaac accataacaa cagctgctca aactgtaaac 1561 aaggaaaaca agttgatgac ttcacactgt ggacagcttt tcccaagatg tcagaataag 1621 actccccatc atgatgaggc tctcacccct cttagctgtc cttgcttgtg cctgcctctt 1681 tcacttggca ggataatgca gtcattagaa tttcacatgt agtataggag cttctgaggg 1741 taacaacaga gtgtcagata tgtcatctca acctcagact tttacataac atctcaggag 1801 gaaatgtggc tctctccatc ttgcatacag ggctcccaat agaaatgaac acagagatat 1861 tgcctgtgtg tttgcagaga agatggtttc tataaagagt aggaaagctg aaattatagt 1921 agactcccct ttaaatgcac attgtgtgga tggctctcac catttcctaa gagatacatt 1981 gtaaaacgtg acagtaagac tgattctagc agaataaaac atgtactaca tttgctaaaa 2041 aaaaaa |
| **SEQ ID NO:8 PAPPA mRNA transcript 11025 bp** |
| 1 gagcatcttt tgggggaggg gaattcagcg gatcagtctt aagaggagct tttttttgga 61 gcgagaaatc atataaaata aaatgaaata aaacaaggag gaaggcaacc agctgttagg 121 ggaaaaataa ggcagataaa ggagcgggga gagaaattaa ttgccaacca ggaggagttg 181 ggctgtattt ttcaaaggtg gggagagtgg agcacacacc ttgaggagga aagcgagaaa 241 gaaaagaaaa aagcaagtgg aaaggggggc tcgcccaaga agggtgaaga agcgaagaaa 301 gtcgaggcgc cgaggctccc aaagctggca gctccgggtg gcggtgcagg ggcgaagggg 361 gggcgggggg aaccgtcgga catgcggctc tggagttggg tgctgcacct ggggctgctg 421 agcgccgcgc tgggctgcgg gctggccgag cgtccccgcc gggcccggag agacccgcgg 481 gccggccgac ccccgcgccc cgccgccggc ccggccacct gcgccacccg ggcggcccgc 541 ggccgccgcg cctcgccgcc gccgccgccg ccgccgggcg gtgcctggga agccgtgcgc 601 gtcccccggc ggcggcagca gcgggaggcg aggggcgcca ccgaggagcc gagcccgccg 661 agccgggcgc tctatttcag cgggcgaggc gagcagctgc gcctccgggc cgacctcgag 721 ctgccccggg acgcgttcac gctgcaagtg tggctgcgag cggagggggg ccagaggtct 781 ccggcagtga tcacaggcct gtatgacaaa tgttcttata tctcacgtga ccgaggatgg 841 gtcgtgggca ttcacaccat cagtgaccaa gacaacaaag acccacgcta ctttttctcc 901 ttgaagacag accgagcccg gcaagtgacc accatcaatg cccaccgcag ctacctccca 961 ggccagtggg tatacctagc tgccacctat gatgggcagt tcatgaagct ctatgtgaat 1021 ggtgcccagg tggccacctc tggggaacaa gtgggtggca tattcagccc actgacccag 1081 aagtgcaaag tgctcatgtt agggggcagt gccctgaatc acaactaccg gggctacatc 1141 gagcacttca gtctgtggaa ggtggccagg actcagcggg agatactgtc tgacatggaa 1201 acccatggcg cccacactgc tctacctcag ctcctcctcc aggagaactg ggacaatgtg 1261 aagcatgcct ggtcccccat gaaggatggc agcagcccca aagtggaatt cagcaatgcc 1321 cacggctttc tgctggacac gagtctggag cctcctctgt gcggacagac attgtgtgac 1381 aacacagagg tcattgccag ctacaatcag ctctcaagtt tccgccagcc caaggtggtg 1441 cgctaccgcg tggtcaacct ctatgaagat gatcataaga acccgacggt gacgcgcgag 1501 caggtggact tccagcacca tcagctggct gaggccttca agcaatacaa catctcctgg 1561 gagctggacg tgctggaggt gagcaactcc tcccttcgcc gccgcctcat cctggccaac 1621 tgtgacatca gcaagattgg ggatgagaac tgtgaccccg agtgcaacca cacgctgacg 1681 ggccacgacg gcggggattg ccgccacctg cgccaccctg ccttcgtgaa gaagcagcac 1741 aacggggtgt gtgacatgga ctgcaactat gaacggttca actttgatgg tggagagtgc 1801 tgtgaccctg aaatcaccaa tgtcactcag acttgctttg accccgactc tccacacaga 1861 gcctacttgg atgttaatga gctgaagaac attcttaaat tggatggatc aacacatctc 1921 aatattttct ttgcaaaatc ctcagaggag gagttggcag gagtagcaac ttggccatgg 1981 gacaaggagg ccctgatgca cttaggtggc attgtcttga acccatcttt ctatggcatg 2041 cctgggcaca cccacaccat gatccatgag attggtcaca gcctgggcct ctatcacgtc 2101 ttccgaggca tctcagaaat ccagtcctgc agtgacccct gcatggagac agagccctcc 2161 ttcgagactg gagacctctg caatgatacc aacccagccc ctaaacacaa gtcctgtggt 2221 gacccagggc caggaaatga cacctgtggc tttcatagct tcttcaacac tccttacaac 2281 aacttcatga gctatgcaga tgacgactgt acggactcct tcacgcccaa tcaagtcgcc 2341 agaatgcact gttacctgga cctggtctac cagggctggc agccctccag gaaaccagcg 2401 cctgttgccc tcgcccccca agttctgggc cacacaacgg actctgtgac actggagtgg 2461 ttcccaccta tagatggcca tttctttgaa agagaattgg gatcagcatg tcatctttgc 2521 ctggaaggga gaatcctggt gcagtatgct tccaacgctt cctccccaat gccctgcagc 2581 ccatcaggac actggagccc tcgtgaagca gaaggtcatc ctgatgttga acagccctgt 2641 aagtccagtg tccgcacctg gagcccaaat tcagctgtca acccacacac ggttcctcca |
| 2701 gcctgccctg agcctcaagg ctgctacctc gagctggagt tcctctaccc cttggtccct 2761 gagtctctga ccatttgggt gacctttgtc tccactgact gggactctag tggagctgtc 2821 aatgacatca aactgttggc tgtcagtggg aagaacatct ccctgggtcc tcagaatgtc 2881 ttctgtgatg tcccactgac catcagactc tgggacgtgg gcgaggaggt gtatggcatc 2941 caaatctaca cgctggatga gcacctggag atcgatgctg ccatgttgac ctccactgca 3001 gacaccccac tctgtctaca gtgtaagccc ctgaagtata aggtggtccg ggaccctcct 3061 ctccagatgg atgtggcctc catcctacat ctcaatagga aattcgtaga catggatcta 3121 aatcttggca gtgtgtacca gtattgggtc ataactattt caggaactga agagagtgag 3181 ccatcacctg ctgtcacata catccatgga agtgggtact gtggcgatgg cattatacaa 3241 aaagaccaag gtgaacaatg cgacgacatg aataagatca atggtgatgg ctgctccctt 3301 ttctgccgac aagaagtctc cttcaattgt attgatgaac ccagccggtg ctatttccat 3361 gatggtgatg gggtatgtga ggagtttgaa caaaaaacca gcattaagga ctgtggtgtc 3421 tacacgcccc agggattcct ggatcagtgg gcatccaatg cttcagtatc tcatcaagac 3481 cagcaatgcc caggctgggt catcatcgga cagccagcag catcccaggt gtgtcgaacc 3541 aaggtgatag atctcagtga aggcatttcc cagcatgcct ggtacccttg caccatcagc 3601 tacccatatt cccagctggc tcagaccact ttttggctcc gggcgtattt ttctcaacca 3661 atggttgccg cagctgtcat tgtccacctg gtgacggatg ggacatatta tggggaccaa 3721 aagcaggaga ccatcagcgt gcagctgctt gataccaaag atcagagcca cgatctaggc 3781 ctccatgtcc tgagctgcag gaacaatccc ctgattatcc ctgtggtcca tgacctcagc 3841 cagcccttct accacagcca ggcggtacgt gtgagcttca gttcgcccct ggtcgccatc 3901 tcgggggtgg ccctccgttc cttcgacaac tttgaccccg tcaccctgag cagctgccag 3961 agaggggaga cctacagccc tgccgagcag agctgcgtgc acttcgcatg tgagaaaact 4021 gactgtccag agctggctgt ggagaatgct tctctcaatt gctccagcag cgaccgctac 4081 cacggtgccc agtgtactgt gagctgccgg acaggctacg tgctccagat acggcgggat 4141 gatgagctga tcaagagcca gacgggaccc agcgtcacag tgacctgtac agagggcaag 4201 tggaataagc aggtggcctg tgagccagtc gactgcagca tcccagatca ccatcaagtc 4261 tatgctgcct ccttctcctg ccctgagggc accacctttg gcagtcaatg ttccttccag 4321 tgccgtcacc ctgcacaatt gaaaggcaac aacagcctcc tgacctgcat ggaggatggg 4381 ctgtggtcct tcccagaggc cctgtgtgag ctcatgtgcc tcgctccacc ccctgtgccc 4441 aatgcagacc tccagaccgc ccggtgccga gagaataagc acaaggtggg ctccttctgc 4501 aaatacaaat gcaagcctgg ataccatgtg cctggatcct ctcggaagtc aaagaaacgg 4561 gccttcaaga ctcagtgtac ccaggatggc agctggcagg agggagcttg tgttcctgtg 4621 acctgtgacc cacctccacc aaaattccat gggctctacc agtgtactaa tggcttccag 4681 ttcaacagtg agtgtaggat caagtgtgaa gacagtgatg cctcccaggg acttgggagc 4741 aatgtcattc attgccggaa agatggcacc tggaacggct ccttccatgt ctgccaggag 4801 atgcaaggcc agtgctcggt tccaaacgag ctcaacagca acctcaaact gcagtgccct 4861 gatggctatg ccatagggtc ggagtgtgcc acctcgtgcc tggaccacaa cagcgagtcc 4921 atcatcctgc caatgaacgt gaccgtgcgt gacatccccc actggctgaa ccccacacgg 4981 gtagagagag ttgtctgcac tgctggtctc aagtggtatc ctcaccctgc tctgattcac 5041 tgtgtcaaag gctgtgagcc cttcatggga gacaattatt gtgatgccat caacaaccga 5101 gccttttgca actatgacgg tggggattgc tgcacctcca cagtgaagac caaaaaggtc 5161 accccattcc ctatgtcctg tgatctacaa ggtgactgtg cttgtcggga cccccaggcc 5221 caagaacaca gccggaaaga cctccgggga tacagccatg gctaaggaag gacaagaagt 5281 tgtcaaagaa ttcccaacgc caggacccac atccctttgg tattgatttc acagtcagct 5341 gctcaacgga atggcctctc cacaccaggg atccttagca cccaaccggt ctgcctttaa 5401 ttttacccag gaaggactca cattggggcg aatgaaccaa gtttcgccat gctggatgat 5461 gaaatggatt cccatcccaa agtctgagat ggattgcata tacagtgtgc agtcccagag 5521 cctcctaaaa ttctagccat ttgtcacaca accacagcaa gaaacgtgtt ctatatctag 5581 agtgtgccca tctgtgttta gtacacatgc atgcatacac acccatacaa acatctgtgt 5641 gagggcagtt ctggagatga gcagagagag accggaataa actcaatctt ttctttccca 5701 agctcctagc caacactatc cttgggagaa agaaatttgc agaaactgct aagaccaagt 5761 gtggagatgt caagctagtt cacactctga ggctcagaat atgtaggaca tgcacaattg 5821 tgcagtcctt tgggattgga agtgaaacag tctgtgatcc cctaccttct agggaactag 5881 gacctaggaa gaggtaaaga ttatcaggta tgcaaagcgc cccaattctt ctgctgccat 5941 gggggatttt accccaactc cagggttcga ggccaatctg agaatggctt aggattgcaa 6001 tgtcaaggta ttatatcagc cccttgcttg aggcttgagg tcataatatc cctctaggac 6061 ttacctgttc ccccagatct tgccttggga ccacatttgc tgctactttt cctgctgctc 6121 tatcctatac attgaataat ccaagatggt agaactaggt taggaaaaat tccacacaac 6181 caaacagtct gccttaaaag tgacccacat ttttccatag ctcctcactt tttagccctt 6241 ctgcaagaga aaaaccctca tgggtccaca tggtgagaag ttaagtttcc tgtaagtggg 6301 cctctcaccc tggaaaggag ttgagggaca tcagatgctg gaaccctcac tgaaagtcca |
| 6361 gaatgtctaa gccagtgtta gattttgtaa acaagtggaa cagtgttaaa tttctatgat 6421 gttggagcca tccagagact actggaattg tcgagacttt tggattatta tccttatcct 6481 tatcctaatc ttcctagccc ttcaggctag agtaggcttc gatcctgaga accttgctgt 6541 tgctctgagg agatataatt ctgggagaaa gaatctttta taagaacagt acagattgtt 6601 ctcaagaggg ccatcagaag gaagccaaag agttcacagc ctcagcacca acaactcaac 6661 atggtcatca tgttttctat atggtttttc cagctagcag tactcccttc catacctgtg 6721 actgggcagt gcttttctct ctcccatgtc tagcctccaa aagttaagtg aaaattagtc 6781 aactgcacgt ggaagccccc accactttgg ggatctcttt atttcttttc agccagggac 6841 ctgtccactc cctttgaatt aatatgggaa gaaattaata caggatgaac tggagagaag 6901 ggttgagtgt ggcatacttt ctgaaacctg gagctgggaa ttgcggagaa gggaaggtct 6961 agactagtta catcacatag ggattactgt aaatcaagtc atctcaagtc tagtgaagac 7021 agccaacaga aacaaaacct agcataggga tagaaaatac catgcacgtg tgcagcccca 7081 cctaattcct gcatccaagg caggtgttgt taatctatca tagcacttaa aaaaaaaaaa 7141 aaaaagagac caaaaataac tttaggaacc accatattat atcactccca atagcactga 7201 cctggtgatc aaaaacactt gagaagacat ctattggcca tctctggcca attacactaa 7261 gaaacatatc aaggtgcttt tggcacaggt gcccacaaat acggatgcag tgctgagata 7321 gtttatgaga cttgtaccat ttcacaaact ctgaaattgg gttccatatt ggcaaggctg 7381 ccacagttgt taagaataat cctctatgtt tcttcctcac aaaaccatat ctcatttata 7441 tccagaccat tacttcacta taattacaag gacaaattat tagcaagaaa taagaatagt 7501 attagaagaa ttgatcctat tttgaacccc tctccagtat cttcacactc ttgtcaactc 7561 tccaggcctc tctcttgccc tgagttatca gcctgtgtgg tgttaactac cttagaaggt 7621 acaagctaag aaatgtaaca gtatcaaccc tcccagttgc ttaattatac ccataggtaa 7681 tacaaaaagc tctgaagacc caaagatgac attactaatg atgtgatttc aggagccaca 7741 gaagaacctt accagcttcc ctcaaatcag tccttatcct ctttctatct tcactcccat 7801 catcatctat tttcacacta tccagctaag caaagattcc tggaggctga cttgtatctt 7861 cagactcaca gagtgaattc agctcttctg aatcaagacc cacccagtct ctttcattca 7921 gacctgttgc taacaaattt atatttgcca aggatattag gcaaaagagg ctacttgatt 7981 ggtggccaac ctcgtgccca catggaaggt atctttaata gggtcttttc aaaccttagt 8041 ggaggagggt cagctcaatt tgggcaatgc atttgttccc agtttcattt tcttcctggg 8101 aattaactcg tcatttcatt ccttcagtca tcttctgtgt aggtgaccgg agcactgaga 8161 ggcagctctg atgcactatt gtgtgtcagc agctcaaagg ccctaaaaca ctgaaggttc 8221 tgcatctgaa gtattagatt gttagcagca aaatatgaaa gatgaggtgg acagtcctct 8281 aagccctatt tagggaagct tttccaagcc acaatcttaa ctacctaccc aaaggatttg 8341 cattaccccc agattctgtg ccaacaacct tttaaggaaa tacagtcctt gggaaatgag 8401 ttttgatggt gaattggggt gttaaggaag ggaaagattg tcatagatgg tagggctttg 8461 aaaatgcagg gtatcagctg ccactcctgg cttcaacaca ttgagtcact gcctagacgg 8521 ttctcttggt cttattccca tcctggccaa tgcttaaata ctatttgttg aaaataattc 8581 tttgagacag atttcagcta cctcccttcc aggttcgatt taacttggtt gtaattgtca 8641 atttgttgtt ataggtctta cctgtgtgaa agaaagaaaa agaaagaaag aaagaaagag 8701 aaaggaaatt ataaggtcaa gttaacagtt ttgaggtttt gtgttttttt ctggaactac 8761 ttcaagtgag aaaataaaaa aaaatggtga caaagctgta cagatagaga taatagaaga 8821 caaagagatt aaaaggaaat aaaaatgcat gattaaaaac taagaataaa aaacctattt 8881 ttatgtttcc taaaggaaat tgtttattct acagcctcag taggtagaca caaacataaa 8941 gatttcccta gaagacatag agtgggattt gataacactg tctgttattt tctgtacatt 9001 gtggtaggtc caggaaatat gacattttcc cccttgatgt gttattgttg ttgttgggtg 9061 gggtgggcat tttgtttatt tgtttggtgg caatcagtgg tagtagggag tgggagggct 9121 tatattggtt tttccagcta ttaaggggac atattgtgtc gttgtgcttt tcacgttata 9181 aaatgtttat atttaccagt acagcactgg gctttataaa gactgcactc agaaccacac 9241 tgcacagtcc agttttttaa aaagctgcta catgacagac aggtaatccc actgagtgag 9301 ttttgagaaa caaatcaaac gaagtaaaca agaaacataa aaaccaaata gcaaatgaat 9361 aaaagcctgt tcttgtaact tattcaactt ttgccaaatt cctaccaatc acttgctttt 9421 taaaagaaat gtataatagc caaaagagaa attatgtccc tgttgtacag aagttagaat 9481 ttttgactcc aggcagcagt ttgctcagtg atcttgaaca agttatccaa ttgcctctac 9541 atttgcatca gtttctctag ctgcaaaatg gggataatac tatataccta cctcacagtg 9601 ggagggcagg agattttgag gccctgaggt tttaggtggg ctgtgagggc caacgcttga 9661 cacaaagtcc atgggttatt attcaagaat gcacaggccc atcggccttt tagaaagaca 9721 agacagggag tgcttgtttg atatttcaag gaataaagcc ggagctcctg aattgtagtc 9781 caccttaaaa gagagacctg tattggagaa tattttattt ttttggcaaa tttgatctta 9841 ccctttacca gttctataat ttggttaaaa gctgattatg tcctacaatg tcaaagtcag 9901 ctaactgtcg tctacttaag acttctggtc atttccaact tatagaggaa gggagtctct 9961 aaaatctctt cttcagaagg cacctcactt ctcagactta aaattccaca tcaagtgttc |
| 10021 cattaaaaga agataaggca ttctgagtgc aaacaaatgg gggcttctta aactacacac 10081 cagcagtcag tgaggaaaac tttgaacaat tattgagttg ctttcttggg tctctataat 10141 caataacctg tctgcagata tctatctata taaagatatt atatataaat ataaatttac 10201 atatatatgc acatgtatat atagttgtac atatatgtgt gtatatatat acttaaatgt 10261 aatatttaca aaataaaact gtgatctcgt ctagagaaaa tgtattcata ttacaaactg 10321 ctcttccata tttatgtacc atattatacc tttttattat tgttataatt attatgggta 10381 tttctaatta atatgatgtt gaaacctgtt tggcaccttc tggaagctac caaaaaaatg 10441 acactccatt gaagtgctta aaagctgttc tcataagaat tctactggcc tattgtaaaa 10501 aagaaaaaaa aaaagaaaaa gaagaaagac acaaagaaaa taatctaaac accaaaaact 10561 aaacacaatt ccaatccttt ttctgtacct cacgcgcata aatttgctgc tcctattttt 10621 ttttctgttt atgtgttttt atggatctaa gttaaatctt ttggcaatat ataaaaatgt 10681 aaatagtaaa ctttatttat taagaatgtc atctttttta atttatattt acacaattgt 10741 tcatctaatt tattttttct atacagtttt aaatactcag acatattttg ctgttcatga 10801 tatttttatc ctgttctcat ggatttgttt tcccatactg ttttctctga tctcaattac 10861 aggttggatc tcacaaataa taatgtcaga gacagaaata ttttgccact gttgattact 10921 atactttaaa gttctatatt atgaaaatat ataatagctt gtacgcttca aaaaaaaaaa 10981 aaaaaaaaaa aaaaaaaaaa aaaaaaaaaa aaaaaaaaaa aaaaa |
| SEQ ID NO:9 LGALS14 mRNA transcript 794 bp |
| 1 gctgcattac agacacagac ctgcaaacat ctatggttgt gacagagttt ctttctgaca 61 cctgagtctt tctcctgctg cacggaaagc ttgctgggag gggcttggaa tctggcatga 121 agccaaaggg catctctgag ttgcagcatt taaatgatcc cactcagaga ttcacacaga 181 agactggaca caattccgaa gagctgccca gaaggagaga acaatgtcat cactacccgt 241 accatacaca ctgcctgttt ccttgcctgt tggttcgtgc gtgataatca cagggacacc 301 gatcctcact tttgtcaagg acccacagct ggaggtgaat ttctacactg ggatggatga 361 ggactcagat attgctttcc aattccgact gcactttggt catcctgcaa tcatgaacag 421 ttgtgtgttt ggcatatgga gatatgagga gaaatgctac tatttaccct ttgaagatgg 481 caaaccattt gagctgtgca tctatgtgcg tcacaaggaa tacaaggtaa tggtaaatgg 541 ccaacgcatt tacaactttg cccatcgatt cccgccagca tctgtgaaga tgctgcaagt 601 cttcagagat atctccctga ccagagtgct tatcagcgat tgagggagat gatcagactc 661 ctcattgttg aggaatccct ctttctacct gaccatggga ttcccagagc ctactaacag 721 aataatccct cctcacccct tcccctacac ttgatcatta aaacagcacc aaacttcaaa 781 aaaaaaaaaa aaaa |
| SEQ ID NO:10 CLCN3 mRNA transcript 6299 bp |
| 1 gtgacgtcac gcgtcgacgc tggggcgtac ctttcgggct cctgactcct gccgcttctc 61 ttccccttcc gtgggtcagg gccggtccgg tccggaacct gcagcccctt tcccagtgtt 121 ctagttcgcc cgtgacccgg aataatgagc aaggagggtg tggtgggttg aaagccatcc 181 tactttactc ccgagttaga gcatggattc agttttagtc ttaaggggga agtgagattg 241 gagattttta tttttaattt tgggcagaag caggttgact ctagggatct ccagagcgag 301 aggatttaac ttcatgttgc tcccgtgttt gaaggaggac aataaaagtc ccaccgggca 361 aaattttcgt aacctctgcg gtagaaaacg tcaggtatct tttaaatcgc gatagttttc 421 gctgtgtcag gctttcttcg gtggagctcc gagggtagct aggttctagg tttgaaacag 481 atgcagaatc caaaggcagc gcaaaaaaca gccaccgatt ttgctatgtc tctgagctgc 541 gagataatca gacagctaaa tggagtctga gcagctgttc catagaggct actatagaaa 601 cagctacaac agtataacaa gtgcaagtag tgatgaggaa cttttagatg gagcaggtgt 661 tattatggac tttcaaacat ctgaagatga caatttatta gatggtgaca ctgcagttgg 721 aactcattat acaatgacaa atggaggcag cattaacagt tctacacatt tactggatct 781 tttggatgaa ccaattccag gtgttggtac atatgatgat ttccatacta ttgattgggt 841 gcgagaaaaa tgtaaagaca gagaaaggca tagacggatc aacagcaaaa agaaagaatc 901 agcatgggaa atgacaaaaa gtttgtatga tgcgtggtca ggatggctag tagtaacact 961 aacaggattg gcatcagggg cactggccgg attaatagac attgctgccg attggatgac 1021 tgacctaaag gagggcattt gccttagtgc gttgtggtac aaccacgaac agtgctgttg 1081 gggatctaat gaaacaacat ttgaagagag ggataaatgt ccacagtgga aaacatgggc 1141 agaattaatc ataggtcaag cagagggtcc tggttcttat atcatgaact acataatgta 1201 catcttctgg gccttgagtt ttgcctttct tgcagtttcc ctggtaaagg tatttgctcc 1261 atatgcctgt ggctctggaa ttccagagat taaaactatt ttaagtggat tcatcatcag 1321 aggttacttg ggaaaatgga ctttaatgat taaaaccatc acattagtcc tggctgtggc 1381 atcaggtttg agtttaggaa aagaaggtcc cctggtacat gttgcctgtt gctgcggaaa 1441 tatcttttcc tacctctttc caaagtatag cacaaacgaa gctaaaaaaa gggaggtgct |
| 1501 atcagctgcc tcagctgcag ggggtttctgt agcttttggt gcaccaattg gaggagttct |
| 5161 cgtgagcctc ccaaagtggt ggaattacag gcgtgggcca ctgcgcctgg cccagacaga 5221 cattttctga aacacaactg gcaatgagct gtttttacat tttgaaagtg attcttcact 5281 tcctagttct taattatagt atacctatta agatctgtaa gatcctgaag acataagatc 5341 atgaagccat ataagaatga ggattgaaag ttgagcaaaa ttttcgggat tttgggaaac 5401 attcttagct gtgctatctg cctaaaatta ttccttatta cttctctcct ttgacagact 5461 tcaagttttc ttcatagccc tttcaaagtt ttttgagcca tccagagtaa aatcatttct 5521 aaatgatagt tctgtatatc tccaactcgt cttaagtgta tttgcctgtg tgcaacgtat 5581 tgctagacta tgaactcctc agcatggctg ctggataact taattgtcct gagttaatag 5641 ccttcaaagg acaaatcggt ttctttgcag atagcttcgt aaaacttcac atggagttta 5701 ttttatcata tttccctttt ttatttctgc tcctccttta attgcccatc ttgcttcaga 5761 gactgacatt tcagggtgga tattaattaa agcattaatt ttgttttttg gtatatttct 5821 atccctagta tttctatctt actgctaaaa tacaggaaaa gtgccgtatt tttaatgcat 5881 ttagtggttt tctttggtgt tatctgttcc atttttcttt ttcatacatt gaagtgtgtc 5941 tccttttcaa ccaaaataat gaaatagtgg agaccatgaa attgttgtgc ctggctaatt 6001 ggcaaattaa tttaccaata taataagtgt agcgccttgt ttgaataccc tttttgagaa 6061 ggtatgatga gaatgggcaa gggtgtcagc atctcttctt cttaataatt aattgttttc 6121 agttttggtt cacgaagaat gcttagttaa tctgtaatgt tgcctagagc tgtatttatc 6181 tgtttttatt tatactagtg tagtaaagct gcatatcatt acagtaaaaa cgactactgt 6241 gatgagttaa tcagaaaatc tattaaaatc tatatgacaa tgaaaaaaaa aaaaaaaaa |
| **SEQ ID NO:11 DAPP1 mRNA transcript 3006 bp** |
| 1 gcaggctgct gtctcacaga gcgagaaggt gtcaggagca gcccagttgt gtctctctct 61 ctacctctgt gaagggcgcg aatgggcaga gcagaacttc tagaagggaa gatgagcacc 121 caggatccct cagatctgtg gagcagatcc gatggagagg ctgagctgct ccaggacttg 181 gggtggtatc acggcaacct cacacgccat gctgctgaag ctcttctcct ctcaaatgga 241 tgtgacggca gctaccttct gagggacagc aatgagacca ccgggctgta ctctctctct 301 gtgagggcca aagattctgt taaacacttt catgttgaat atactggata ttcatttaaa 361 tttggcttta atgaattctc atctttgaag gattttgtca agcattttgc aaatcagcct 421 ttgattggaa gcgagacagg cactctgatg gttctaaaac atccctaccc aagaaaagtg 481 gaagaaccct ccatttatga atctgtccgg gttcacacag caatgcagac aggaagaaca 541 gaagatgacc ttgtgcccac agcaccttct ctgggcacca aagaaggtta cctcaccaaa 601 cagggaggcc tggtcaagac ctggaaaaca agatggttta ctctgcacag gaatgaactg 661 aaatacttca aagaccagat gtcaccagaa ccaattcgga tcctagacct aacagaatgt 721 tcagctgtac aattcgatta ttcacaagaa agggtaaact gtttttgttt ggtatttcca 781 ttcaggacat tttatctctg tgcaaagacc ggagtagaag ctgatgagtg gatcaagata 841 ttacgctgga aattggtcaa ggacaaaagc tgatttattt tgtctgctct ctgtatatct 901 cccgaggaga agactgatca caaataagaa aacagctcaa ccaaggggaa ggcacgatcc 961 gatctcggtc gttcatcttt aaatagatct ttcttgccaa ggaatgctct ggcccaggag 1021 caaggtggaa tgtttccctg acgctgtgat ctgcagcagg cttcaaatga aaaccgacta 1081 aggattttct ttcaaaaaca aatcagaagc agatgctgat tgggacccat ataccacgtt 1141 gctgactcac gttgctgccc ttccatgatg ttgccatctc cttgagaaca ctgaagcaat 1201 caccattctg atagaaagtg cttaaaccac cactcttagg tctgctcact cttagaacac 1261 acaatggaag aggaagggtt tttgttttca ctcattgtgg tccccaagcc tattgacact 1321 agttgcctag agtcccactg tgagtcatgg tcagcctgtc tgacatccag gttgtgctat 1381 taaccaagaa ggaaacagat acttggaggc ttagatgact tctgcaggat ttatattcag 1441 atagaaaaca tcaaatattt tcaggggaga ggtttttttt tttaattttt ccccctttat 1501 acaaaaaaaa aagaacattt ccaaaactaa aatagaaaat gcttgtggca tttattttct 1561 ctttttaaaa ggttcagaaa tttggcaggt cctttgcttc taatgacaaa actgtgagag 1621 ctagatgtcc tatgggcaat taggtagtat aataaaggta aatgaaggta caatttttaa 1681 accattattt tcaccctgtt ggggtaaatg ttttaaagag tgagaaaaca taaattgaga 1741 aagggtgata aagtaataga taacttttag tttaataata attattgtta ttatactact 1801 aataatagag cacttgtaag cactaagtta tctttatcca acatttctcc aaatggactg 1861 aaagaaactt ttcaaggaca gtgtattata acaatccctt tcccagaatt agttgtatag 1921 ggttggccca agagatgtaa gaaaaatctc gcattgctcc ctaagcaccc tgggccttat 1981 taaagagcaa cttctatttc cagtcggggg agtaacacta aagctacaag aaatatgtaa 2041 taatgatagg taataatgtg ttccaaagct ttttcaaact agaataagga ggcaaataga 2101 agaatgagat actgatgtcc acagttcatt ggcagaatct aaccccttct gttatctttt 2161 ttaatactat ttttgtttag atagaagttt caaagaagat aaaaatgctt gaagagcctg 2221 agagtaaaaa gattatgctg caaagctatg atataaactg ctcttgcagt ccaaagggat 2281 acctgattaa agaagtttct tatttaaaca tctcagacgc aaaaattaca ttaaattttt 2341 gtatatttca acaacatttt aaatgtattt tgttatgttt gtattatata ggataaagca |
| 2401 aatgtcaagt taaaatgtat tgtgttgttt gtaaagtaag aagttactgg ccaggagcgg 2461 cggctcatgc ctgtaatccc aggactttgg taggccaaga caagcagatc acttgaggtc 2521 aggagttcaa catcagcctg gccaacatga tgaaaccttg tctttactaa aaatacaaaa 2581 attagctggg catggtggca ggcgcctgta atcccagcta ctcaggaggc tgaggcagga 2641 gaattgcttg aacccgggag gtggaggttg cagtgaacca agatcgcggc gctgcactct 2701 agcctgggtg acagagtcag actccgtccc aaaaaaacaa acaaacaaaa caaaacaaaa 2761 aaaaacagaa gttacaaatg aatactcacg gatatgtata gttttatgtt tgttttctta 2821 gaaacaaatg tgtttctttg ggtgggtaat attgtgtttt actatgttta ccttttataa 2881 aacataacct gtttatttat attctttggc tttgtttatt aaaaagcatg attttgctgt 2941 gcatgtacca ttttgctatt aaaatttatt tttaatattt gtaacttgaa aaaaaaaaaa 3001 aaaaaa |
| **SEQ ID NO:12 POLE2 mRNA transcript 1861 bp** |
| 1 agcctactcg gtccggggtt gcgaactgta aggtctgagt tgctgcggcg caggcagcgg 61 agaccaagca gggatcttaa cagggtttag cgccacgcgg gccagggccg aggccggagc 121 tgggaggggc gcgcccggga aggggcggag ctgcggcggt ggcgccaaat cgcaaatatg 181 gcgccggagc ggctgcggag ccgggcgctc tccgccttca agttgcgggg cttgctgctc 241 cgtggtgaag ctattaagta cctcacagaa gctcttcagt ctatcagtga attagagctt 301 gaagataaac tggaaaagat aattaatgca gttgagaagc aacccttgtc atcaaacatg 361 attgaacgat ctgtggtgga agcagcagtc caggaatgca gtcagtctgt tgatgaaact 421 atagagcacg ttttcaatat cataggagca tttgatattc cacgctttgt gtacaattca 481 gaaagaaaaa aatttcttcc tctgttaatg accaaccacc ctgcaccaaa tttatttgga 541 acaccaagag ataaagcaga gatgtttcgt gagcgatata ccattttgca ccagaggacc 601 cacaggcatg aattatttac tcctccggtg ataggttctc accctgatga aagcggaagc 661 aaattccagc ttaaaacaat agaaacctta ttgggtagta caaccaaaat cggagatgcg 721 attgttcttg gaatgataac gcagttaaaa gagggaaaat tttttctgga agatcctact 781 ggaacagtcc aactagacct tagtaaagct cagttccata gtggtttata cacagaggca 841 tgctttgtct tagcagaagg ttggtttgaa gatcaagtgt ttcatgtcaa tgcctttgga 901 tttccaccca ctgagccctc tagtactact agggcatact atggaaatat taattttttt 961 ggaggtcctt ctaatacatc tgtgaagact tctgcaaaac taaaacagct agaagaggag 1021 aataaagatg ctatgtttgt gtttttatct gatgtttggt tggaccaggt ggaagtattg 1081 gaaaaacttc gcataatgtt tgctggttat tcaccagcac ctccaacctg ctttattctg 1141 tgtggtaatt tttcatctgc accatatgga aaaaatcaag ttcaagcttt gaaagattcc 1201 ctaaaaactt tggcagatat aatatgtgaa tacccagata ttcaccaaag tagtcgtttt 1261 gtgtttgtac ctggtccaga ggatcctgga tttggttcca tcttaccaag gccaccactt 1321 gctgaaagca tcactaatga attcagacaa agggtaccat tttcagtttt tactactaat 1381 ccttgcagaa ttcagtactg tacacaggaa attactgtct tccgtgaaga cttagtaaat 1441 aaaatgtgca gaaactgcgt ccgttttcct agcagcaatt tggctattcc taatcacttt 1501 gtaaagacta tcttatccca aggacatctg actcccctac ctctttatgt ctgcccagtg 1561 tattgggcat atgactatgc tttgagagtg tatcctgtgc ccgatctact tgtcattgca 1621 gacaaatatg atcctttcac tacgacaaat accgaatgcc tctgcataaa ccctggctct 1681 tttccaagaa gtggattttc attcaaagtt ttttatcctt ctaataagac agtagaagat 1741 agcaaacttc aaggcttttg agattcttaa agatcatctg aagaaaattc atcagttttc 1801 tgcttaactc tatatcttat gtgattctga tattacaata aaattatggt aaactttagg 1861 a |
| **SEQ ID NO:13 PPBP mRNA transcript 1307 bp** |
| 1 acttatctgc agacttgtag gcagcaactc accctcactc agaggtcttc tggttctgga 61 aacaactcta gctcagcctt ctccaccatg agcctcagac ttgataccac cccttcctgt 121 aacagtgcga gaccacttca tgccttgcag gtgctgctgc ttctgtcatt gctgctgact 181 gctctggctt cctccaccaa aggacaaact aagagaaact tggcgaaagg caaagaggaa 241 agtctagaca gtgacttgta tgctgaactc cgctgcatgt gtataaagac aacctctgga 301 attcatccca aaaacatcca aagtttggaa gtgatcggga aaggaaccca ttgcaaccaa 361 gtcgaagtga tagccacact gaaggatggg aggaaaatct gcctggaccc agatgctccc 421 agaatcaaga aaattgtaca gaaaaaattg gcaggtgatg aatctgctga ttaatttgtt 481 ctgtttctgc caaacttctt taactcccag gaagggtaga attttgaaac cttgattttc 541 tagagttctc atttattcag gatacctatt cttactgtat taaaatttgg atatgtgttt 601 cattctgtct caaaaatcac attttattct gagaaggttg gttaaaagat ggcagaaaga 661 agatgaaaat aaataagcct ggtttcaacc ctctaattct tgcctaaaca ttggactgta 721 ctttgcattt ttttctttaa aaatttctat tctaacacaa cttggttgat ttttcctggt |
| 781 ctactttatg gttattagac atactcatgg gtattattag atttcataat ggtcaatgat 841 aataggaatt acatggagcc caacagagaa tatttgctca atacattttt gttaatatat 901 ttaggaactt aatggagtct ctcagtgtct tagtcctagg atgtcttatt taaaatactc 961 cctgaaagtt tattctgatg tttattttag ccatcaaaca ctaaaataat aaattggtga 1021 atatgaatct tataaactgt ggttagctgg tttaaagtga atatatttgc cactagtaga 1081 acaaaaatag atgatgaaaa tgaattaaca tatctacata gttataattc tatcattaga 1141 atgagcctta taaataagta caatatagga cttcaacctt actagactcc taattctaaa 1201 ttctactttt ttcatcaaca gaactttcat tcatttttta aaccctaaaa cttataccca 1261 cactattctt acaaaaatat tcacatgaaa taaaaatttg ctattga |
| **SEQ ID NO:14 LYPLAL1 mRNA transcript 1922 bp** |
| 1 gtgcgcggcc ccgcgcggca acgcaggggc ggaaccgcat gactggcagt ggcatcagcg 61 atggcggctg cgtcggggtc ggttctgcag cgctgtatcg tgtcgccggc agggaggcat 121 agcgcctctc tgatcttcct gcatggctca ggtgattctg gacaaggatt aagaatgtgg 181 atcaagcagg ttttaaatca agatttaaca ttccaacaca taaaaattat ttatccaaca 241 gctcctccca gatcatatac tcctatgaaa ggaggaatct ccaatgtatg gtttgacaga 301 tttaaaataa ccaatgactg cccagaacac cttgaatcaa ttgatgtcat gtgtcaagtg 361 cttactgatt tgattgatga agaagtaaaa agtggcatca agaagaacag gatattaata 421 ggaggattct ctatgggagg atgcatggca atacatttag catatagaaa tcatcaagat 481 gtggcaggag tatttgctct ttctagtttt ctgaataaag catctgctgt ttaccaggct 541 cttcagaaga gtaatggtgt acttcctgaa ttatttcagt gtcatggtac tgcagatgag 601 ttagttcttc attcttgggc agaagagaca aactcaatgt taaaatctct aggagtgacc 661 acgaagtttc atagttttcc aaatgtttac catgagctaa gcaaaactga gttagacata 721 ttgaagttat ggattcttac aaagctgcca ggagaaatgg aaaaacaaaa atgaatgaat 781 caagagtgat ttgttaatgt aagtgtaatg tctttgtgaa aagtgatttt tactgccaaa 841 ttataatgat aattaaaata ttaagaaata acactttcct gactttttta ttattaaaat 901 gcttatcact gtagacagta gctaatctta ttaatgaaaa acaatagaca aacatctgtg 961 cataattttt cagacacaat tctgtaaata tttggaaacc ttttaagtat ttaaactttt 1021 aaatttttga aataaagtat tctaaactaa tataaataag gacaatgaaa aaacatgaaa 1081 ggacttagca taatgttatt ttatcttttc tacaactttg tttaaattac ctttccaaag 1141 atatttgtgt ttatgtaatt ttccacggaa taacattaat actctaggtt tataaaccgg 1201 tttcacatta tttcatttga tcatcacaag agctttgtga agtaagccga gaagttgtta 1261 ctggtattta ataatagcaa tagaggagtt aaagactttc ccacagcttg caggtcaaga 1321 caagaaattc aggtctccta attctcagtg gagctctatt tctgttaacc caaattgctg 1381 ctctgtttta ggtctcaatt tcatctgtaa aatgatacta atagtactta tcccattgga 1441 tttttgttga gatttaaata aatagccaaa agccaataca taataaacac tcaataaaga 1501 ttaaccataa ggagagtcat gatctggttc caggaataca ttgttagatg actgaaaaat 1561 tgtattactt caatgaaaat actataaata ataacatttt catatattag ttggttctca 1621 tgcatacata atctaatttt atttgatcct cacaactgtt taagttttat taaatataca 1681 ttatccctat ttgtataaat agaatcatac aatacctgcc tgctttcatt caacaaaatt 1741 atcatgagat ttttccatgt tgtgtacatc aatagttcat ctattttatt gctcagtaat 1801 attccattgt gtggatgtat cactatttgt ttacacattc accactgata tataagttgc 1861 ttccagtgtg aggctgtttt aaataaagct gctatgaata ttcatgtaag aaaaaaaaaa 1921 aa |
| **SEQ ID NO:15 MAP3K7CL mRNA transcript 2269 bp** |
| 1 cgcagccccg gttcctgccc gcacctctcc ctccacacct ccccgcaagc tgagggagcc 61 ggctccggcc tcggccagcc caggaaggcg ctcccacagc gcagtggtgg gctgaagggc 121 tcctcaagtg ccgccaaagt gggagcccag gcagaggagg cgccgagagc gagggagggc 181 tgtgaggact gccagcacgc tgtcacctct caatagcagc ccaaacagat taagacatgg 241 gaggtgaaag acaacttgag tggttaaatt actgtcatgc aaagcgacta gatggttcag 301 ctgattgcac ctttagaagt tatgtggaac gaggcagcag atcttaagcc ccttgctctg 361 tcacgcaggc tggaatgcag tggtggaatc atggctcact acagccctga cctcctgggc 421 ccagagatgg agtctcgcta ttttgcccag gttggtcttg aacacctggc ttcaagcagt 481 cctcctgctt ttggcttctt gaagtgcttg gattacagta tttcagtttt atgctctgca 541 acaagtttgg ccatgttgga ggacaatcca aaggtcagca agttggctac tggcgattgg 601 atgctcactc tgaagccaaa gtctattact gtgcccgtgg aaatccccag ctcccctctg 661 gattgtcagt ggctgctatg cagcaggtgc agcctggtct ctcactgagt ctctactcca 721 caaaggcaac gactggccaa ggcagtggct ggctctgggt tacacaagtg cagacactca 781 actaagtgag ctggaagacc caggagaagg cggaggctca ggtgcccaca tgatcagcac |
| 841 agccagggta cctgctgaca agcctgtacg catcgccttt agcctcaatg acgcctcaga 901 tgatacaccc cctgaagact ccattccttt ggtctttcca gaattagacc agcagctaca 961 gcccctgccg ccttgtcatg actccgagga atccatggag gtgttcaaac agcactgcca 1021 aatagcagaa gaataccatg aggtcaaaaa ggaaatcacc ctgcttgagc aaaggaagaa 1081 ggagctcatt gccaagttag atcaggcaga aaaggagaag gtggatgctg ctgagctggt 1141 tcgggaattc gaggctctga cggaggagaa tcggacgttg aggttggccc agtctcaatg 1201 tgtggaacaa ctggagaaac ttcgaataca gtatcagaag aggcagggct cgtcctaact 1261 ttaaattttt cagtgtgagc atacgaggct gatgactgcc ctgtgctggc caaaagattt 1321 ttattttaaa tgaatagtga gtcagatcta ttgcttctct gtattaccca catgacaact 1381 gtctataatg agtttactgc ttgccagctt ctagcttgag agaagggata ttttaaatga 1441 gatcattaac gtgaaactat tactagtata tgtttttgga gatcagaatt cttttccaaa 1501 gatatatgtt tttttctttt ttaggaagat atgatcatgc tgtacaacag ggtagaaaat 1561 gataaaaata gactattgac tgacccagct aagaatcgtg ggctgagcag agttaaacca 1621 tgggacaaac ccataacatg ttcaccatag tttcacgtat gtgtattttt aaatttcatg 1681 cctttaatat ttcaaatatg ctcaaattta aactgtcaga aacttctgtg catgtattta 1741 tatttgccag agtataaact tttatactct gatttttatc cttcaatgat tgattatact 1801 aagaataaat ggtcacatat cctaaaagct tcttcatgaa attattagca gaaaccatgt 1861 ttgtaaccaa agcacatttg ccaatgctaa ctggctgttg taataataaa cagataaggc 1921 tgcatttgct tcatgccatg tgacctcaca gtaaacatct ctgcctttgc ctgtgtgtgt 1981 tctgggggag gggggacatg gaaaaatatt gtttggacat tacttgggtg agtgcccatg 2041 aaaacatcag tgaacttgta actattgttt tgttttggat ttaaggagat gttttagatc 2101 agtaacagct aataggaata tgcgagtaaa ttcagaattg aaacaatttc tccttgttct 2161 acctatcacc acattttctc aaattgaact ctttgttata tgtccatttc tattcatgta 2221 acttcttttt cattaaacat ggatcaaaac tgacaaaaaa aaaaaaaaa |
| **SEQ ID NO:16 MOB1B mRNA transcript 7091 bp** |
| 1 gctacccact tccgccccct ccccctgcca ttggaactag ctgagccgaa ctagttgcgg 61 ccaccgagca gccggctctc ggcacctcct cctccgcctc cctgtctcct gttccattcg 121 cctttcctct tctttcctgg cccacgccgc tccgaggcct cgcgaccgcc gagcctgcag 181 cctgccccgc ggccaacatg agcttcttgt tgagttctca gcctgaagtt gactggaact 241 ttcagttaac aagtatttat cgaatacctg atctgtagtg ttggacttag acctatggaa 301 ggagctactg atgtgaatga aagtggtagt cgctcttcta aaacttttaa accaaagaag 361 aacattccag agggttctca ccagtatgag ctcttaaaac acgcagaagc cacacttggc 421 agtggcaacc ttcggatggc tgtcatgctt cctgaagggg aagatctcaa tgaatgggtt 481 gcagttaaca ctgtggattt cttcaatcag atcaacatgc tttatggaac tatcacagac 541 ttctgtacag aagagagttg tccagtgatg tcagctggcc caaaatatga gtatcattgg 601 gcagatggaa cgaacataaa gaaacctatt aagtgctctg caccaaagta tattgattac 661 ttgatgactt gggttcagga ccagttggat gatgagacgt tatttccatc aaaaattggt 721 gtcccgttcc caaagaattt catgtctgtg gcaaaaacta tactcaaacg cctctttagg 781 gtttatgctc acatttatca tcagcatttt gaccctgtga tccagcttca ggaggaagca 841 catctaaata catctttcaa gcactttatt ttttttgtcc aggaattcaa ccttattgat 901 agaagagaac ttgcaccact ccaagaactg attgaaaaac tcacctcaaa agacagataa 961 aaggatgcag agctgtgcaa attgttcctc aaatgaagca gtgtggagtg tattggggat 1021 tttgttatat tttgttttta tctggattgt tttgtcctta ggtttggggg cgggggcttg 1081 tttgggttcc tttttcttta ttctgattat gtgaaaccat attctattgc taggggaagc 1141 caagaaccat tctctataca cttgataagg gtaaatttat cttagtgttt ttaaacttgg 1201 ttttggttac ttgaggagtt ttttaataat attgtgtgct gcaagaaagt gcttgttgat 1261 tgaactgccg atggattggt ttctgtgtgg tataaattgt ggcccattta tgaagtcccc 1321 aaaagagtta tgtttttaag tgccttggca ggctcacttc tgaggtgcaa aacatagata 1381 tagaactgaa caggcttgaa aacaatatta ggattactac ccagggcact tactggtgca 1441 tgttgtaaca tatctatgat aaaagccata gtttacctaa aatggtgatt tccagccttt 1501 actgctttga agaaacagaa tttgtaaagg tatgcatgta gaacataaaa aatatttctt 1561 aattattttt tatattgatg gtaatatatt acgttcaaca atgcttaaag ctctacaagc 1621 aggtcttttc ccacctcttg atatctgtga tactgaaact tgaggatgtt gaaatgtatt 1681 acattttggc ttctttctac atgttaactg cactgtagat gtaaaaattc aggttatata 1741 taggattgcc atcttcagag gtgatgctga actgtgaggt ttcctagtaa ttgccaaatg 1801 agccgtaagt ctgcagaatt cccttctact ttgaagagaa ggggatagga atgtatattt 1861 ggctgggggc atggagatgt tcgtatgtat gaggagttag ggatggggag tcaagttcta 1921 gaaagttttg tctgaaaacc tttgaataga atggcatgaa gattttaatc aattacttat 1981 aaacaaagtc ttagagactt ccttttagga atcaacttcc atgagaagtt aaaaataaat 2041 tattaatttt aggtacagac attaaacatg gaatttaagg actgttgggg gaaattgatc |
| 2101 acttcttagc atttccattc agtgaatgga gctgatgttt gcctgtcatt ttaagatgat 2161 accatacctt ctttggttat tataggtcca gtttgaagca ttctgacttc tggtttttcc 2221 accctgaaag gaaatgtttt tctttgcagc agtattagat aatgaaaaat gctaattcag 2281 tagttattaa cttctaaatt ttattcgcca tgactttcta gtgaattatt accataaata 2341 acaatttcag aaacttagtt tttagaataa atattaattt ttccacttca gttttattct 2401 agaaaatacc ctttttagaa atccagtttt agttttgtca ttttcgataa atctttcttc 2461 agttagaaat atatatcctt ccttcagttg aaacatatac ctttttcaca tctaggaaga 2521 aatgcttgct ctgaaatagt atagattaaa aacactcagt agaaaagaat ctaaaattaa 2581 atgaatttgt tttgccatta aagtagagca gtgatacaat ttaatgccat tacaattatg 2641 ttgactagaa actgcctttt tctccacttc atttctagca attatttacc aagtaccaac 2701 agtagaagta acaggaaagc ctggcagagt taaatatctt ggacatttat tggtaaagct 2761 tatttataaa ctgcagtcag agctagttaa tttccttaaa tctttttgta ttcagataga 2821 taatatgaat cattatgggt tgattcagaa ataaaatttg tgaggtgatt ttgaatcttg 2881 tccatatagg aaaatgaagc acagaattac tcagtcttcc atattgtatt tgacttcata 2941 tcaatctagt aaaaaaggag ttgcaatagc caagtataga gagaatagtg aaaaattaat 3001 cttgcccttt caagccttat acagtagtac actgtacttg tttttagtag taagacctac 3061 tttcccacta tatgtagata gtttgttttc actgtgccag aatctcaggt gcctgcttag 3121 agtatttctt taatcacagt cactgggaag taaggagatg tatatatgtg tatatatggt 3181 aacaaagcat agcagttctc taggggagag gcctggcatt gcacatggtg ttacatggct 3241 acaagtaagg aaaaaatcag aaagtgaaag aactgatgta ataaaaggtt gatttggttg 3301 gttcccatga aagttagtaa gatgcccttt taaatataag gatcagtgct ttgttctgca 3361 gcagagtttg ctgataaatg tctgttggat tctttttgga tttctttaat taatttgtaa 3421 gtaaccaaga taattatttt cccccttgct ctctatatta atacgtagct ataaagcaac 3481 agttggtttt cttatccttt gataaaagca tcccataaaa tataaagtag taagttaaca 3541 tagtattatt gtcacacaca atgctttttt tggttaaatg ttgatacgaa gcaatgtttt 3601 ggaattactt taattgatgg agtagtggtg gtagagagaa attaataaca aaaagagtga 3661 aaatatttta attagcagta gatggtgcta ctggctttca tttgctgact tgattattcc 3721 ctttctctta aaaaccatgg cattagactg cactaaatta acaagcatgt tagttgctgg 3781 tagaggtttt ggaggttaat ttaccttaaa ttggaagact tttaattgca gtctctttct 3841 accttccctc tgttagtcat ttgtaaattc taaatggtca ccataaaatg tattaggtag 3901 gagaagatac gttttatgta taatatatct cagactgagt tactgcctgt cttatcagga 3961 tggataaaac actacagtct cttatcagga aatagagatg atgtggatat ttatatatta 4021 catatataac caccagactc cattttatat attagcattt tccttgctta tgggaaaata 4081 gcaaaacaac atttcattta tacttttgtt tacccctctc tgagacaggt tttgataacc 4141 actgaaatgg tagaatatgt gagatacaaa tattgagttg tagaactttc tttttaaggt 4201 gaataagtca tgccttaaca tccaaataag agttcatctt cagagtggtt cttttgggag 4261 cactgtttat tccagctata ctgcaaaagt ataatgtttt tggaactgtt ctagagcata 4321 ccatgaaaag cagtttgtta ttatgcagga aaatcagttt catcatttta gttacactaa 4381 acacttttgg cagcttaata tgaccttttt aaattttttt tatttttttt atttttattt 4441 ctttaagatg gagtcttgct ctgttgcccg ggctggagta caatggcatg atctcagctc 4501 actgcaacct ccacctcctg ggttcaagca tttctcctgc ctcagcctcc caagtagctg 4561 ggattacagg cagcaccaca cctggctaat tttcatattt ttagtagaga tggggtttca 4621 acatattggc caggctggtc tcaaactcct gacctcaagt gatccgccct ccccagcctc 4681 ccaaagtgct gggattacag gtgtgagcca ccacagccag ccagtatgac ctatcttaat 4741 catcagctca actgtaattt aaatttggct gttctctgga gctaaaccat tagggaagtt 4801 caaaggaatg tgccatgatt tccgaatttg cacaagagaa tgttttaagc attggtagca 4861 taattgaata aaagaatagt ttcctgatgt cactattttg aagtggaaat tatcacttgg 4921 atgtggaggt tttacttttt aaaaacattc agcttaatta ccttacctta attaccttag 4981 ttagatatac taatggaaaa aaccaagtcc ctttctctag aacttgtttt ctatttttgt 5041 tccttttcat gaaaacttct caatttaatt ttaactactg taggatagta ttgattgaat 5101 ggatactatg gaaaagtgga tccaatattt aagatagaag tagtttaagg agacaacagc 5161 ctttactgcc attttttttt aaatgttttc actcagatga acaatttgac tttaataaaa 5221 gactggagat ttttgtacaa agaaatagga ataagtttca tatactaatt atgctgagtt 5281 ttaagcctac atatcacaaa atatttagaa ttgtataacc ttttcatata tttataactt 5341 ttaatgtctt tttaaaagat gtgggaccaa aaatatattt ataatttgga aatgtgactg 5401 cataccaata agaaaactta ccttattttg aaatttatct gggatattaa agaatctacc 5461 aattcttaaa aacacagatt tatactttaa gcttatttta aaattaaaga atatatacca 5521 attcttagaa acactttaag gactactctt aaataactta aatatcagag ttttgttgta 5581 atattaaaat ttaccgtgga aatcactgtt gttcagctat caccttaatt gtgtatgata 5641 tgataaatgt ttagcagtaa agctatctta agatttaatg gaaaagttta atttgaagat 5701 gtaacaaaaa ttctgaccac agttgattct gaatttttaa ggctttccta ataggctgat |
| 5761 cacagagaat aatccatttt gaaggtataa aactgcactg tatgtctgtc acttgtagct 5821 gaactgattc acattttgac aaaagagaga aaatacaaaa atgagttttg caaatgtaat 5881 aactttttct gcatatagaa ctaaataatt gaaaaatatg ggctatagtt ctcaaaggta 5941 gatagtaaaa tcactggctt tttccagctg tatgtttttc cactgtgcgt gtacacacac 6001 actggaaaat aattaggctg attttgcagg tcttcattgt tagagattct gaagtattta 6061 ctgtcaattc ataggtttca gtttatttag gaaattagtg tttgacagct ttttttaaat 6121 tatttcactg aagctgagat tattagtgat acaaagttaa aatttcaata tttaatttct 6181 ctatatatta ttaatattaa attgtttttt acttataaat tcatgttctc atctgattta 6241 atattaaatt tgtataggtg ggcgtttctt accattttgc acaagttttt gtttttctga 6301 aatacttaat tgtgcaggtt gtaaaaaaga ttagtgcatt ttcattttaa ggatgctttg 6361 ctccttaaat tgttcgacag aaatgacttt ttagggaaag tagttttttt ggagctacta 6421 acttgtattt attattgtac atgcataacc agggtggtga gggcactaat cttgtaggaa 6481 acacttactt gatgttttat ttgaactttt cctataggtt taacttttac tgcatagaat 6541 taacactagg aacagtgtca tgaaatctgg gttgaaggag aatacagtat atatgagaac 6601 acttaaagtt caaatagaaa tcatttctga agacaaaagc agaggaatat tgtcagtgcc 6661 aagtaatgga agaataaggg cggcatttac actgtgcaag tattgagaag agtgcataaa 6721 gacagggaac tactctcatg gagacagttt ctctcttata atcaagtaac tagaagggga 6781 aaaatcatct aagttatgaa atccaacata ggcgctatat tacaaactgt gccggattat 6841 gcaaattgta gttgttactg atcaaagttt aattgcttca tttttgttta aaaagggata 6901 ctgatgtcag aaaatctgta atatgtttta ttcaaaagat gtaaataatg tatacagact 6961 tgtatgtgat gggatgggaa atatttaaat tctaggtgtt tttttttttt taaagaagaa 7021 actcaatgtt tataagaaaa aaatgaataa atagttacgt ttggccatga atcctgaaaa 7081 aaaaaaaaaa a |
| **SEQ ID NO:17 RAB27B mRNA transcript 7003 bp** |
| 1 actcgcagtc ctgacgggca ggggctgcgg accgcccggc cttggaccca tccggagcca 61 caggttggag gagataagta gctgtccccg tgctcatcgc cctgtggagc agatcctgtc 121 tccttgctga cggtggagcc cgggagttcc agggcttggg aaggggaagg aaacctctct 181 gaaatctgac acctgctctc ccggcaagga aacttcgcag gctgaccgac caagaccatc 241 actatgaccg atggagacta tgattatctg atcaaactcc tggccctcgg ggattcaggg 301 gtggggaaga caacatttct ttatagatac acagataata aattcaatcc caaattcatc 361 actacagtag gaatagactt tcgggaaaaa cgtgtggttt ataatgcaca aggaccgaat 421 ggatcttcag ggaaagcatt taaagtgcat cttcagcttt gggacactgc gggacaagag 481 cggttccgga gtctcaccac tgcatttttc agagacgcca tgggcttctt attaatgttt 541 gacctcacca gtcaacagag cttcttaaat gtcagaaact ggatgagcca actgcaagca 601 aatgcttatt gtgaaaatcc agatatagta ttaattggca acaaggcaga cctaccagat 661 cagagggaag tcaatgaacg gcaagctcgg gaactggctg acaaatatgg cataccatat 721 tttgaaacaa gtgcagcaac tggacagaat gtggagaaag ctgtagaaac ccttttggac 781 ttaatcatga agcgaatgga acagtgtgtg gagaagacac aaatccctga tactgtcaat 841 ggtggaaatt ctggaaactt ggatggggaa aagccaccag agaagaaatg tatctgctag 901 actctacata gaaactgaac atcaagaacc ccaccaaaat attactttta aaaacaatga 961 caaaccacac aattgttgtt gagtaaacca cgcacaatgg catgtctttc tttttctgcc 1021 agaaaatcta ttttaagaaa ccagaatagt caacagtgtt caaaagaatt gactagttat 1081 ccctgaggcc ctttcaaaca tgatcaaaga tttcccaatg tgatctcatc atcatggata 1141 ctcaatttgt tttttcttat agagaaaatg agtatataag acaatataca agaagaaata 1201 tcagtgagtt ttaaatcaga acaagttacc tgtcacattg aagaaaaggg taggcactaa 1261 agggagaaca cagaaagaag aatttctaaa atattggatt tacttcttat attgagtcag 1321 atgcatactt ttagatttgc attggggaaa atgtactagc taaaaatgga tacacaatga 1381 agaattctat ttggctaatt aagaatgata tactatgtac acccaataag ctgtactaga 1441 atgaataaat tactgataag gttacaaata ggtaaatgtc acacttctgt taaaatgcag 1501 gaggtagtgt cataatgccg tctttatatt cttaataaat agcactttga caagaacagg 1561 actgtaaatg atgaagtaca agacaaatac cctgggaaaa aaaatgaaag tatgagaaat 1621 tggcatttct acagctgaaa ttcaatgtat ctgttagaga tgtctggaag ggttacttag 1681 ccaaatttta ctcaagccaa ttaggagctg atattatcag ttggaattaa gagaactcca 1741 gaggtttcca tttcaaacaa aattttagaa attggtttgg tgttcagctt cacatttcat 1801 tttttcttag cacatgttga taaaatagtc acaaggagaa attaccagtt acggtttatt 1861 aaatctcttt taaaatgcag tcaaggaaaa ctagccttga atttttttta gataaaataa 1921 gatggtgata tgaaacaaaa agtggcaatt attgcaggtt tccttttagt ttacaaaagt 1981 actggaaact aaatcatatt tcttccctcc aaatttcacc cattcctgac tttgaatcaa 2041 ttgcagaaat gcaggtgtgt tactttgttg atcaataact ttggaacaat tatggatcaa 2101 ttctatggtc actctgaatt ttcatgtcat taatcacata aaaattgata atacctcatt |
| 2161 ctgtattaca atatgatttt attttgccaa aggcaagaca cctatagttg agctgtattt 2221 tgggggattg ggtgaggaag gacttctgat cttatctcaa caaaaaactg gccagtattt 2281 ttgttaatgt aaagcttcct tttctttcta aaaaatagta acaaaattat ttttcattgg 2341 cctattctgt tcttgtgtct aaactaacat tacattaatt tttaatctta gtttctgata 2401 aacacaagcc attcctatca aaatattatt tatttcagtc aattttacca aataacaaag 2461 acaatatatt ttcgtttttt tttattatga gcatatgatt ttttgacagg ctgtttcctc 2521 gtcgtataga ttttttccaa tcaaacctac tttttccata ctctgtgcat attttttgtg 2581 aagttataca cattgaagac cctaaaaatc ccagtccatc attcagctta cctctgcgaa 2641 cttctatctg gtattgaatc agtttcagaa acacagacag atccaaggaa atgtctcttt 2701 ataatgttct taggatggac tagacccata aatgtgccat gaatcaaaat attaataatt 2761 tgaaagcttt catgctgtta gcccctgatg aaattctcag cattaactgg ccagctcctc 2821 tgatttctgc agcatcgcaa caggttcgaa gatgggttgt ggctgggtat tccctcccat 2881 ggtgtttcct ctgggatgct cttcattatc tcaatgcctg tgccatgaag atagaaaact 2941 gtaagctaac atttaagatg tttcttctgg aaggaaagtg agcaggaaca agttatattg 3001 ccactgctgt ggcaaatttt ggtgaacttt tggggtcatt atatcaattt tttctttgga 3061 ttcaaattgt aatgtcccct gcatttcctt aatagggaat gtgaaacctt tataaaactc 3121 taaaagtatt ctgttttgat atgtcttttt gtttctattc attttcagtt atatgattga 3181 tttacttatg ccaagattct gtcactgtca gttatttaat gagtgttttt tcagggtctg 3241 ttttaagatc attatttgat agctgtagca tgaagcagag gttgatgatg cccataattg 3301 caagactatt cctgtaaaaa taacaattat tgggtaataa cttcaagagg aatgagaagt 3361 gacaaaattg atttaaaata ttgttctact tataaataaa tgcttgatat aaaaaatttt 3421 ctccataaag tttgacatct gaccccagat tctatgtaat cattattaga aattccttct 3481 ctcattattt caggattagt agttctgtgt aattcatttt acaatttcaa attgttctgg 3541 tgccataaag tatacagact actttaaaga tttccaaatc ccctaattta ccccacaaca 3601 gcatgtaatt ttagccaaga tatgtcctgt tactaagtat ctcccaatgc tttagtaaaa 3661 cgtatttagg agaaatgttg aaaatgtaca tgaagctcct ttctgatata gaaaccattt 3721 ctggagtatt tacactggtt tgatgtttac attgctctaa ctcggtgcct cagatacctc 3781 tgtgaccaaa tttgtctcca accacatagc tcatttccta taatgttata tcataggaag 3841 ccctcacaga gacactaaca cagctaaaga tcttctgata ttatcagcaa gggatgcaag 3901 gactttattg gaatctggag agtttaactg ccttctcttg gtctcctcac ttacttctta 3961 tgaagttggc attacctgag actcttagct gtgattaggt acaagcttac cttttagggt 4021 agaaaaagaa agatcatttg aaaaatgtat ctaaaataat ccagagaaca taatgtttgt 4081 cttggtctga taatgataag aagtcaagga ttggcagaga aaatactaaa cgccaagagt 4141 tgagcctgtg ggtctctcca taagagtttt aaaactcttg ccagttacca ctttatccaa 4201 tttgctatca ttttcgtatt atcagctatc gccctgtaaa atattcaaaa ctagctattt 4261 ctaaagtaaa cattttatct gttactttta accagatagg tgtctttgtc atccttctac 4321 tataaattgt tctttgccaa cctgtacagg tagatgaacc aggcgagagt tttaatcagc 4381 cttttcttgt cccctttgta agaaagagat gcttgccata gagaaggaca tgagtacatt 4441 aaaaataatt taatagccac aatatgatgt tctttaagct gcaaattgag tacactggga 4501 atcaacaaat ttgatgaagc ctgtctgtct cttcaccagt ggagtgagtg cagcagttag 4561 aaagagaagc aatattgtgc aactggtgca gtggtgagtt aatcatagtg tataaccttg 4621 tgttcatgaa acaggttgtt cattgttctg catctctctt catttaaaaa ggatacacaa 4681 ttctttcctc attgcatatt acaccaaacg tttgagggaa aaatcctcat tcgtaaagga 4741 ttttggatgt ataatctaaa actcaacaat aaagaaataa tattccaagt ctctggtttc 4801 ctaagataca taataactgt ttataaagaa ggtctaagag ctgatatttg ccaaagtgat 4861 agaagagttg ttttttcctc tctactacca agctttaaga cattaaaaga agtctagtgt 4921 atttgaatat tttagagaaa gctttatcat tttttaagat gccaagatgc tgcctacgtt 4981 tgcaaaagtt gtctaagaat tcaccatgag ctatattttc ttctggatct ttgaccaagg 5041 tgatgtcagc ttatttctgg ggaaggtgtt gagctcttat acatgaaaat ggatataggc 5101 tattctctgg gatgagtgtc atttcaatgc tttataaatc catgaagctg cttgtctcat 5161 aaagtagaac tgatacaaat tttggttgga tatatagaga attttataaa tgtattgcct 5221 tagaatttct gggtggagac ccaactacaa tgacattgtc atgccagaac tataaagata 5281 attagagtta aaagttgttt aaattgtgcc cttaaataca gcagaacctg gagaaggtca 5341 tacttcaaag gtcgattttg agtccgaata aagaaagacc tagtaacaga tagttttttt 5401 ttgttcattt tcttctacca agtagaggtt tatgccctca gaactaaact agtaaaaata 5461 tctgaacaaa aaacctttcg ttgttggcat aaaaatgtga tacacttaga gacattttgt 5521 ttattgcata taaatctaat ttttccataa attagattta tgatattttc ataaagcact 5581 tgattagttt ttcaaggcgt accatcacaa agatgctttc ctgcagagtt ctttgtatca 5641 acagcctatg gttgagatgt tttctcattt cctgtagaga gagaatacca ctaacaaaca 5701 aacaaaaact ttagtgccaa aatagtggaa ctattttgtc atcttttgag aaaaaaatat 5761 acaaagaagt catcttttca ttaagtggat tccctggttc ctttccagct ggttgtggaa |
| 5821 gtaatggcta acatccttca gctgactttg tctacaagga ttattagcaa attctgtagg 5881 agcaagcatg tctgacctta acttaatgga tcccttattc aatcagtggc ttctgtcttt 5941 atgtctgttg gcatatcaaa atggtttctg ttcctagaaa agtaataaca tatgcttatc 6001 tttattcttt ttccaggtga ttttgttttc aaatgctcct tgtgaaaaca cctagtgttg 6061 tagaaaggaa agtggccaga aagaacaact tgggaccatg agtaggtcat taaatagctt 6121 agtgatttat cctcatatag ggcttataaa ccctgtatgt gtttatatgt gcttcacaga 6181 gttcgtgtca ggctcaaagg agatatgtat aagaaagtgg tttgtaaatt atgttccatt 6241 tcataaatag acactattca caaactaaaa tctaataaaa aaccacagtt gtaatttaaa 6301 ctgcttgata taaaaagagg tatcatagca gggaaaacac actaattttc atacagtaga 6361 ggtattgaaa actgaaaatg ggaaggcaac ttgaagtcat tgtatttgat tgaaaatgtt 6421 taatacatct cattattgac aaaatatgtc atcttgtatt tatttcaagg aaaccaatga 6481 attctaggta gtatattaca agttggtcaa aatattccat gtacaaatag ggcttctgtg 6541 tccatagcct tgtaagagat actgattgta tctgaaatta ttttttaaaa aaataaatta 6601 tcctgcttta gttagtgtgt taaaagtaga cgatgttcta atataacact gaagtgcttc 6661 attgtatccc aacagtttac cttcaagtaa tattatcttt atttttaggc taagcacgtt 6721 tgattatttt gtctgtctcc tatatagatc tgttttgtct agtgctatga atgtaactta 6781 aaactataaa cttgaagttt ttattctata tgccccttaa tagactgtgg ttcctgacgc 6841 acactgttag gtcattattt tgttgtacca aagttctagt ggcttcagaa atcatagcat 6901 ccaatgattt tttggtgtct ggctatgaat actatggttg agaattgtat tcagtgattg 6961 tttctgcaca cttttcaaat aaaaaatgaa tttttatcaa tta |
| **SEQ ID NO:18 RGS18 mRNA transcript 2158 bp** |
| 1 agttctgcat ttctgcagag acagaaagaa acgcagctct tgacttcttt tttgtaaaca 61 ttactgtaag agttgtgata actttttatt ctactatgta tatgtatgga atagtattaa 121 taaatgaact agggaaggat gtaataaatt agacatctct tcattttaga gagaagatgg 181 aaacaacatt gcttttcttt tctcaaataa atatgtgtga atcaaaagaa aaaacttttt 241 tcaagttaat acatggttca ggaaaagaag aaacaagcaa agaagccaaa atcagagcta 301 aggaaaaaag aaatagacta agtcttcttg tgcagaaacc tgagtttcat gaagacaccc 361 gctccagtag atctgggcac ttggccaaag aaacaagagt ctcccctgaa gaggcagtga 421 aatggggtga atcatttgac aaactgcttt cccatagaga tggactagag gcttttacca 481 gatttcttaa aactgaattc agtgaagaaa atattgaatt ttggatagcc tgtgaagatt 541 tcaagaaaag caagggacct caacaaattc accttaaagc aaaagcaata tatgagaaat 601 ttatacagac tgatgcccca aaagaggtta accttgattt tcacacaaaa gaagtcatta 661 caaacagcat cactcaacct accctccaca gttttgatgc tgcacaaagc agagtgtatc 721 agctcatgga acaagacagt tatacacgtt ttctgaaatc tgacatctat ttagacttga 781 tggaaggaag acctcagaga ccaacaaatc ttaggagacg atcacgctca tttacctgca 841 atgaattcca agatgtacaa tcagatgttg ccatttggtt ataaagaaaa ttgattttgc 901 tcatttttat gacaaactta tacatctgct tctaacatat cgcatgttta tgttaagatt 961 tggtcccatc ctttaaactg aaatatgtca tgtgaaatta ttttaaaaat gtaaaaacaa 1021 aactttctgc taacaaaata catacagtat ctgccagtat attctgtaaa accttctatt 1081 tgatgtcatt ccatttataa tcagaaaaaa aacttatttc ttaatcaaaa ggcagtacaa 1141 aaaaagtaat aatgttttat aagattgtag agttaagtaa aagttaagct tttgcaaagt 1201 tgtcaaaagt tcaaacaaaa gtctagttgg gattttttac caaagcagca taatatgtgt 1261 tatataaaca taataatact cagatatcca aatgttcaga tagcattttt cataatgaat 1321 gttctctttt ttttggtaat agtgtagaag tgatctggtt cttacaatgg gagatgaaga 1381 acatttatta ttgggttact actaaccctg tcccaagaat agtaatatca cctctagtta 1441 taagccagca acaggaactt ttgtgaagac acattcatct ctacagaact tcagattaaa 1501 tataatctag attaatgact gagaataaga tccacatttg aactcattcc taagtgaaca 1561 tggacgtacc cagttataca aagtacttct gttggtcaca gaaacatgac cagattttgc 1621 atatctccag gtagggaact aagtagacta ccttatcacc ggctaagaaa acttgctact 1681 aaactattag gccatcaatg gcttgaataa aaaccagaga aggtttttcc caggacgtct 1741 catgtttggc cctttagaat tggggtagaa atcagaaatg agatgagggg aagaagcaag 1801 gagtctaagg ccctagcgat ttgggcatct gccacattgg ttcatattca gaaagtgtta 1861 tctcattgat tatattcttg ttaagcaaat ctccttaagt aattattatt caaataagat 1921 tatactcata catctatatg tcactgtttt aaagagatat ttaattttta atgtgtgtta 1981 catggtctgt aaatatttgt atttaaaaat gccatgcatt aggctttgga aatttaatgt 2041 tagttgaaat gtaaaatgtg aaaactttag atcatttgta gtaataaata tttttaactt 2101 cattcataca gttaagttta tctgacaata aaagctctga ctgaaaaaaa aaaaaaaa |
| **SEQ ID NO:19 TBC1D15 mRNA transcript 5852 bp** |
| 1 ttttgccgga tgttgttgta tgtccgagag acacgtgagg ttctgctacg tcattaccag 61 gcacgcgcag gaaacatggc ggcggcgggt gttgtgagcg ggaaggtttt tggtttcttc 121 ttgattcaat cttgataagt agtatgtgtc caggacttta tccatactcc agtttgttgg 181 agtatggtag gagtatgatt atatatgaac aagaaggagt atatattcac tcatcttgtg 241 gaaagaccaa tgaccaagac ggcttgattt caggaatatt acgtgtttta gaaaaggatg 301 ccgaagtaat agtggactgg agaccattgg atgatgcatt agattcctct agtattctct 361 atgctagaaa ggactccagt tcagttgtag aatggactca ggccccaaaa gaaagaggtc 421 atcgaggatc agaacatctg aacagttacg aagcagaatg ggacatggtt aatacagttt 481 catttaaaag gaaaccacat accaatggag atgctccaag tcatagaaat gggaaaagca 541 aatggtcatt cctgttcagt ttgacagacc tgaaatcaat caagcaaaac aaagagggta 601 tgggctggtc ctatttggta ttctgtctaa aggatgacgt cgttctccct gctctacact 661 ttcatcaagg agatagcaaa ctactgattg aatctcttga aaaatatgtg gtattgtgtg 721 aatctccaca ggataaaaga acacttcttg tgaattgtca gaataagagt ctttcacagt 781 cttttgaaaa tcttcttgat gagccagcat atggtttaat acaaaaaatt aaaaaggacc 841 cttatacggc aactatgata ggattttcca aagtcacaaa ctacattttt gacagtttga 901 gaggcagcga tccctctaca catcaacgac caccttcaga aatggcagat ttcttagtg 961 atgctattcc aggtctaaag ataaatcaac aagaagaacc aggatttgaa gtcatcacaa 1021 gaattgattt gggggaacgc cctgttgttc aaaggagaga accggtatca ctggaagaat 1081 ggactaagaa cattgattct gaaggaagaa ttttaaatgt agataatatg aagcagatga 1141 tatttagagg gggacttagt catgcattga gaaagcaagc atggaaattt cttctgggtt 1201 attttccctg ggacagtacc aaggaggaaa gaacccaatt acaaaagcaa aaaactgatg 1261 aatacttcag aatgaaactg cagtggaaat ccatcagcca ggaacaagag aaaagaaatt 1321 cgaggttaag agattataga agtcttatcg aaaaagatgt taacagaaca gatcgaacaa 1381 acaagtttta tgaaggccaa gataatccag ggttgatttt acttcatgac attttgatga 1441 cctactgtat gtatgatttt gatttaggat atgttcaagg aatgagtgat ttactttccc 1501 ctcttttata tgtgatggaa aatgaagtgg atgccttttg gtgctttgcc tcttacatgg 1561 accaaatgca tcagaatttt gaagaacaaa tgcaaggcat gaagacccag ctaattcagc 1621 tgagtacctt acttcgattg ttagacagtg gattttgcag ttacttagaa tctcaggact 1681 ctggatacct ttatttttgc ttcaggtggc ttttaatcag attcaaaagg gaatttagtt 1741 ttctagatat tcttcgatta tgggaggtaa tgtggaccga actaccatgt acaaatttcc 1801 atcttcttct ctgttgtgct attctggaat cagaaaagca gcaaataatg gaaaagcatt 1861 atggcttcaa tgaaatactt aagcatatca atgaattgtc catgaaaatt gatgtggaag 1921 atatactctg caaggcagaa gcaatttctc tacagatggt aaaatgcaag gaattgccac 1981 aagcagtctg tgagatcctt gggcttcaag gcagtgaagt tacaacacca gattcagacg 2041 ttggtgaaga cgaaaatgtt gtcatgactc cttgtcctac atctgcattt caaagtaatg 2101 ccttgcctac actctctgcc agtggagcca gaaatgacag cccaacacag ataccagtgt 2161 cctcagatgt ctgcagatta acacctgcat gatcactgtt cttgcttttt tgggaagaga 2221 cactttgttg caaccctttt tcaagtactt gaaagttgaa aatttgaaat cttggtattg 2281 atcatgcttt aaggtttatg taaagaaagt gtactgatgt tcttacatta aagctttaca 2341 aagatttaaa ctaattattt ttgtagttac ttctaccaaa tagcctttcc ttttcgataa 2401 cattcctcag tatttttata gccaagtaca ttttattttc ttgctgatga actggaattg 2461 gataaatatt gcaagtggat gagttggaaa ttatgcactt tgaaaaacat tcactttgtt 2521 taagcttatt gggtttcaga tttgattaaa ttaaatgtgg aggctttcta tagcattcta 2581 agctgagaag tagattgtta cccagtaatg aaataaaaaa taaaaataaa aggatttttt 2641 tctctattgt ttacgacagt actcagctta aatatttatg ctggtcaaat gtgatttaaa 2701 ttggacattt tcatcaatgc agtctaatgt gtagataaat atttcaacca taataagtgg 2761 attggcagta tattttttac attgaacttt tcttcacttg tatataaaga ttatatataa 2821 gtacttattt atgagtataa gaaaggttag gcatattttc attaactgaa taaacgactt 2881 gatttatata acctggttta tcaaaattta acatggcttc agtatgagat ctttttcaaa 2941 actattttct taaacattta tttcatgaga ttatgttcaa ccctgtacct ggtgtaattt 3001 taaaattaat tgcttgtaac ctcactttac taataatgtt tattatcttt cctaataatg 3061 cattaactga ttaatcaggt gtttaaattt ttataaaata ctcttgcaaa aagtttattt 3121 gaaaaatttc tagatggtct catgagtttc aaaataataa tttttgtgta tgaacaaagc 3181 tgttgttttt accatgcagt attgcatgat tttaagttat gtggaattaa cataactgat 3241 tttgttttaa ttgtaagttg ttaactcctg tatatatcat taaaataaat ctgaagttga 3301 agtagtgttt ttagttaaat tatacttaga aatagtctgc ttttttaaaa ttttttttct 3361 tgagaaagag tcttgctctg ttgcccaggc tggagtgcag tggcgcagtc ctggctcact 3421 gcagcctccg ccttctgggt tcaagcgatt ctcctgtctc agcctcccga gcagctggga 3481 ctacaggctt gtgccatcgc gcctgactaa tttttgtatt ttgagtagag atggggtttc 3541 accatgttgg ccaggctggt ctcgaactct tgacctcaag tgatccactc gcttcagcct 3601 cccaaagtgc tgagattaca ggtgtgagcc actgtgcccg gctaattctt taatagaaga |
| 3661 aaaaacatcc aagatggacc tcaattcatc tcttattttt atatgattaa aatgataatc 3721 tggccgggcg cggtggctca cgcctgtaat cccagcactt tgggaggccg aggcgggcgg 3781 atcacgaggt caggagatcg agaccatccc ggctaaaacg gtgaaacccc gtctctacta 3841 aaaatacaaa aaattagccg ggcgtagtgg cgggcgcctg tagtcccagc tacttgggag 3901 gctgaggcag gagaatggcg tgaacccggg aggcggagct tgcagtgagc cgagatcccg 3961 ccactgcact ccagcctggg cgacagagcg agactccgtc tcaaaaaaaa aaaaaaaaaa 4021 atgataatct gaataagtta tggaaatgaa aaccatcctt tttataactg aaaaaaaatt 4081 ttcattagca tggaaatggg cacagtgttg ccttgaaaga tacagttatt tgactcagta 4141 aagcagctta ttacaactga tgctaatagt atagagaaaa aagttgtgca gttctaaaat 4201 ggtcctagag attgactttt ttcccccaag aaagttaggg aacaaaacga acttttttcc 4261 tggttgagca ttaactgaca atcacgacag tagaaccgtt agagtttagt ttttaatatt 4321 atgtgtgtta tctttcatca gttaataatg agtaagccta ttcagaaaaa gaacataaac 4381 tgatcaaaaa ctcagcatct ccagcctttc atttcctgct attcaggaaa ttgcttagaa 4441 catcttgatg tcctccttgt tcttcctgga cagtgacttt ttgggagttt gttcctgctg 4501 cgtaatgtga tacccacttc agattttttt tttatcaata catttagtaa gttgaacttc 4561 tgtcaagttt tattacaaaa ttacttgtta aaacaatttt tactaaactg catttctatc 4621 tagcatattt ttgatatgga agtgatagta tagtatagtt ccaggagaag tcttaaatca 4681 gtccacagag tccagttagc aaatactctg tgccattaag attgctaaaa tacacagttc 4741 aggtaaattt actagtgttt tttaaaggtt tatttgtttt cacaagatgc tctgtccaca 4801 cccttataac atgtaaaata ttgtgtgctg tattatgtgg taaagttgtt aaaattcagt 4861 ttctaacatt aacttaaaag tacagacaat ctaacatgat gatttgactt acaaactttc 4921 aactaaattt atgatggctt taaagcagtg cactgaatag aaaccatact ttgagtaccc 4981 atacagccat ttttcacttt tactacaata ttctataaat cacatgagat atttaacact 5041 ttattataaa ataggctttg tgttagatga ttttgcccaa atgtaaacta atgtagtgtt 5101 ctgagcatgt ttaagttagg gtaggctaaa ctatgtttgg taggttagat gtattaaaag 5161 catttttgat taatgatgtc ttcaatttat gatgtgttta ttggaacata acctcaatat 5221 aagttgaaaa gcatatgtat tttcaattct ggcatgaacc tatgggaatc ttttgcattt 5281 aagaacctcc ccattttaat aatttcatgg gtctaagatt cttcatctgt ttataaggaa 5341 ctttagtctt agtgattaga gactaaattt ttttttgagc agtaagaaaa cagccttttg 5401 ggacagatag tgagtgattc ttaggaactt gacattgcca agaaatttta tagatgccga 5461 agaattctta tgtgaaattc acataagcat gcccattact aaagacagtt tgtataaagt 5521 aaccctaaat gtttattgag gaacctacag cttcaactga cttacgtgca gatatgtacc 5581 aggagaacat cattttagct tgggcgtctt tacttggggt tttcagagga tccaggaacc 5641 tcactgtatg caaagtcttg tggatgtacc tgaatgtttt tggaggcagg tcacatagtt 5701 tctgaaagtg ttctcttatt ttcctcaaat gtaggtaacc attgttacaa gttatttaac 5761 aggagaatag taacaatgtc taacttatgc taatgatttt gtgtgctgag ctcccattaa 5821 ttaaaatgtc ttcagaaaaa aaaaaaaaaa aa |

Ngo et al., Science 360, 1133-1136 (2018) is referenced.

## Claims

1. A method for assessing risk of preterm delivery by a pregnant woman, comprising analyzing a maternal sample of the pregnant woman to determine an expression profile from a panel comprising TBC1D15, wherein the three or more genes have higher expression levels in the preterm population RAB27B and two or more genes selected from PPBP, DAPP1, RGS18, CLCN3, MAP3K7CL, MOB1B, POLE2, LYPLAL1, and than in a normal population.

2. The method of claim 1 or 2, wherein the maternal sample is maternal blood, maternal blood plasma, maternal blood serum, or urine.

3. The method of claim 2, wherein the maternal sample is the maternal blood serum.

4. The method of claim 2, wherein the maternal sample is the maternal blood plasma.

5. The method of any of claims 1 to 4 wherein the panel comprises RAB27B, PPBP, and DAPP1.

6. The method of any of claims 1 to 5, wherein the expression profile is determined by measuring cell-free RNAs (cfRNAs) in the maternal sample.

7. The method of claim 6, wherein the expression profile comprises expression levels of individual genes that are determined by qPCR or massively parallel sequencing.

8. The method of claim 1, wherein the expression profile is determined by measuring proteins in the maternal sample, optionally wherein the proteins are measured by mass spectrometry.

9. The method of any of claims 1 to 8, wherein the maternal sample is from the second trimester of pregnancy.

10. The method of any of claims 1 to 8, wherein the maternal sample is from the third trimester of pregnancy.

11. The method of any of claims 1 to 8, wherein the maternal sample is from more than 28 days prior to preterm delivery or is from more than 45 days prior to preterm delivery.

12. The method of any of claims 1 to 11, further comprising analyzing two or more maternal samples of the pregnant woman from different times during the course of a pregnancy.

13. A method performed using a computer for assessing risk of preterm delivery by a pregnant woman comprising:
(a) obtaining one or more expression profiles from a maternal sample of the pregnant woman, wherein the one or more expression profiles correspond to the expression of a plurality of cell-free RNA (cfRNA) transcripts from a first panel of genes;
(b) comparing, using a computer system, the one or more expression profiles to one or more reference profiles characteristic of a woman with (a) a high risk of preterm delivery or (b) a low risk of preterm delivery, or characteristic of a woman with a defined length of pregnancy, wherein the one or more reference profiles are determined using a machine learning model that analyzes first training samples that are cfRNA expression profiles labeled as preterm or full-term, or labeled with a length of pregnancy; and
(c) updating, using the computer system, the one or more reference profiles by:
(1) receiving second training samples, wherein the second training samples are cfRNA expression profiles labeled as preterm or full-term or labeled with a length of pregnancy, and
(2) iteratively adjusting the one or more reference profiles using a machine learning model to increase the number of the first training samples and the second training samples that are classified correctly,
wherein the first panel of genes comprises RAB27B and two or more genes selected from PPBP, DAPP1, RGS18, CLCN3, MAP3K7CL, MOB1B, POLE2, LYPLAL1, and TBC1D15.

14. A computer system comprising: (a) a database comprising one or more reference profiles, each including (i) a level of expression in a population of pregnant women of cell-free RNA (cfRNA) transcripts corresponding to a first panel of genes and (ii) a risk of preterm delivery; (b) a user interface configured to interact with a client computer over a network and to receive one or more expression profiles including the level of expression in a pregnant woman of cfRNA transcripts corresponding to the first panel of genes; and (c) one or more processors configured to analyze the one or more reference profiles and the one or more expression profiles, including comparing the one or more reference profiles to the one or more expression profiles to determine the risk of preterm delivery; and (d) a network interface that transmits the risk of preterm delivery to the client computer,
wherein the first panel of genes comprises RAB27B and two or more genes selected from PPBP, DAPP1, RGS18, CLCN3, MAP3K7CL, MOB1B, POLE2, LYPLAL1, and TBC1D15.

## Patentansprüche

1. Verfahren zum Bewerten des Risikos einer Frühgeburt bei einer schwangeren Frau, umfassend Analysieren einer mütterlichen Probe der schwangeren Frau, um ein Expressionsprofil aus einem Panel zu bestimmen, das RAB27B und zwei oder mehr Gene ausgewählt aus PPBP, DAPP1, RGS18, CLCN3, MAP3K7CL, MOB1B, POLE2, LYPLAL1 und TBC1D15 umfasst, wobei die drei oder mehr Gene höhere Expressionsniveaus in der Frühgeborenenpopulation als in einer normalen Population aufweisen.

2. Verfahren nach Anspruch 1 oder 2, wobei die mütterliche Probe mütterliches Blut, mütterliches Blutplasma, mütterliches Blutserum oder Urin ist.

3. Verfahren nach Anspruch 2, wobei die mütterliche Probe das mütterliche Blutserum ist.

4. Verfahren nach Anspruch 2, wobei die mütterliche Probe das mütterliche Blutplasma ist.

5. Verfahren nach einem der Ansprüche 1 bis 4, wobei das Panel RAB27B, PPBP und DAPP1 umfasst.

6. Verfahren nach einem der Ansprüche 1 bis 5, wobei das Expressionsprofil durch Messen von zellfreien RNAs (cfRNAs) in der mütterlichen Probe bestimmt wird.

7. Verfahren nach Anspruch 6, wobei das Expressionsprofil Expressionsniveaus von einzelnen Genen umfasst, die durch qPCR oder massiv parallele Sequenzierung bestimmt werden.

8. Verfahren nach Anspruch 1, wobei das Expressionsprofil durch Messen von Proteinen in der mütterlichen Probe bestimmt wird, wobei optional die Proteine durch Massenspektrometrie gemessen werden.

9. Verfahren nach einem der Ansprüche 1 bis 8, wobei die mütterliche Probe aus dem zweiten Schwangerschaftstrimester ist.

10. Verfahren nach einem der Ansprüche 1 bis 8, wobei die mütterliche Probe aus dem dritten Schwangerschaftstrimester ist.

11. Verfahren nach einem der Ansprüche 1 bis 8, wobei die mütterliche Probe von mehr als 28 Tagen vor Frühgeburt ist oder von mehr als 45 Tagen vor Frühgeburt ist.

12. Verfahren nach einem der Ansprüche 1 bis 11, ferner umfassend Analysieren von zwei oder mehr mütterlichen Proben der schwangeren Frau von unterschiedlichen Zeiten während des Verlaufs einer Schwangerschaft.

13. Verfahren, durchgeführt unter Verwendung eines Computers zum Bewerten des Risikos einer Frühgeburt bei einer schwangeren Frau, umfassend:
(a) Erhalten von einem oder mehreren Expressionsprofilen aus einer mütterlichen Probe der schwangeren Frau, wobei das eine oder die mehreren Expressionsprofile der Expression einer Vielzahl von zellfreien RNA(cfRNA-)Transkripten aus einem ersten Panel von Genen entsprechen;
(b) Vergleichen, unter Verwendung eines Computersystems, des einen oder der mehreren Expressionsprofile mit einem oder mehreren Referenzprofilen, die für eine Frau mit (a) einem hohen Risiko einer Frühgeburt oder (b) einem niedrigen Risiko einer Frühgeburt charakteristisch sind, oder für eine Frau mit einer definierten Schwangerschaftslänge charakteristisch sind, wobei das eine oder die mehreren Referenzprofile unter Verwendung eines Modells für maschinelles Lernen bestimmt werden, das erste Trainingsproben analysiert, die cfRNA-Expressionsprofile sind, die als Frühgeburt oder voll ausgetragen markiert sind oder mit einer Schwangerschaftslänge markiert sind; und
(c) Aktualisieren, unter Verwendung des Computersystems, des einen oder der mehreren Referenzprofile durch:
(1) Empfangen von zweiten Trainingsproben, wobei die zweiten Trainingsproben cfRNA-Expressionsprofile sind, die als Frühgeburt oder voll ausgetragen markiert sind oder mit einer Schwangerschaftslänge markiert sind, und
(2) iteratives Anpassen des einen oder der mehreren Referenzprofile unter Verwendung eines Modells für maschinelles Lernen, um die Anzahl der ersten Trainingsproben und der zweiten Trainingsproben, die korrekt klassifiziert sind, zu erhöhen,
wobei das erste Panel von Genen RAB27B und zwei oder mehr Gene ausgewählt aus PPBP, DAPP1, RGS18, CLCN3, MAP3K7CL, MOB1B, POLE2, LYPLAL1 und TBC1D15 umfasst.

14. Computersystem, umfassend: (a) eine Datenbank, die ein oder mehrere Referenzprofile umfasst, jeweils beinhaltend (i) ein Niveau an Expression von zellfreien RNA(cfRNA-)Transkripten in einer Population von schwangeren Frauen, die einem ersten Panel von Genen entsprechen, und (ii) ein Risiko einer Frühgeburt; (b) eine Benutzerschnittstelle, die konfiguriert ist, um mit einem Client-Computer über ein Netzwerk zu interagieren und um ein oder mehrere Expressionsprofile zu empfangen, beinhaltend das Niveau an Expression von cfRNA-Transkripten, die dem ersten Panel von Genen entsprechen, bei einer schwangeren Frau; und (c) einen oder mehrere Prozessoren, die konfiguriert sind, um das eine oder die mehreren Referenzprofile und das eine oder die mehreren Expressionsprofile zu analysieren, beinhaltend Vergleichen des einen oder der mehreren Referenzprofile mit dem einen oder den mehreren Expressionsprofilen, um das Risiko einer Frühgeburt zu bestimmen; und (d) eine Netzwerkschnittstelle, die das Risiko einer Frühgeburt an den Client-Computer überträgt,
wobei das erste Panel von Genen RAB27B und zwei oder mehr Gene ausgewählt aus PPBP, DAPP1, RGS18, CLCN3, MAP3K7CL, MOB1B, POLE2, LYPLAL1 und TBC1D15 umfasst.

## Revendications

1. Procédé permettant l'évaluation du risque d'accouchement prématuré chez une femme enceinte, comprenant l'analyse d'un échantillon maternel de la femme enceinte pour déterminer un profil d'expression à partir d'un panel comprenant RAB27B et deux gènes ou plus sélectionnés parmi PPBP, DAPP1, RGS18, CLCN3, MAP3K7CL, MOB1B, POLE2, LYPLAL1, et TBC1D15, dans lequel les trois gènes ou plus comportent des niveaux d'expression plus élevés dans la population prématurée que dans une population normale.

2. Procédé selon la revendication 1 ou 2, dans lequel l'échantillon maternel est du sang maternel, du plasma sanguin maternel, du sérum sanguin maternel ou de l'urine.

3. Procédé selon la revendication 2, dans lequel l'échantillon maternel est le sérum sanguin maternel.

4. Procédé selon la revendication 2, dans lequel l'échantillon maternel est le plasma sanguin maternel.

5. Procédé selon l'une quelconque des revendications 1 à 4, dans lequel le panel comprend RAB27B, PPBP et DAPP1.

6. Procédé selon l'une quelconque des revendications 1 à 5, dans lequel le profil d'expression est déterminé en mesurant les ARN acellulaires (cfRNA) dans l'échantillon maternel.

7. Procédé selon la revendication 6, dans lequel le profil d'expression comprend des niveaux d'expression de gènes individuels qui sont déterminés par qPCR ou séquençage massivement parallèle.

8. Procédé selon la revendication 1, dans lequel le profil d'expression est déterminé en mesurant les protéines dans l'échantillon maternel, éventuellement dans lequel les protéines sont mesurées par spectrométrie de masse.

9. Procédé selon l'une quelconque des revendications 1 à 8, dans lequel l'échantillon maternel provient du deuxième trimestre de la grossesse.

10. Procédé selon l'une quelconque des revendications 1 à 8, dans lequel l'échantillon maternel provient du troisième trimestre de la grossesse.

11. Procédé selon l'une quelconque des revendications 1 à 8, dans lequel l'échantillon maternel provient de plus de 28 jours avant un accouchement prématuré ou provient de plus de 45 jours avant un accouchement prématuré.

12. Procédé selon l'une quelconque des revendications 1 à 11, comprenant en outre l'analyse de deux échantillons maternels ou plus de la femme enceinte à différents moments au cours d'une grossesse.

13. Procédé réalisé à l'aide d'un ordinateur pour évaluer le risque d'accouchement prématuré par une femme enceinte comprenant :
(a) l'obtention d'un ou plusieurs profils d'expression à partir d'un échantillon maternel de la femme enceinte, dans lequel l'un ou plusieurs profils d'expression correspondent à l'expression d'une pluralité de transcrits d'ARN acellulaire (cfRNA) à partir d'un premier panel de gènes ;
(b) la comparaison, à l'aide d'un système informatique, de l'un ou plusieurs profils d'expression à un ou plusieurs profils de référence caractéristiques d'une femme ayant (a) un risque élevé d'accouchement prématuré ou (b) un risque faible d'accouchement prématuré, ou une caractéristique d'une femme ayant une durée de grossesse définie, dans lequel l'un ou plusieurs profils de référence sont déterminés à l'aide d'un modèle d'apprentissage automatique qui analyse des premiers échantillons d'apprentissage qui sont des profils d'expression de cfRNA étiquetés comme étant prématurés ou à terme, ou étiquetés avec une durée de grossesse ; et
(c) l'actualisation, à l'aide du système informatique, de l'un ou plusieurs profils de référence par :
(1) la réception de seconds échantillons d'apprentissage, dans lequel les seconds échantillons d'apprentissage sont des profils d'expression de cfRNA étiquetés comme prématurés ou à terme ou étiquetés avec une durée de grossesse, et
(2) l'ajustement de manière itérative de l'un ou plusieurs profils de référence à l'aide d'un modèle d'apprentissage automatique pour augmenter le nombre des premiers échantillons d'apprentissage et des seconds échantillons d'apprentissage qui sont classés correctement,
dans lequel le premier panel de gènes comprend RAB27B et deux gènes ou plus sélectionnés parmi PPBP, DAPP1, RGS18, CLCN3, MAP3K7CL, MOB1B, POLE2, LYPLAL1 et TBC1D15.

14. Système informatique comprenant : (a) une base de données comprenant un ou plusieurs profils de référence, chacun comprenant (i) un niveau d'expression dans une population de femmes enceintes de transcrits d'ARN acellulaire (cfRNA) correspondant à un premier panel de gènes et (ii) un risque d'accouchement prématuré ; (b) une interface utilisateur configurée pour interagir avec un ordinateur client sur un réseau et pour recevoir un ou plusieurs profils d'expression comprenant le niveau d'expression chez une femme enceinte de transcriptions de cfRNA correspondant au premier panel de gènes ; et (c) un ou plusieurs processeurs configurés pour analyser l'un ou plusieurs profils de référence et l'un ou plusieurs profils d'expression, comprenant la comparaison de l'un ou plusieurs profils de référence à l'un ou plusieurs profils d'expression pour déterminer le risque d'accouchement prématuré ; et (d) une interface réseau qui transmet le risque d'accouchement prématuré à l'ordinateur client,
dans lequel le premier panel de gènes comprend RAB27B et deux gènes ou plus sélectionnés parmi PPBP, DAPP1, RGS18, CLCN3, MAP3K7CL, MOB1B, POLE2, LYPLAL1 et TBC1D15.
